(19) Europäisches Patentamt / European Patent Office / Office européen des brevets

(11) **EP 3 848 935 A1**

(12) **EUROPEAN PATENT APPLICATION**
published in accordance with Art. 153(4) EPC

(43) Date of publication:
**14.07.2021 Bulletin 2021/28**

(51) Int Cl.:
**G16B 20/00** *(2019.01)*

(21) Application number: **18932448.6**

(22) Date of filing: **07.09.2018**

(86) International application number:
**PCT/JP2018/033329**

(87) International publication number:
**WO 2020/049748 (12.03.2020 Gazette 2020/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **FUJITSU LIMITED**
**Kanagawa 211-8588 (JP)**

(72) Inventors:
• **KATAOKA, Masahiro**
  **Kawasaki-shi, Kanagawa 211-8588 (JP)**
• **MIYAZAKI, Toshiya**
  **Kawasaki-shi, Kanagawa 211-8588 (JP)**
• **TOMOBE, Akihiro**
  **Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **SPECIFICATION METHOD, SPECIFICATION PROGRAM, AND INFORMATION PROCESSING DEVICE**

(57) An information processing device (100) compares the codons included in reference codon sequence data and the codons included in analysis-target codon sequence data, at each sequence position of the codons. Then, the information processing device (100) identifies a plurality of codons positioned at the sequence positions that are subsequent to the sequence position at which codons are nonidentical. Moreover, the information processing device (100) refers to a memory unit that stores the type of mutation, which has occurred at a particular codon, in a corresponding manner to a plurality of codons subsequent to the particular codon, on account of occurrence of the mutation in the particular codon. Then, the information processing device (100) identifies the type of mutation corresponding to the plurality of codons.

FIG.5

**EP 3 848 935 A1**

**Description**

[Technical Field]

The present invention is related to an identification method.

[Background Art]

In recent years, the base sequences constituting the DNA (deoxyribonucleic acid) and the RNA (ribonucleic acid) of living organisms are analyzed so as to predict the impact of new types of viruses, and accordingly vaccines are developed. Moreover, research is being carried out for detecting mutation (point mutation) such as cancer and detecting genetic abnormality such as genetic mutation, and diagnosing the risk of developing diseases.

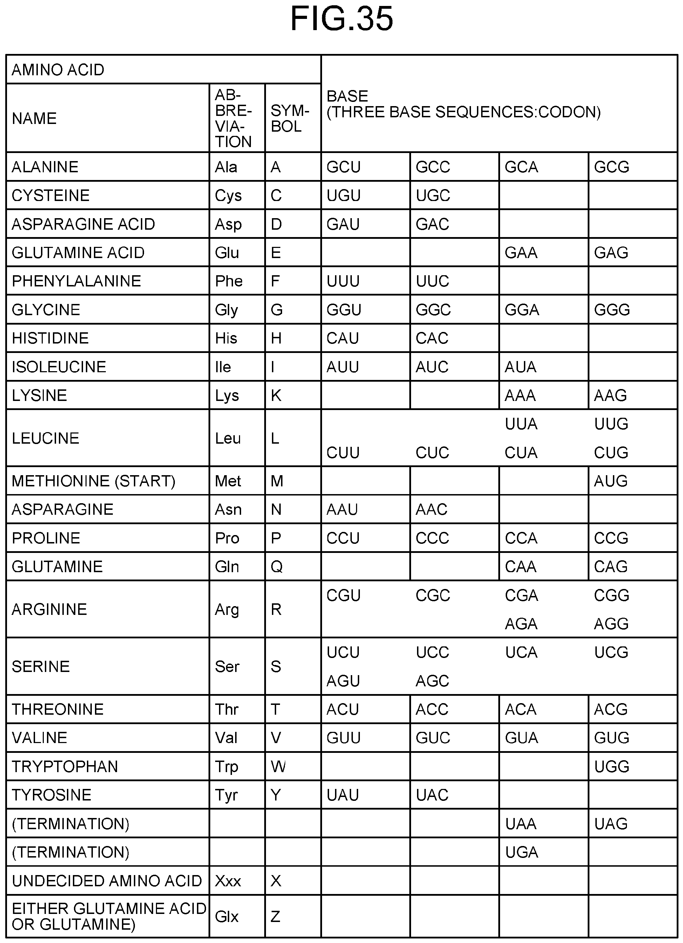

The DNA and the RNA have four types of bases represented by symbols "A", "G", "C", and "T" or "U". Moreover, a mass of three base sequences decides 20 types of amino acids. Each amino acid is represented by a symbol from "A" to "Y". FIG. 35 is a diagram illustrating the relationship of the amino acids with the base sequences and with codons. Herein, a mass of three base sequences is called a "codon". A codon is decided according to the arrangement of the bases; and, once a codon is decided, an amino acid gets decided.

As illustrated in FIG. 35, a single amino acid is associated to a plurality of types of codons. Hence, when a codon gets decided, an amino acid gets decided. However, even if an amino acid gets decided, the codon does not get uniquely identified. For example, the amino acid "alanine (Ala)" is associated to codons "GCU", "GCC", GCA", and "GCG".

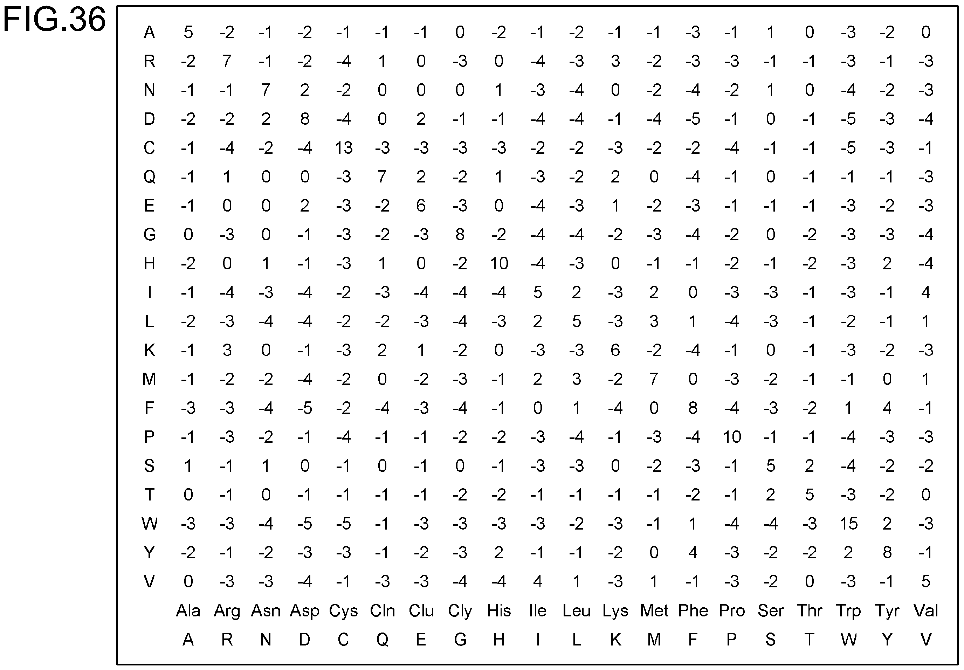

In the related technology, in the case of analyzing a new type of virus, FASTA or BLAST is implemented. In FASTA or BLAST, the base sequences are translated into the symbols of amino acids; a homology search is performed with the amino acids serving as the units for comparison; and similarities with the viruses discovered in the past are determined. FIG. 36 is a diagram illustrating a score matrix used in performing a homology search.

Moreover, in the related technology, in the case of analyzing mutation such as cancer, mutation in the form of "base insertion", "base deletion", or "base substitution" is determined; the frameshift of the sequences attributed to mutation is determined; and the underlying genetic mutation developed from the mutation point onward is further detected.

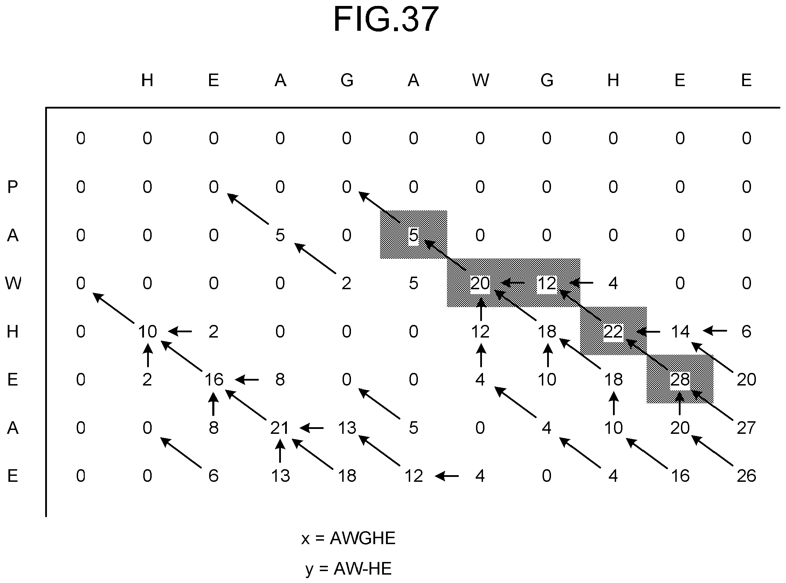

FIG. 37 is a diagram illustrating an example of the related technology for determining the frameshift of mutation. Regarding the frameshift of mutation, in order to enhance the accuracy, the Smith-waterman algorithm is implemented and local alignment determination is performed in the units of bases. In the Smith-waterman algorithm, Equation (1) given below is used. In the related technology, after initialization is performed, the matrix illustrated in FIG. 37 is searched for the maximum score F(i, j) given in Equation (1), and the cell in which "0" is reached is traced back from the searched location.

$$F(i, j) = \max \begin{cases} 0 \\ F(i-1, j-1) + s(x_i, y_j) \\ F(i-1, j) - d \\ F(i, j-1) - d \end{cases} \quad (1)$$

[Citation List]

[Patent Citation]

Patent Document 1: International Publication Pamphlet No. WO 2009/013910
Patent Document 2: Japanese Laid-open Patent Publication No. 2002-132781
Patent Document 3: Japanese Laid-open Patent Publication No. 2004-355522
Patent Document 4: International Publication Pamphlet No. WO 2008/108297
Patent Document 5: Japanese National Publication of International Patent Application No. 2015-536156

[Summary of Invention]

[Technical Problem]

However, in the related technology explained above, a long period of time is requested in determining the frameshift of the mutation and detecting the underlying genetic mutation developed from the mutation point onward. Moreover, in order to speed up the search (collation), the base sequences need to be partitioned.
In the related technology, in the case of determining the frameshift of the mutation, such as cancer, or detecting the underlying genetic mutation developed from the mutation point onward, local alignment determination is performed in the units of bases in order to enhance the accuracy. However, that results in a decline in the speed. On the other hand, in a genome search, as compared to a text search, the size of the pointer-type inverted index becomes enormous. Hence, an index-based search cannot be performed, thereby resulting in a low speed. In order to hold down the decline in the speed, the base data is partitioned, and automaton collation is performed in parallel operations. However, it results in losses attributed to partitioning, such as complications in management and decline in operability.
According to an aspect, it is an objective of the present invention to provide an identification method, an identification program, and an information processing device that enable achieving reduction in the time requested in determining the frameshift of the mutation and detecting the underlying genetic mutation developed from the mutation point onward. Moreover, according to an aspect, it is an objective of the present invention to provide an identification method, an identification program, and an information processing device that enable speeding up the search and the analysis without having to partition the base sequences.

[Solution to Problem]

In one proposal, a computer performs following processing. The computer obtains reference codon sequence data and analysis-target codon sequence data. The computer compares codons included in the obtained reference codon sequence data and codons included in the obtained analysis-target codon sequence data, at each sequence position of codon. The computer identifies, based on result of the comparing, from among codons included in the analysis-target codon sequence data, codon positioned at each of a plurality of sequence positions subsequent to sequence position at which codons are nonidentical. The computer referrs to a memory unit configured to store type of mutation, which has occurred at a particular codon included in particular codon sequence data, in a corresponding manner to codon positioned at each of a plurality of sequence positions subsequent to the particular codon, on account of occurrence of the mutation in the particular codon, and identifies type of mutation associated to codon positioned at each of the plurality of identified sequence positions.

[Advantageous Effects of Invention]

It becomes possible to reduce the time requested in determining the type of frameshift of the mutation and detecting the genetic mutation.

[Brief Description of Drawings]

FIG. 1 is a diagram (1) for explaining the operations performed in an information processing device according to a first embodiment.
FIG. 2 is a diagram (2) for explaining the operations performed in the information processing device according to the first embodiment.
FIG. 3 is a diagram (3) for explaining the operations performed in the information processing device according to the first embodiment.
FIG. 4 is a diagram (4) for explaining the operations performed in the information processing device according to the first embodiment.
FIG. 5 is a functional block diagram illustrating a configuration of the information processing device according to the first embodiment.
FIG. 6 is a diagram illustrating an exemplary data structure of reference codon sequence data.
FIG. 7 is a diagram illustrating an exemplary data structure of analysis-target codon sequence data.
FIG. 8 is a diagram illustrating an exemplary data structure of a code conversion table.
FIG. 9 is a diagram illustrating an exemplary data structure of first-type sequence data.
FIG. 10 is a diagram illustrating an exemplary data structure of second-type sequence data.

FIG. 11 is a diagram illustrating an exemplary data structure of an insertion transition table.

FIG. 12A is a diagram illustrating a data structure of a transition table 50U in the insertion transition table.

FIG. 12B is a diagram illustrating a data structure of a transition table 50C in the insertion transition table.

FIG. 12C is a diagram illustrating a data structure of a transition table 50A in the insertion transition table.

FIG. 12D is a diagram illustrating a data structure of a transition table 50G in the insertion transition table.

FIG. 13 is a diagram illustrating an exemplary data structure of a deletion transition table.

FIG. 14A is a diagram illustrating a data structure of a transition table 55U in the deletion transition table.

FIG. 14B is a diagram illustrating a data structure of a transition table 55C in the deletion transition table.

FIG. 14C is a diagram illustrating a data structure of a transition table 55A in the deletion transition table.

FIG. 14D is a diagram illustrating a data structure of a transition table 55G in the deletion transition table.

FIG. 15 is a flowchart for explaining a sequence of operations performed in the information processing device according to the first embodiment.

FIG. 16 is a diagram (1) for explaining the operations performed in an information processing device according to a second embodiment.

FIG. 17 is a diagram (2) for explaining the operations performed in the information processing device according to the second embodiment.

FIG. 18 is a diagram (3) for explaining the operations performed in the information processing device according to the second embodiment.

FIG. 19 is a functional block diagram illustrating a configuration of the information processing device according to the second embodiment.

FIG. 20 is a flowchart (1) for explaining a sequence of operations performed in the information processing device according to the second embodiment.

FIG. 21A is a diagram illustrating an exemplary data structure of a codon-amino acid conversion table.

FIG. 21B is a diagram for explaining the other operations performed in the information processing device according to the second embodiment.

FIG. 22 is a flowchart (2) for explaining a sequence of operations performed in the information processing device according to the second embodiment.

FIG. 23 is a diagram (1) for explaining the operations performed in an information processing device according to a third embodiment.

FIG. 24 is a diagram (2) for explaining the operations performed in the information processing device according to the third embodiment.

FIG. 25 is a functional block diagram illustrating a configuration of the information processing device according to the third embodiment.

FIG. 26 is a diagram for explaining an example of the operations for hashing an inverted index.

FIG. 27 is a diagram illustrating an example of the operations for restoring an inverted index.

FIG. 28 is a diagram for explaining the operations performed by an identifying unit according to the third embodiment.

FIG. 29 is a flowchart (1) for explaining a sequence of operations performed in the information processing device according to the third embodiment.

FIG. 30 is a flowchart for explaining the operations performed by the identifying unit according to the third embodiment for identifying the offset corresponding to point mutation.

FIG. 31 is a diagram for explaining the other operations performed in the information processing device according to the third embodiment.

FIG. 32 is a flowchart (2) for explaining a sequence of operations performed in the information processing device according to the third embodiment.

FIG. 33 is a diagram illustrating an exemplary hardware configuration of a computer that implements the functions identical to the functions of the information processing devices according to the first and second embodiments.

FIG. 34 is a diagram illustrating an exemplary hardware configuration of a computer that implements the functions identical to the functions of the information processing device according to the third embodiment.

FIG. 35 is a diagram illustrating the relationship between amino acids and codons.

FIG. 36 is a diagram illustrating a score matrix used in performing a homology search.

FIG. 37 is a diagram illustrating an example of the related technology for determining the frameshift of mutation.

[Embodiments for Carrying Out the Invention]

Exemplary embodiments of an identification method, an identification program, and an information processing device according to the present invention are described below in detail with reference to the accompanying drawings. However, the present invention is not limited by the embodiments described below.

[First embodiment]

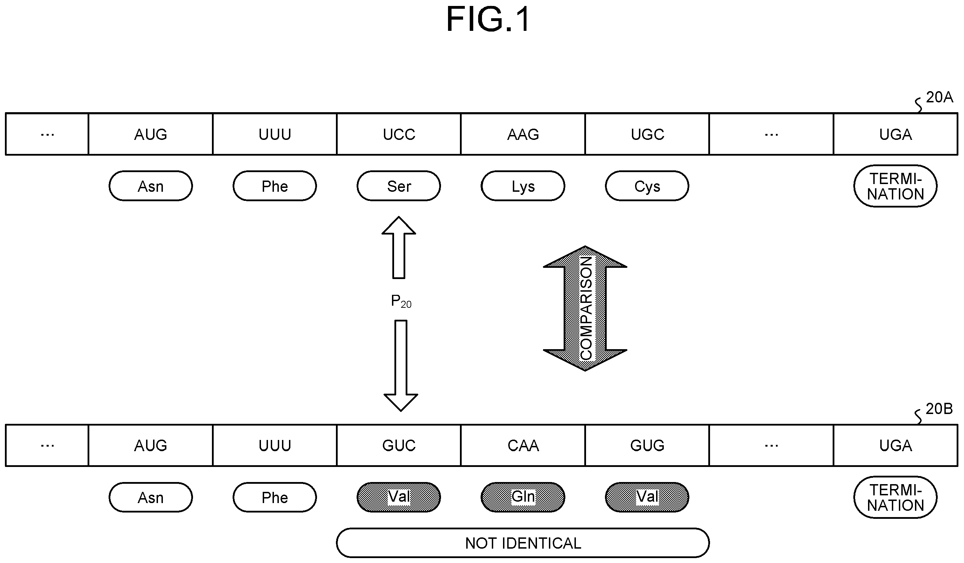

FIGS. 1 to 4 are diagrams for explaining the operations performed in an information processing device according to a first embodiment. The information processing device performs the operations explained below and identifies point mutation that has occurred in the target base sequence for analysis. Herein, point mutation includes "base insertion", "base deletion", and "base substitution". In the first embodiment, the information that is about the normal base sequence and that is represented in the units of codons is referred to as "reference codon sequence data". Moreover, the information that is about the target base sequence for analysis and that is represented in the units of codons is referred to as "analysis-target codon sequence data".

The following explanation is given about FIG. 1. The information processing device compares reference codon sequence data 20A and analysis-target codon sequence data 20B in sequence from the beginning in the units of codons. As a result of comparing the reference codon sequence data 20A and the analysis-target codon sequence data 20B, the information processing device identifies that the codons are nonidentical from a sequence position $P_{20}$ onward. Hence, the information processing device determines that mutation is present in the analysis-target codon sequence data 20B. In the following explanation, the reference codon sequence data and the analysis-target codon sequence data are compared in sequence from the beginning; and a position having nonidentical codons is referred to as a "mutation position" and the concerned codons are referred to as "mutant codon" and "mutation codon", respectively.

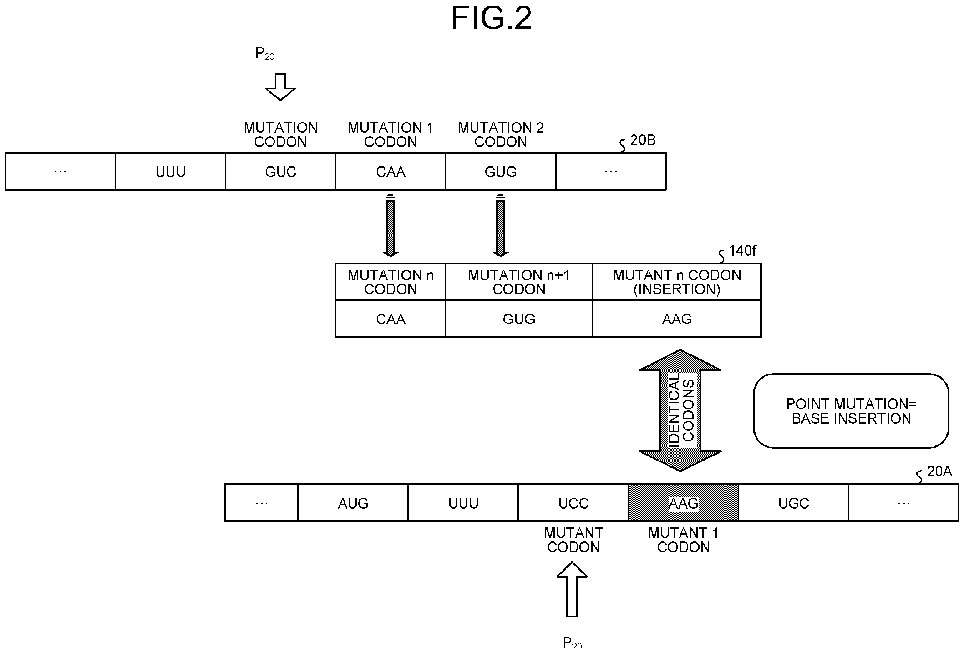

The following explanation is given about FIG. 2. When it is determined that mutation is present in the analysis-target codon sequence data 20B, the information processing device identifies, from the codons included in the analysis-target codon sequence data 20B, the mutation codon and the subsequent two codons. The subsequent two codons are referred to as a "mutation n codon" (where n is an integer equal to or greater than one) and a "mutation n+1 codon". For example, with reference to FIG. 2, if "GUC" represents the mutation codon, then "CAA" represents the mutation 1 codon and "GUG" represents the mutation 2 codon.

Then, based on an insertion transition table 140f and based on the mutation n codon and the mutation n+1 codon that are positioned subsequent to the mutation codon, the information processing device identifies the mutant n codon that is the subsequent codon of the mutant codon. Herein, n is an integer equal to or greater than one. Herein, the codon subsequent to the mutant codon is referred to as "mutant n codon (base insertion)". The insertion transition table 140f is a table in which two codons subsequent to the mutation codon and a single codon subsequent to the pre-base-insertion mutant codon are held in a corresponding manner. When the mutant n codon in the insertion transition table 140f is identical to the codon subsequent to the mutation position in the reference codon sequence data, the point mutation that has occurred in the analysis-target codon sequence data is "base insertion".

In the example illustrated in FIG. 2, in the insertion transition table 140f, "AAG" represents the mutant n codon associated to the mutation n codon "CAA" and the mutation n+1 codon "GUG" that are subsequent to the mutation codon "GUC". When the information processing device compares the codon "AAG", which is subsequent to the reference position $P_{20}$ in the reference codon sequence data 20A, with the mutant n codon (insertion) "AAG", the two codons "AAG" happen to be identical. Hence, the information processing device determines that the mutation that has occurred in the analysis-target codon sequence data 20B is "base insertion".

Meanwhile, if the mutation n codon in the insertion transition table 140f is not identical to the subsequent codon of the mutation position in the reference codon sequence data, the point mutation that has occurred in the analysis-target codon sequence data is "base deletion" or "base substitution".

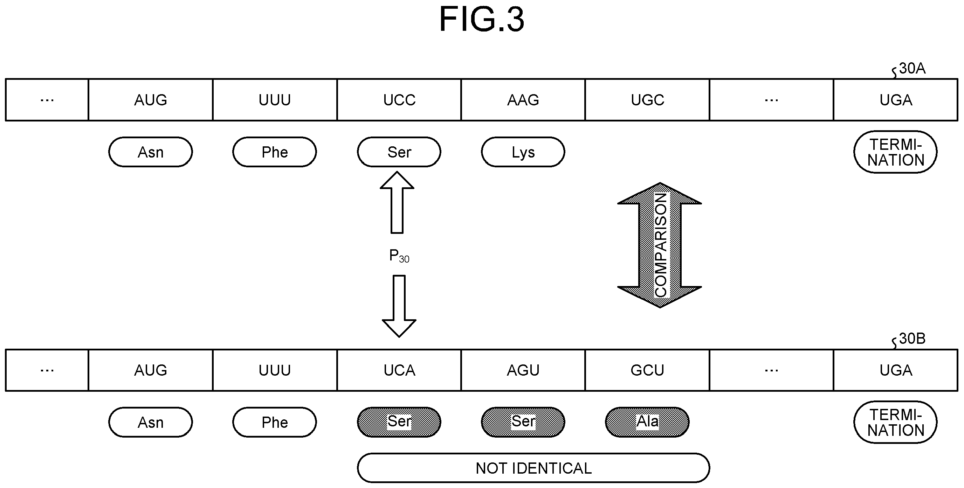

The following explanation is given about FIG. 3. The information processing device compares reference codon sequence data 30A and analysis-target codon sequence data 30B in sequence from the beginning in the units of codons. As a result of comparing the reference codon sequence data 30A and the analysis-target codon sequence data 30B, the information processing device identifies that the codons are nonidentical from a sequence position (mutation position) $P_{30}$ onward. Hence, the information processing device determines that mutation is present in the analysis-target codon sequence data 30B.

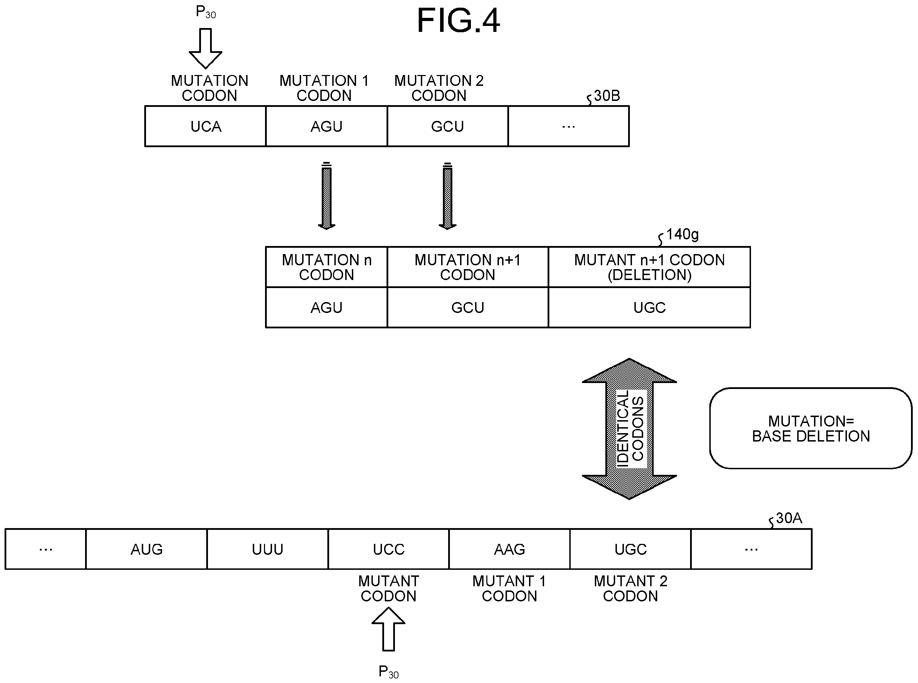

The following explanation is given about FIG. 4. When it is determined that mutation is present in the analysis-target codon sequence data 30B, the information processing device identifies, from the codons included in the analysis-target codon sequence data 30B, the mutation codon and two subsequent codons. For example, in the example illustrated in FIG. 4, "UCA" represents the mutation codon. Moreover, "AGU" and "GCU" represent the two subsequent codons.

Then, based on a deletion transition table 140g and based on the two codons that are positioned subsequent to the mutation codon, the information processing device identifies the second subsequent codon of the pre-base-deletion mutant codon. The second subsequent codon is referred to as "mutant n+1 codon (base deletion)". The deletion transition table 140g is a table in which the mutation codon, the subsequent two codons, and the second subsequent codon of the pre-base-deletion mutant codon are held in a corresponding manner. When the mutant n+1 codon in the deletion transition table 140g is identical to the second subsequent codon of the mutation position in the reference codon sequence data, the point mutation that has occurred in the analysis-target codon sequence data is "base deletion".

In the example illustrated in FIG. 4, in the deletion transition table 140g, "UGC" represents the pre-base-deletion mutant n+1 codon associated to "AUG" and "GCU" that represent the two codons subsequent to the mutation codon "UCA". When the information processing device compares the pre-base-deletion mutant n+1 codon "UGC" with the second subsequent codon "UGC" of the codon "UUU" at the mutation position $P_{30}$ in the reference codon sequence data 30A, the two codons "UGC" happen to be identical. Hence, the information processing device determines that the mutation that has occurred in the analysis-target codon sequence data 30B is "base deletion".

Till now, for convenience, the explanation was given about an example of determining deletion regarding the mutant 2 codon "UGC". However, regarding the mutant 1 codon "AAG" too, the deletion transition table 140g can be used and the mutant 1 codon "AAG" can be referred to using the mutation (0) codon "UCA" and the mutation 1 codon "AUG", and deletion can be determined (herein, n is an integer equal to or greater than zero).

Meanwhile, if the mutant n+1 codon in the deletion transition table 140g is not identical to the second subsequent codon of the mutation position in the reference codon sequence data, then the point mutation that has occurred in the analysis-target codon sequence data is "base insertion" or "base substitution".

On the other hand, if a plurality of codons subsequent to the mutation codon in the analysis-target codon sequence data is identical to a plurality of mutant codons in the reference codon sequence data, then the point mutation that has occurred in the analysis-target codon sequence data is "base substitution".

As explained above, the information processing device according the first embodiment compares the reference codon sequence data and the analysis-target codon sequence data in the units of codons, and identifies nonidentical codons. Then, based on the two subsequent codons of the nonidentical codon, the information processing device obtains the subsequent codon of the mutant codon from the insertion transition table 140f; obtains the second subsequent codon of the mutant codon from the deletion transition table 140g; compares the obtained codons with the subsequent codon of the mutant codon included in the analysis-target-codon sequence data; and identifies the type of point mutation. Thus, as a result of performing comparison in the units of encoded codons in a consistent manner, the type of mutation can be determined while identifying the nonidentical codons. That enables achieving reduction in the time requested in determining the type of mutation.

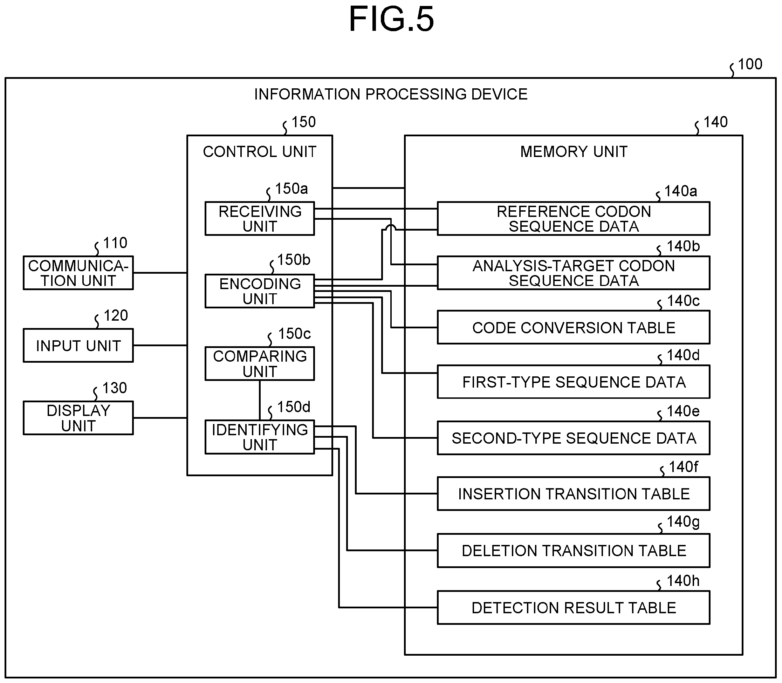

Given below is the explanation of a configuration of the information processing device according to the first embodiment. FIG. 5 is a functional block diagram illustrating a configuration of the information processing device according to the first embodiment. As illustrated in FIG. 5, an information processing device 100 includes a communication unit 110, an input unit 120, a display unit 130, a memory unit 140, and a control unit 150.

The communication unit 110 is a processing unit that performs data communication with external devices (not illustrated) via a network. The communication unit 110 is an example of a communication device. For example, the information processing device 100 can receive information such as reference codon sequence data 140a and analysis-target codon sequence data 140b from an external device via a network.

The input unit 120 is an input device for enabling input of a variety of information to the information processing device 100. Examples of the input unit 120 include a keyboard, a mouse, or a touch-sensitive panel.

The display unit 130 is a display device that displays a variety of information output from the control unit 150. Examples of the display unit 130 include an organic EL (electro-luminescence) display, a liquid crystal display, and a touch-sensitive panel.

The memory unit 140 is used to store the reference codon sequence data 140a, the analysis-target codon sequence data 140b, a code conversion table 140c, first-type sequence data 140d, and second-type sequence data 140e. Moreover, the memory unit 140 is used to store the insertion transition table 140f, the deletion transition table 140g, and a detection result table 140h. Examples of the memory unit 140 include a semiconductor memory such as a RAM (Random Access Memory), a ROM (Read Only Memory), or a flash memory; and a memory device such as an HDD (Hard Disk Drive).

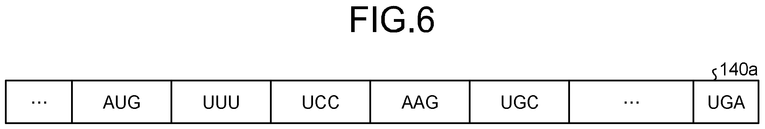

The reference codon sequence data 140a represents the information about normal base sequences indicated in the units of codons. FIG. 6 is a diagram illustrating an exemplary data structure of the reference codon sequence data. As illustrated in FIG. 6, in the reference codon sequence data 140a, a plurality of codons from the start codon to the termination codon is arranged. For example, "AUG" represents the start codon, and "UGA" represents the termination codon.

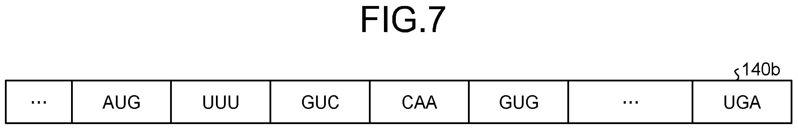

The analysis-target codon sequence data 140b represents the information about the target base sequence for analysis indicated in the units of codons. FIG. 7 is a diagram illustrating an exemplary data structure of the analysis-target codon sequence data. As illustrated in FIG. 7, in the analysis-target codon sequence data 140b, a plurality of codons from the start codon to the termination codon is arranged. For example, "AUG" represents the start codon, and "UGA" represents the termination codon.

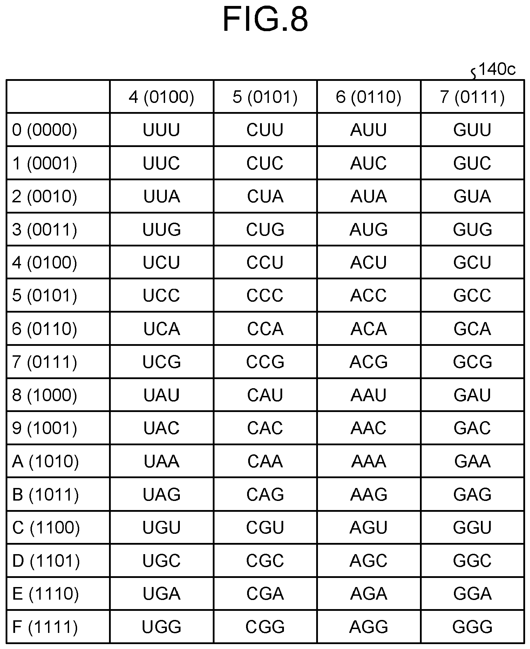

The code conversion table 140c is a table in which codons and codes are held in a corresponding manner. FIG. 8 is a diagram illustrating an exemplary data structure of the code conversion table. For example, the codon "UUU" is held in a corresponding manner to a code "40h (01000000)". Herein, "h" is a code indicating a hexadecimal numeral. For the purpose of illustration, the encoded form of the codon "UUU" is referred to as "UUU (40h)". Regarding the other codons too, the encoded form is illustrated using a bracket.

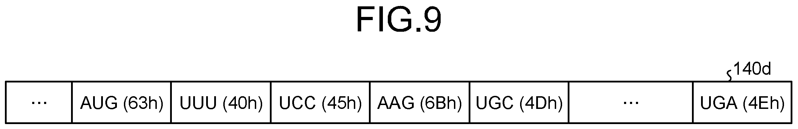

The first-type sequence data 140d represents the sequence data obtained as a result of encoding the reference codon sequence data 140a based on the code conversion table 140c. FIG. 9 is a diagram illustrating an exemplary data structure of the first-type sequence data. As illustrated in FIG. 9, in the first-type sequence data 140d, a plurality of encoded codons from the start codon to the termination codon is arranged.

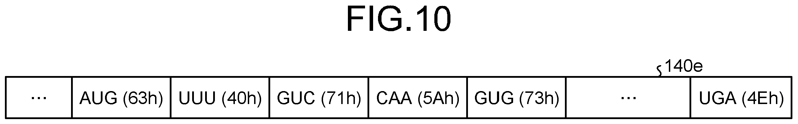

The second-type sequence data 140e represents sequence data obtained as a result of encoding the analysis-target codon sequence data 140b based on the code conversion table 140c. FIG. 10 is a diagram illustrating an exemplary data structure of the second-type sequence data. As illustrated in FIG. 10, in the second-type sequence data 140e, a plurality of encoded codons from the start codon to the termination codon is arranged.

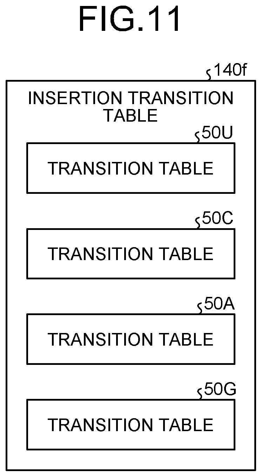

The insertion transition table 140f is a table in which mutation n codons and mutation n+1 codons, which are positioned subsequent to mutation codons, are held in a corresponding manner with pre-base-insertion mutant n codons. FIG. 11 is a diagram illustrating an exemplary data structure of the insertion transition table. As illustrated in FIG. 11, the insertion transition table 140f includes transition tables 50U, 50C, 50A, and 50G.

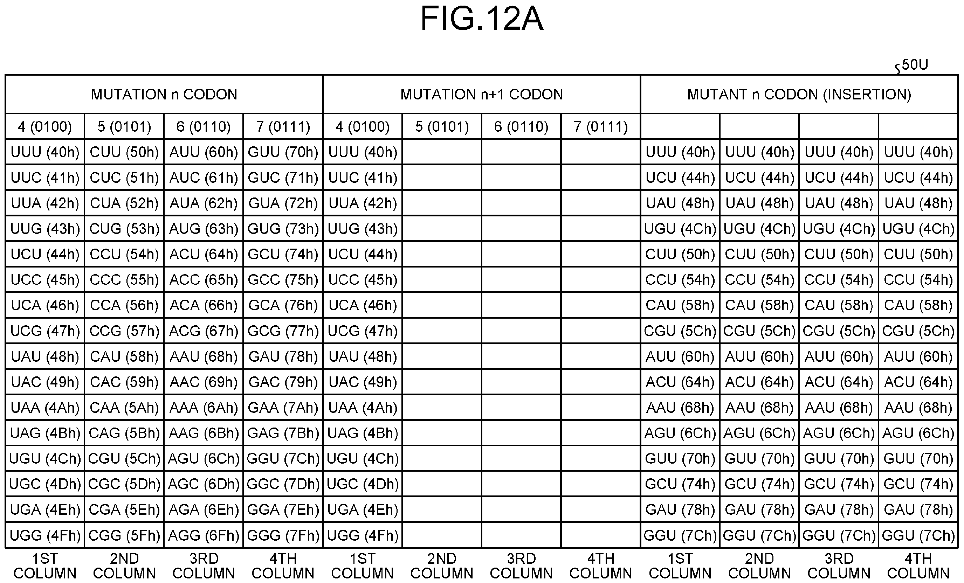

In the transition table 50U, all mutation n codons, the mutation n+1 codons (the codons starting with U), and the pre-base-insertion mutant n codons are held in a corresponding manner. The relationship among the codons is defined by the encoded codons. FIG. 12A is a diagram illustrating a data structure of the transition table 50U in the insertion transition table. Regarding the mutation n codon in the i-th row and the j-th column and a mutation n+1 codon, the corresponding codon is the pre-base-insertion mutant n codon in the i-th row and the j-th column.

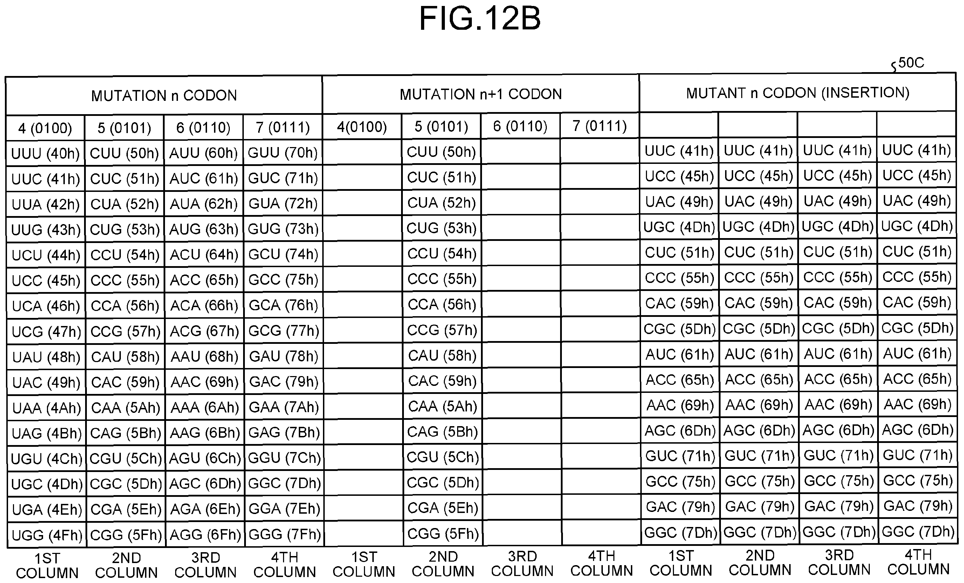

In the transition table 50C, all mutation n codons, the mutation n+1 codons (the codons starting with C), and the pre-base-insertion mutant n codons are held in a corresponding manner. The relationship among the codons is defined by the encoded codons. FIG. 12B is a diagram illustrating a data structure of the transition table 50A in the insertion transition table. Regarding the mutation n codon in the i-th row and the j-th column and a mutation n+1 codon, the corresponding codon is the pre-base-insertion mutant n codon in the i-th row and the j-th column.

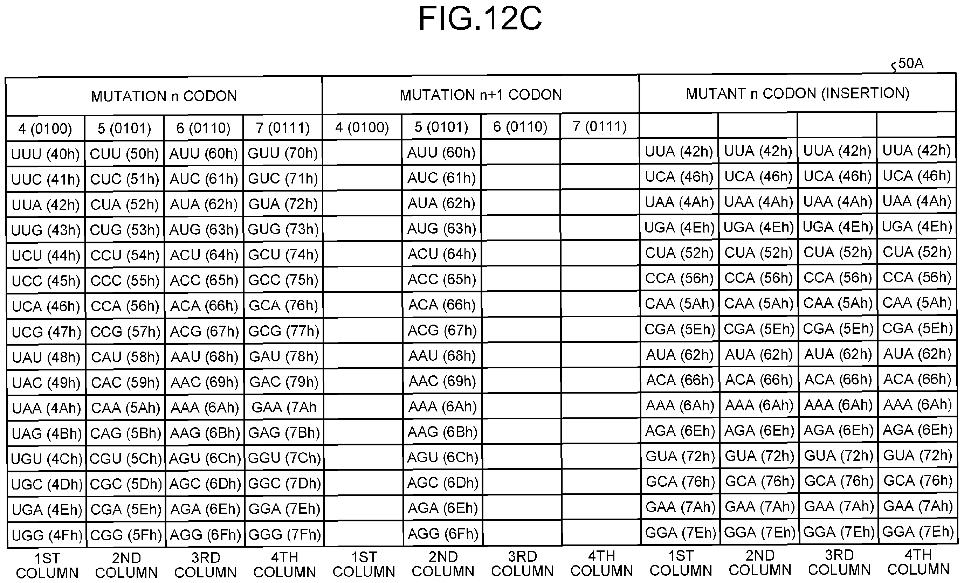

In the transition table 50A, all mutation n codons, the mutation n+1 codons (the codons starting with A), and the pre-base-insertion mutant n codons are held in a corresponding manner. The relationship among the codons is defined by the encoded codons. FIG. 12C is a diagram illustrating a data structure of the transition table 50A in the insertion transition table. Regarding the mutation n codon in the i-th row and the j-th column and a mutation n+1 codon, the corresponding codon is the pre-base-insertion mutant n codon in the i-th row and the j-th column.

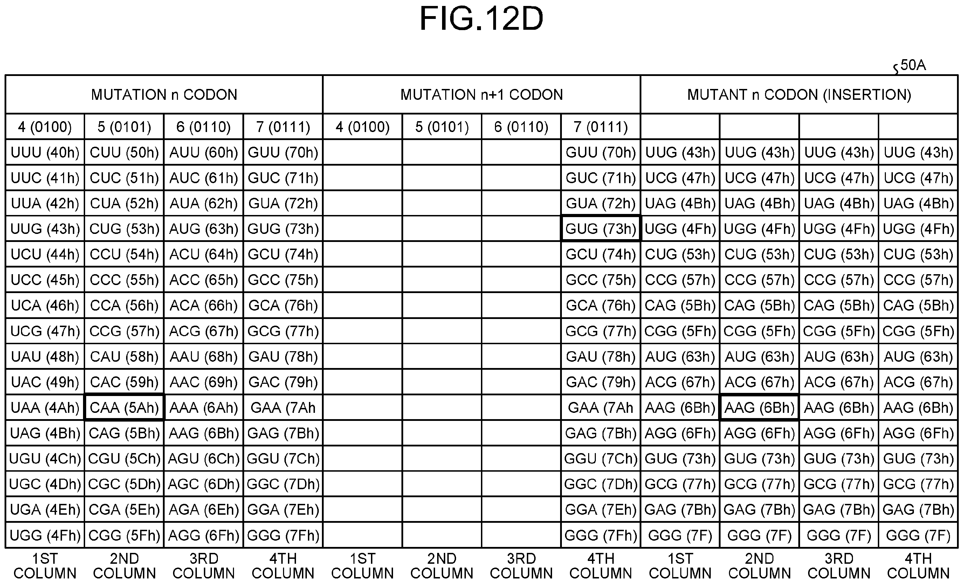

In the transition table 50C, all mutation n codons, the mutation n+1 codons (the codons starting with G), and the pre-base-insertion mutant n codons are held in a corresponding manner. The relationship among the codons is defined by the encoded codons. FIG. 12D is a diagram illustrating a data structure of the transition table 50G in the insertion transition table. Regarding the mutation n codon in the i-th row and the j-th column and a mutation n+1 codon, the corresponding codon is the pre-base-insertion mutant n codon in the i-th row and the j-th column. For example, regarding the mutation n codon "CAA (5Ah)" in the 11-th row and the second column and the mutation n+1 codon "GUG (73h)", the corresponding codon is the pre-base-insertion mutant n codon "AAG (6Bh)" in the 11-th row and the second column.

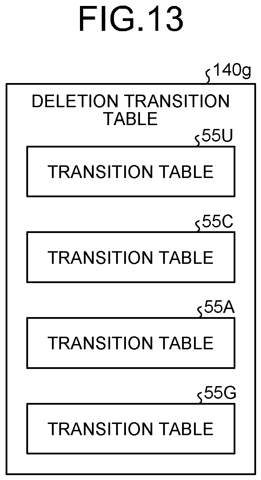

In the deletion transition table 140g, the mutation n codons, all mutation n+1 codons, and the pre-base-deletion mutant n+1 codons are held in a corresponding manner. FIG. 13 is a diagram illustrating an exemplary data structure of the deletion transition table. As illustrated in FIG. 13, the deletion transition table 140g includes transition tables 55U, 55C, 55A, and 55G.

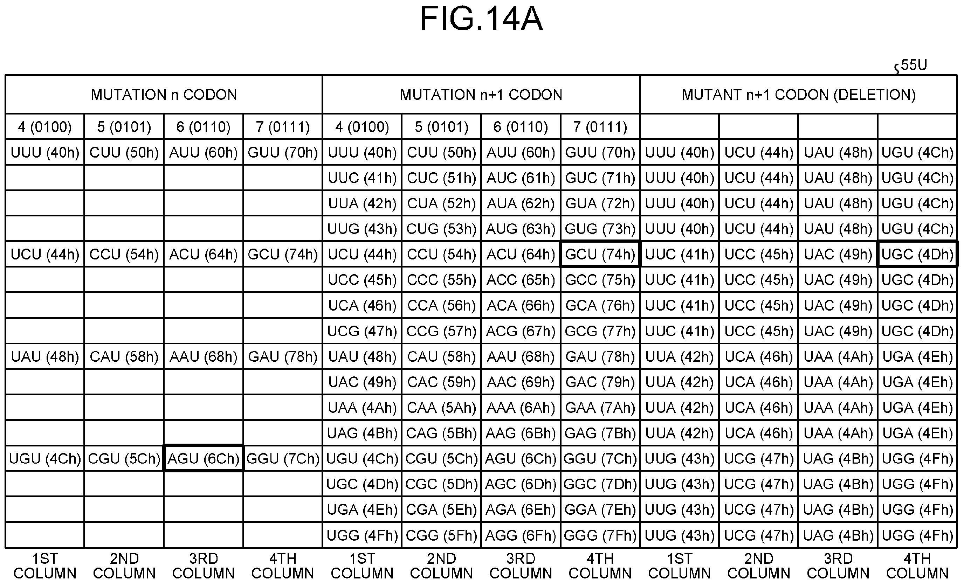

In the transition table 55U, the mutation n codons (the codons ending with U), all mutation n+1 codons, and the pre-base-deletion mutant n+1 codons are held in a corresponding manner. The relationship among the codons is defined by the encoded codons. FIG. 14A is a diagram illustrating a data structure of the transition table 55U in the deletion transition table. With reference to FIG. 14A, regarding any one mutation n codon and the mutation n+1 codon in the i-th row and the j-th column, the corresponding codon is the pre-base-deletion mutant n+1 codon in the i-th row and the j-th column. For example, regarding the mutation n codon "AGU (6Ch))" and the mutation n+1 codon "GCU (74h)" in the fifth row and the fourth column, the corresponding codon is the mutant n+1 codon "UGC (4Dh)" in the fifth row and the fourth column.

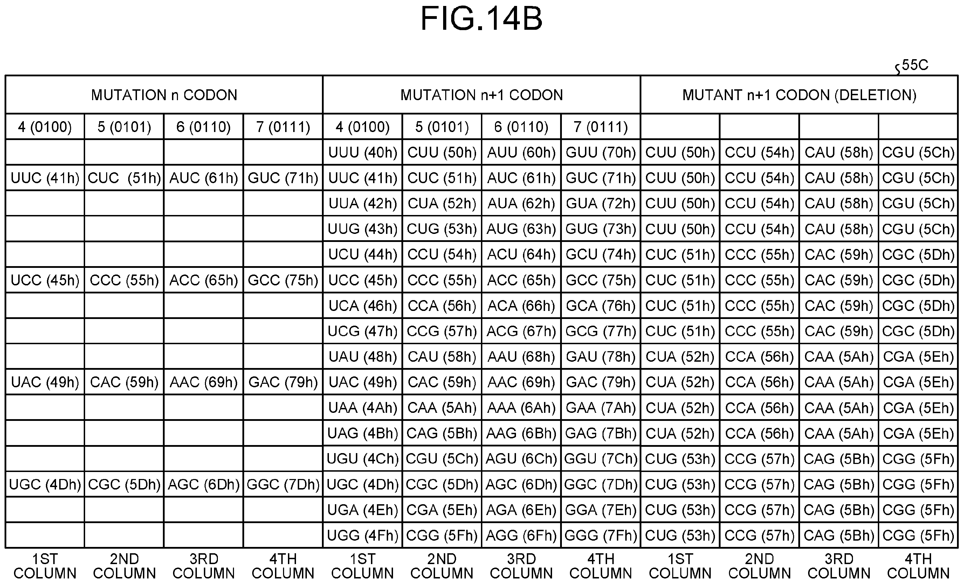

In the transition table 55C, the mutation n codons (the codons ending with C), all mutation n+1 codons, and the pre-base-deletion mutant n+1 codons are held in a corresponding manner. The relationship among the codons is defined by the encoded codons. FIG. 14B is a diagram illustrating a data structure of the transition table 55C in the deletion transition table. With reference to FIG. 14B, regarding any one mutation n codon and the mutation n+1 codon in the i-th row and the j-th column, the corresponding codon is the pre-base-deletion mutant n+1 codon in the i-th row and the j-th column.

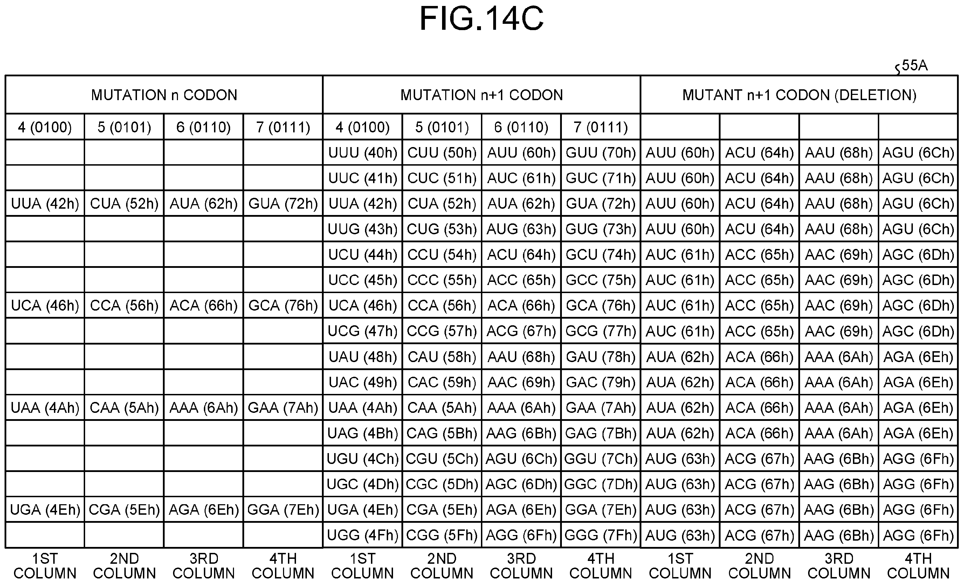

In the transition table 55A, the mutation n codons (the codons ending with A), all mutation n+1 codons, and the pre-base-deletion mutant n+1 codons are held in a corresponding manner. The relationship among the codons is defined by the encoded codons. FIG. 14C is a diagram illustrating a data structure of the transition table 55A in the deletion transition table. With reference to FIG. 14C, regarding any one mutation n codon and the mutation n+1 codon in the i-th row and the j-th column, the corresponding codon is the pre-base-deletion mutant n+1 codon in the i-th row and the j-th column.

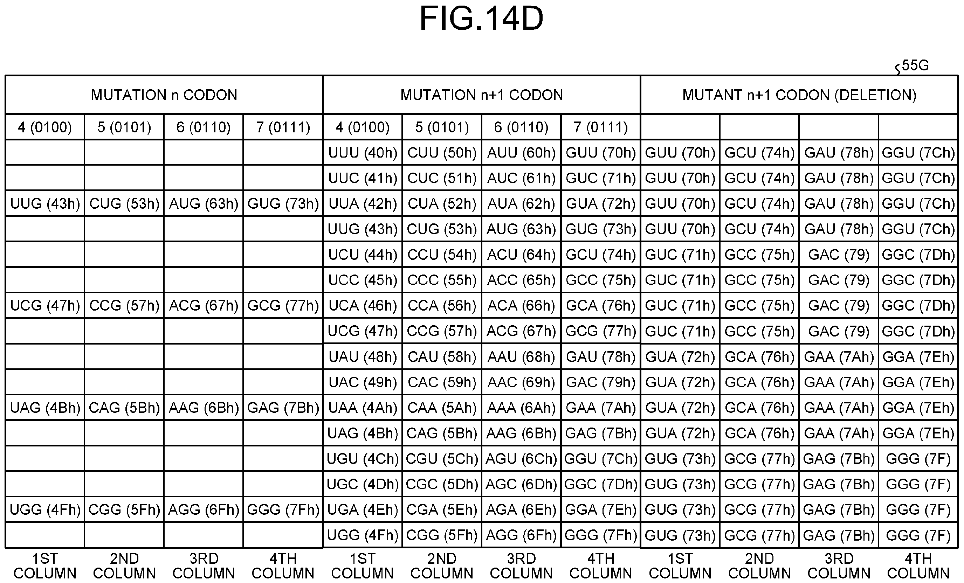

In the transition table 55G, the mutation n codons (the codons ending with G), all mutation n+1 codons, and the pre-base-deletion mutant n+1 codons are held in a corresponding manner. The relationship among the codons is defined by the encoded codons. FIG. 14D is a diagram illustrating a data structure of the transition table 55G in the deletion transition table. With reference to FIG. 14D, regarding any one mutation n codon and the mutation n+1 codon in the i-th row and the j-th column, the corresponding codon is the pre-base-deletion mutant n+1 codon in the i-th row and the j-th column.

Returning to the explanation with reference to FIG. 5, the detection result table 140h is a table for holding the information about the point mutations detected from the analysis-target codon sequence data 140b.

The control unit 150 includes a receiving unit 150a, an encoding unit 150b, a comparing unit 150c, and an identifying unit 150d. The control unit 150 is implemented using a CPU (Central Processing Unit) or an MPU (Micro Processing Unit). Alternatively, the control unit 150 can also be implemented using a hardwired logic such as an ASIC (Application Specific Integrated Circuit) or an FPGA (Field Programmable Gate Array).

The receiving unit 150a is a processing unit that receives the reference codon sequence data 140a and the analysis-target codon sequence data 140b from the input unit 120 or an external device. Then, the receiving unit 150a registers the reference codon sequence data 140a and the analysis-target codon sequence data 140b in the memory unit 140.

Moreover, when the insertion transition table 140f and the deletion transition table 140g are received from the input unit 120 or an external device, the receiving unit 150a registers the insertion transition table 140f and the deletion transition table 140g in the memory unit 140.

The encoding unit 150b is a processing unit that encodes the reference codon sequence data 140a and the analysis-target codon sequence data based on the code conversion table 140c. The encoding unit 150b compares the reference codon sequence data 140a and the code conversion table 140c and encodes each codon, so as to generate the first-type sequence data 140d. Similarly, the encoding unit 150b compares the analysis-target codon sequence data 140b and the code conversion table 140c and encodes each codon, so as to generate the second-type sequence data 140e. Then, the encoding unit 150b stores the first-type sequence data 140d and the second-type sequence data 140e in the memory unit 140.

As illustrated in FIG. 8, according to the code conversion table 140c, each codon is assigned with a 1-byte code. For example, the codon "UUU" gets converted into "40h (01000000)". The encoded codon is referred to as "UUU (40h) ".

The comparing unit 150c is a processing unit that compares the first-type sequence data 140d and the second-type sequence data 140e, and identifies mutation positions at which the encoded codons are not identical. As explained above, each codon is assigned with a 1-byte code. Hence, from the first-type sequence data 140d and the second-type sequence data 140e, the comparing unit 150c reads the codes one byte at a time from the beginning, and performs comparison.

If a mutation position having nonidentical codes is identified, the comparing unit 150c outputs the comparison result to the identifying unit 150d. The comparison result includes the information about the mutation position, a first-type mutant codon, a second-type mutation codon, the mutation n codon, and the mutation n+1 codon. The first-type mutant codon represents the encoded codon at the mutation position as included in the first-type sequence data 140d. The second-type mutation codon represents the encoded codon at the mutation position as included in the second-type sequence data 140e. The mutation n codon represents the codon (encoded codon) subsequent to the second-type mutation codon. The mutation n+1 codon represents the codon (encoded codon) positioned after the subsequent codon of the second-type mutation codon.

Meanwhile, when the first-type sequence data 140d is identical to the second-type sequence data 140e, the comparing unit 150c outputs the information indicating identicalness as the comparison result to the identifying unit 150d.

The identifying unit 150d is a processing unit that, based on the comparison result obtained by the comparing unit 150c and based on the insertion transition table 140f and the deletion transition table 140g, identifies the type of point mutation that has occurred at the mutation position.

If the pre-base-insertion mutant n codon, which is identified by the comparison of the mutation n codon and the mutation n+1 codon with the insertion transition table 140f, is identical to the subsequent codon of the first-type mutant codon; then the identifying unit 150d sets "base insertion" as the type of point mutation that has occurred at the mutation position.

For example, assume that the following information is included in the comparison result: the first-type mutant n codon "AAG (6Bh)", the second-type mutation n codon "CAA (5Ah))", and the mutation n+1 codon "GUG (73h)". As explained with reference to FIG. 12D, regarding the mutation n codon "CAA (5Ah)" and the mutation n+1 codon "GUG (73h)", the corresponding pre-base-insertion mutant n codon is "AAG (6Bh)". Since the pre-base-insertion mutant n codon "AAG (6Bh)" is identical to the codon "AAG (6Bh) that is subsequent to the first-type mutant codon, the identifying unit 150d sets "base insertion" as the type of point mutation that has occurred at the mutation position.

On the other hand, when the pre-base-insertion mutant n codon, which is identified by the comparison of the mutation n codon and the mutation n+1 codon with the insertion transition table 140f, is not identical to the subsequent codon of the first-type mutant codon; the identifying unit 150d excludes "base insertion" from the types of point mutation that has occurred at the mutation position.

When the pre-base-deletion mutant n+1 codon, which is identified by the comparison of the mutation n codon and the mutation n+1 codon with the deletion transition table 140g, is identical to the codon positioned after the subsequent codon of the first-type mutant codon; the identifying unit 150d sets "base deletion" as the type of point mutation that has occurred at the mutation position.

For example, assume that the following information is included in the comparison result: the first-type mutant n+1 codon "UGC (4Dh)", the second-type mutation n codon "AGU (6Ch)", and the mutation n+1 codon "GCU (74h)". As explained with reference to FIG. 14A, regarding the mutation n codon "AGU (6Ch)" and the mutation n+1 codon "GCU (74h))", the corresponding pre-base-deletion mutant n+1 codon is "UGC (4Dh)". Since the pre-base-deletion mutant codon "UGC (4Dh)" is identical to the codon "UGC (4Dh)" that is positioned after the subsequent codon of the first-type mutant codon, the identifying unit 150d sets "base deletion" as the type of point mutation that has occurred at the reference position.

On the other hand, when the pre-base-deletion mutant n+1 codon, which is identified by the comparison of the mutation n codon and the mutation n+1 codon with the deletion transition table 140g, is not identical to the codon positioned after the subsequent codon of the first-type mutant codon; the identifying unit 150d excludes "base deletion" from the types of point mutation that has occurred at the mutation position.

Meanwhile, as a result of performing identification using the insertion transition table 140f and performing identification using the deletion transition table 140g, if "base insertion" and "base deletion" are excluded from the types of point mutation that has occurred at the mutation position, then the identifying unit 150d sets "base substitution" as the type of point mutation that has occurred at the mutation position.

The identifying unit 150d registers, in the detection result table 140h, the information associating the mutation positions and the types of point mutation. Meanwhile, if information indicating identicalness is included in the comparison result, then the identifying unit 150d registers, in the detection result table 140h, the information indicating the absence of abnormalities. The information processing device 100 either can notify the external devices about the information of the detection result table 140h via a network, or can output the information of the detection result table 140h to the display unit 130 for display purposes.

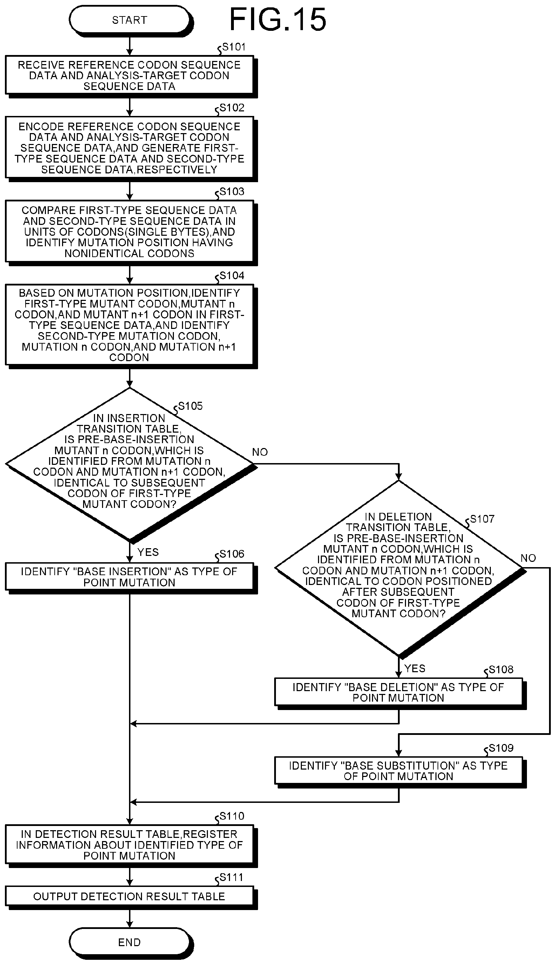

Given below is the explanation of an exemplary sequence of operations performed in the information processing device 100 according to the first embodiment. FIG. 15 is a flowchart for explaining a sequence of operations performed in the information processing device according to the first embodiment. As illustrated in FIG. 15, the receiving unit 150a of the information processing device 100 receives the reference codon sequence data 140a and the analysis-target codon sequence data 140b (Step S101) .

The encoding unit 150b of the information processing device 100 encodes the reference codon sequence data 140a and the analysis-target codon sequence data 140b, and generates the first-type sequence data 140d and the second-type sequence data 140e, respectively, (Step S102).

The comparing unit 150c of the information processing device 100 compares the first-type sequence data 140d and the second-type sequence data 140e in the units of codons (single bytes), and identifies mutation positions at which the codons are not identical (Step S103). Then, based on each mutation position, the comparing unit 150c identifies the first-type mutant codon, the mutant n codon, and the mutant n+1 codon in the first-type sequence data 140d; and identifies the second-type mutation codon, the mutation n codon, and the mutation n+1 codon in the second-type sequence data 140e (Step S104).

The identifying unit 150d of the information processing device 100 determines whether or not, in the insertion transition table 140f, the pre-base-insertion mutant n codon, which is identified from the mutation n codon and the mutation n+1 codon, is identical to the subsequent codon of the first-type mutant codon (Step S105). If the two codons are identical (Yes at Step S105), then the identifying unit 150d identifies "base insertion" as the type of point mutation (Step S106). On the other hand, if the two codons are not identical (No at Step S105), then the system control proceeds to Step S107.

The following explanation is given about Step S107. The identifying unit 150d determines whether or not, in the deletion transition table 140g, the pre-base-insertion mutant n codon, which is identified from the mutation n codon and the mutation n+1 codon, is identical to the codon positioned after the subsequent codon of the first-type mutant codon (Step S107). If the two codons are identical (Yes at Step S107), then the identifying unit 150d identifies "base deletion" as the type of point mutation (Step S108).

On the other hand, if the two codons are not identical (No at Step S107), then the identifying unit 150d identifies "base substitution" as the type of point mutation (Step S109).

Then, the identifying unit 150d registers the information about the identified type of point mutation in the detection result table 140h (Step S110). The information processing device 100 outputs the detection result table 140h to the display unit 130 (Step S111).

Given below is the explanation of the effects achieved in the information processing device 100 according to the first embodiment. The information processing device 100 compares the first-type sequence data 140d and the second-type sequence data 140e in the units of one-byte codons, and identifies nonidentical codons (nonidentical encoded codons). Then, the information processing device 100 compares the transition destination codon, for which the nonidentical codons serve as the mutation position, with the insertion transition table 140f and the deletion transition table 140g, and identifies the type of point mutation included in the analysis-target codon sequence data. Thus, as a result of performing comparison in the units of encoded codons in a consistent manner, the type of mutation can be determined while identifying the nonidentical codons. That enables achieving reduction in the time requested in determining the type of mutation.

[Second embodiment]

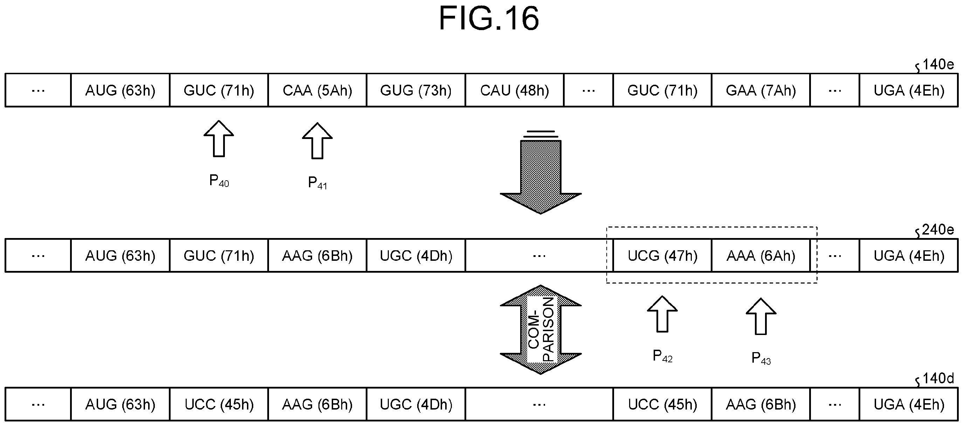

FIGS. 16 to 18 are diagrams for explaining the operations performed in an information processing device according to a second embodiment. With reference to FIG. 16, the explanation is given about the operations performed when point mutation of the "base insertion" type is detected. In an identical manner to the information processing device 100 according to the first embodiment, the information processing device according to the second embodiment compares the first-type sequence data 140d and the second-type sequence data 140e, and identifies a mutation position $P_{40}$ at which the codons are not identical. Regarding the mutation codon "GUC (71h)" at the mutation position $P_{40}$, the information processing device compares the mutation n codon "CAA (5Ah)" and the mutation n+1 codon "GUG (73h)" with the insertion transition table 140f; and identifies the pre-base-insertion mutant n codon "AAG (68h)". Then, the information processing device performs correction by substituting the codon "CAA (5Ah)", which is the subsequent codon of the mutation codon, with the pre-base-insertion mutant n codon "AAG (68h)".

The information processing device shifts the mutation position $P_{40}$ to the sequence position of the subsequent codon. That position is referred to as a sequence position $P_{41}$. Regarding the sequence position $P_{41}$, the information processing device compares the mutation n codon "GUG (73h)" and the mutation n+1 codon "CAU (48h)" with the insertion transition table 140f; and identifies the pre-base-insertion mutant n codon "UGC (4Dh)". Then, the information processing device performs correction by substituting the codon "GUG (73h)", which is the subsequent codon of the mutation codon, with the codon "UGC (4Dh)", which is the subsequent codon of the pre-base-insertion mutant codon.

As explained above, while shifting the sequence position, the information processing device repeatedly performs the operation of substituting the mutation n codon with the pre-base-insertion mutant n codon, and generates third-type sequence data 240e.

Then, the information processing device compares the encoded codons in the third-type sequence data 240e with the encoded codons in the first-type sequence data 140d, and identifies the nonidentical codons. The information processing device identifies the nonidentical codons as the underlying genetic mutation. In the example illustrated in FIG. 16, the information processing device identifies the codon "UCG (47h)" at a sequence position $P_{42}$ and the codon "AAA (6Ah)" at a sequence position $P_{43}$ as genetic mutation.

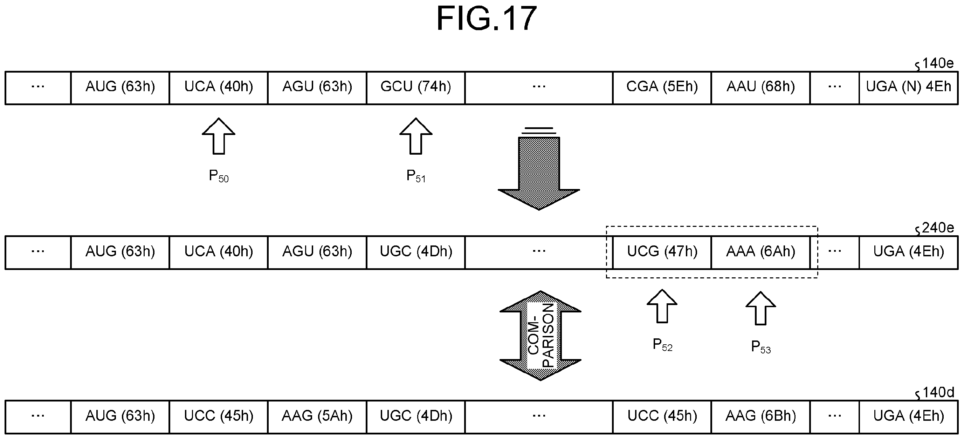

Explained below with reference to FIG. 17 are the operations performed when point mutation of the "base deletion" type is detected. In an identical manner to the information processing device 100 according to the first embodiment, the information processing device according to the second embodiment compares the first-type sequence data 140d and the second-type sequence data 140e, and identifies a mutation position $P_{50}$ at which the codons are not identical. Regarding the mutation codon "UCA (40h)" at the mutation position $P_{50}$, the information processing device compares the mutation n codon "AUG (63h)" and the mutation n+1 codon "GCU (74h)" with the deletion transition table 140g; and identifies the pre-base-deletion mutant n+1 codon "UGC (4Dh)". Then, the information processing device performs correction by substituting the codon "GCU (74h)", which is the codon positioned after the subsequent codon of the mutation codon, with the pre-base-deletion mutant n+1 codon "UGC (4Dh)".

Although not illustrated in FIG. 17, the information processing device shifts the mutation position $P_{50}$ to the sequence position of the subsequent codon. Then, based on the new sequence position, the information processing device compares the mutation n codon and the mutation n+1 codon with the deletion transition table 140g; and identifies the pre-base-deletion mutant n+1 codon. Subsequently, the information processing device performs correction by substituting the mutation n+1 codon with the pre-base-deletion mutant n+1 codon.

As explained above, while shifting the sequence position, the information processing device repeatedly performs the operation of substituting the mutation n+1 codon with the pre-base-deletion mutant n+1 codon, and generates the third-type sequence data 240e.

Then, the information processing device compares the encoded codons in the third-type sequence data 240e and the encoded codons in the first-type sequence data 140d, and identifies the nonidentical codons. The information processing device identifies the nonidentical codons as the underlying genetic mutation. In the example illustrated in FIG. 17, the information processing device identifies the codon "UCG (47h)" at a sequence position $P_{52}$ and the codon

"AAA (6Ah)" at a sequence position $P_{53}$ as genetic mutation.

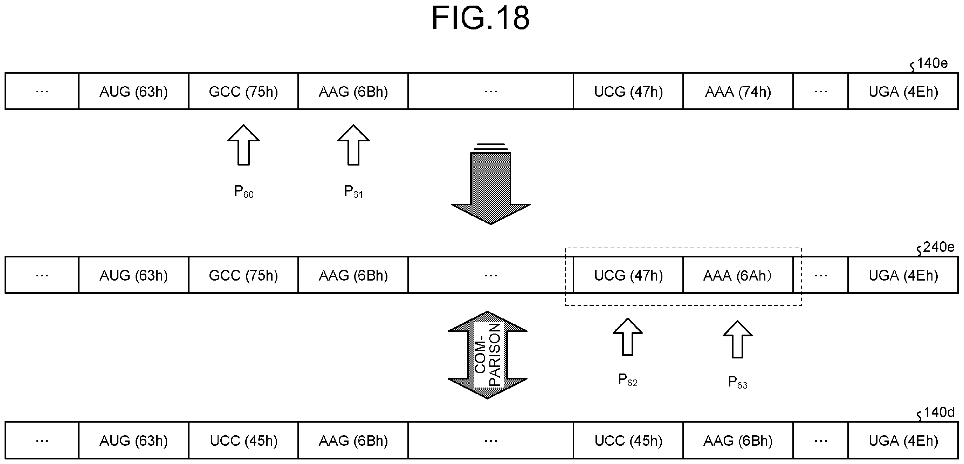

Explained below with reference to FIG. 18 are the operations performed when point mutation of the "base substitution" type is detected. In an identical manner to the information processing device 100 according to the first embodiment, the information processing device according to the second embodiment compares the first-type sequence data 140d and the second-type sequence data 140e, and identifies a mutation position $P_{60}$ at which the codons are not identical. Then, assume that the information processing device determines "base substitution" as the type of point mutation by referring to the insertion transition table 140f and the deletion transition table 140g. In that case, the information processing device copies the codons from the codon at a sequence position $P_{61}$, which is the subsequent position to the mutation codon at the mutation position $P_{60}$ in the second-type sequence data 140e, onward and generates the third-type sequence data 240e.

The information processing device compares the encoded codons in the third-type sequence data 240e with the encoded codons in the first-type sequence data 140d, and identifies the nonidentical codons. The information processing device identifies the nonidentical codons as the underlying genetic mutation. In the example illustrated in FIG. 18, the information processing device identifies the codon "UCG (47h)" at a sequence position $P_{62}$ and the codon "AAA (6Ah)" at a sequence position $P_{63}$ as genetic mutation.

As explained above, after identifying the type of point mutation, the information processing device according to the second embodiment generates the third-type sequence data 240e by correcting the second-type sequence data 140e and identifies the nonidentical codons between the first-type sequence data 140d and the third-type sequence data 240e. As a result, the underlying genetic mutation can be detected.

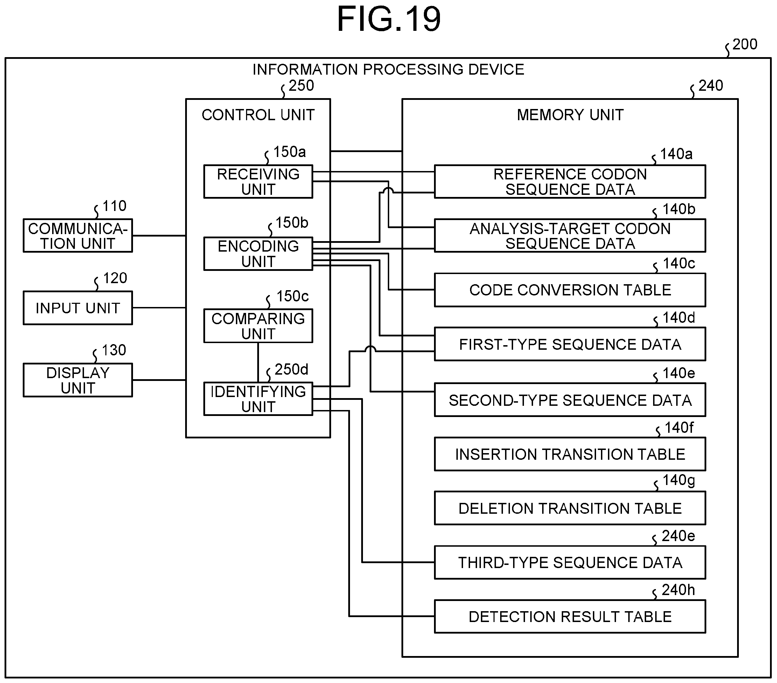

Given below is the explanation of a configuration of the information processing device according to the second embodiment. FIG. 19 is a functional block diagram illustrating a configuration of the information processing device according to the second embodiment. As illustrated in FIG. 19, an information processing device 200 includes the communication unit 110, the input unit 120, the display unit 130, a memory unit 240, and a control unit 250. Herein, regarding the communication unit 110, the input unit 120, and the display unit 130; the explanation is identical to the explanation of the communication unit 110, the input unit 120, and the display unit 130 given with reference to FIG. 5.

The memory unit 240 is used to store the reference codon sequence data 140a, the analysis-target codon sequence data 140b, the code conversion table 140c, the first-type sequence data 140d, and the second-type sequence data 140e. Moreover, the memory unit 240 is used to store the insertion transition table 140f, the deletion transition table 140g, the third-type sequence data 240e, and a detection result table 240h. Examples of the memory unit 240 include a semiconductor memory such as a RAM, a ROM, or a flash memory; and a memory device such as an HDD.

Regarding the reference codon sequence data 140a, the analysis-target codon sequence data 140b, the code conversion table 140c, the first-type sequence data 140d, and the second-type sequence data 140e stored in the memory unit 240; the explanation is identical to the explanation given in the first embodiment. Moreover, regarding the insertion transition table 140f and the deletion transition table 140g stored in the memory unit 240, the explanation is identical to the explanation given in the first embodiment.

The third-type sequence data 240e represents sequence data in which, from among the encoded codons in the second-type sequence data 140e, the codons corresponding to point mutation are corrected to normal codons.

The detection result table 240h is a table for holding the information about point mutation and genetic mutation detected from the analysis-target codon sequence data 140b.

The control unit 250 includes the receiving unit 150a, the encoding unit 150b, the comparing unit 150c, and an identifying unit 250d. The control unit 250 is implemented using a CPU or an MPU. Alternatively, the control unit 250 can be implemented using a hardwired logic such as an ASIC or an FPGA.

The receiving unit 150a is a processing unit that receives the reference codon sequence data 140a and the analysis-target codon sequence data 140b from the input unit 120 or an external device. Then, the receiving unit 150a registers the reference codon sequence data 140a and the analysis-target codon sequence data 140b in the memory unit 240. Besides that, the operations of the receiving unit 150a are identical to the explanation according to the first embodiment.

The encoding unit 150b is a processing unit that encodes the reference codon sequence data 140a and the analysis-target codon sequence data 140b based on the code conversion table 140c. Besides that, the operations of the encoding unit 150b are identical to the explanation according to the first embodiment.

The comparing unit 150c is a processing unit that compares the first-type sequence data 140d and the second-type sequence data 140e, and identifies mutation positions at which the encoded codons are not identical. Then, the comparing unit 150c outputs the comparison result to the identifying unit 250d. Besides that, the operations of the comparing unit 150c are identical to the explanation according to the first embodiment.

The identifying unit 250d identifies the type of point mutation, which has occurred at a mutation position, based on the comparison result of the comparing unit 150c, the insertion transition table 140f, and the deletion transition table 140g. Once the type of point mutation is identified, the identifying unit 250d generates the third-type sequence data 240e by correcting the second-type sequence data 140e. Then, the identifying unit 250d compares the first-type sequence data 140d and the third-type sequence data 240e, and detects genetic mutation. The identifying unit 250d registers the information about the mutation position, the type of point mutation, and the genetic mutation in the detection result table 240h.

Regarding the identifying unit 250d, the operations for identifying the type of point mutation are identical to the operations performed by the identifying unit 150d according to the first embodiment. In the following explanation, the operations performed by the identifying unit 250d are separately explained for the cases in which point mutation of the "base insertion" type is detected, point mutation of the "base deletion" type is detected, and point mutation of the "base substitution" type is detected.

Given below is the explanation of the operations performed by the identifying unit 250d performed when point mutation of the "base insertion" type is detected. As explained with reference to FIG. 16, regarding the mutation codon "GUC (71h)" at the mutation position $P_{40}$, the identifying unit 250d compares the mutation n codon "CAA (5Ah)" and the mutation n+1 codon "GUG (73h)" with the insertion transition table 140f; and identifies the pre-base-insertion mutant n codon "AAG (6Bh)". Then, the identifying unit 250d performs correction by substituting the codon "CAA (5Ah)", which is the subsequent codon of the mutant codon, with the pre-base-insertion mutant n codon "AAG (6Bh)".

Subsequently, the identifying unit 250d shifts the mutation position $P_{40}$ to the subsequent sequence position. That position is referred to as the sequence position $P_{41}$. Regarding the sequence position $P_{41}$, the identifying unit 250d compares the mutation n codon "GUG (73h)" and the mutation n+1 codon "CAU (48h)" with the insertion transition table 140f; and identifies the pre-base-insertion mutant n codon "UGC (4Dh))". Then, the identifying unit 250d performs correction by substituting the codon "GUG (73h)", which is the codon positioned after the subsequent codon of the mutation codon, with the codon "UGC (4Dh)", which is the pre-base-insertion mutant n codon.

As explained above, while shifting the sequence position, the identifying unit 250d repeatedly performs the operation of substituting the mutation n codon with the pre-base-insertion mutant n codon, and generates the third-type sequence data 240e.

Then, the identifying unit 250d compares the encoded codons in the third-type sequence data 240e with the encoded codons in the first-type sequence data 140d, and identifies the nonidentical codons. The identifying unit 250d identifies the nonidentical codons as the underlying genetic mutation. In the example illustrated in FIG. 16, the information processing device identifies the codon "UCG (47h)" at the sequence position $P_{42}$ and the codon "AAA (6Ah)" at the sequence position $P_{43}$ as genetic mutation.

Then, in the detection result table 240h, the identifying unit 250d registers the information indicating "base insertion" as the type of point mutation and indicating the mutation position, as well as registers the information about the codons identified as the genetic mutation and their sequence positions.

Given below is the explanation about the operations performed by the identifying unit 250d when point mutation of the "base deletion" type is detected. With reference to FIG. 17, the identifying unit 250d compares the first-type sequence data 140d and the second-type sequence data 140e, and identifies the mutation position $P_{50}$ at which the codons are not identical. Regarding the mutation codon "UCA (40h)" at the mutation position $P_{50}$, the identifying unit 250d compares the mutation n codon "AGU (63h)" and the mutation n+1 codon "GCU (74h)" with the deletion transition table 140g; and identifies the pre-base-deletion mutant n+1 codon "UGC

(4Dh)". Then, the information processing device performs correction by substituting the codon "GCU (74h)", which is the codon positioned after the subsequent codon of the mutation codon, with the pre-base-deletion mutant n+1 codon "UGC (4Dh)".

Although not illustrated in FIG. 17, the identifying unit 250d shifts the mutation position $P_{50}$ to the subsequent sequence position. Then, based on the new sequence position, the identifying unit 250d compares the mutation n codon and the mutation n+1 codon with the deletion transition table 140g; and identifies the pre-base-deletion mutant n+1 codon. Subsequently, the identifying unit 250d performs correction by substituting the mutation n+1 codon with the pre-base-deletion mutant n+1 codon.

As explained above, while shifting the sequence position, the identifying unit 250d repeatedly performs the operation of substituting the mutation n+1 codon with the pre-base-deletion mutant n+1 codon, and generates the third-type sequence data 240e.

The identifying unit 250d compares the encoded codons in the third-type sequence data 240e and the encoded codons in the first-type sequence data 140d, and identifies the nonidentical codons. The identifying unit 250d identifies the nonidentical codons as the underlying genetic mutation. In the example illustrated in FIG. 17, the identifying unit 250d identifies the codon "UCG (47h)" at the sequence position $P_{52}$ and the codon "AAA (6Ah)" at the sequence position $P_{53}$ as genetic mutation.

Then, in the detection result table 240h, the identifying unit 250d registers the information indicating "base deletion" as the type of point mutation and indicating the mutation position, as well as registers the information about the codons identified as the genetic mutation and their sequence positions.

Given below is the explanation about the operations performed by the identifying unit 250d when point mutation of the "base substitution" type is detected. With reference to FIG. 18, the identifying unit 250d compares the first-type sequence data 140d and the second-type sequence data 140e, and identifies the mutation position $P_{60}$ at which the codons are not identical. Then, assume that the identifying unit 250d determines "base substitution" as the type of point mutation by referring to the insertion transition table 140f and the deletion transition table 140g. In that case, the identifying unit 250d copies the codons from the codon at the sequence position $P_{61}$, which is the subsequent position to the mutation codon at the mutation position $P_{60}$ in the second-type sequence data 140e, onward and generates the third-type sequence data 240e.

The identifying unit 250d compares the encoded codons in the third-type sequence data 240e with the encoded codons in the first-type sequence data 140d, and identifies the nonidentical codons. The identifying unit 250d identifies the nonidentical codons as the underlying genetic mutation. In the example illustrated in FIG. 18, the identifying unit 250d identifies the codon "UCG (47h)" at the sequence position $P_{62}$ and the codon "AAA (6Ah)" at the sequence position $P_{63}$ as genetic mutation.

Then, in the detection result table 240h, the identifying unit 250d registers the information indicating "base substitution" as the type of point mutation and indicating the mutation position, as well as registers the information about the codons identified as the genetic mutation and their sequence positions.

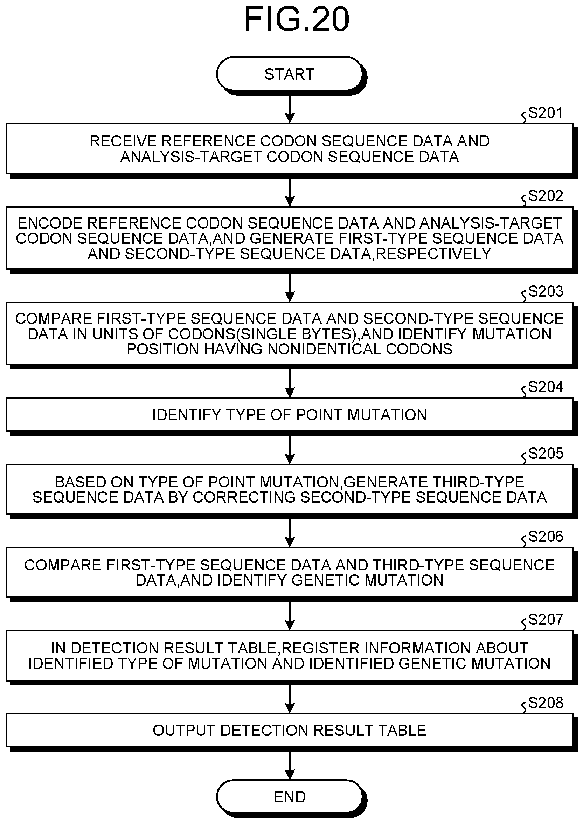

Given below is the explanation of an exemplary sequence of operations performed in the information processing device 200 according to the second embodiment. FIG. 20 is a flowchart (1) for explaining a sequence of operations performed in the information processing device according to the second embodiment. As illustrated in FIG. 20, the receiving unit 150a of the information processing device 200 receives the reference codon sequence data 140a and the analysis-target codon sequence data 140b (Step S201) .

The encoding unit 150b of the information processing device 200 encodes the reference codon sequence data 140a and the analysis-target codon sequence data 140b, and generates the first-type sequence data 140d and the second-type sequence data 140e, respectively, (Step S202).

The comparing unit 150c of the information processing device 200 compares the first-type sequence data 140d and the second-type sequence data 140e in the units of codons (single bytes), and identifies mutation positions at which the codons are not identical (Step S203). Then, the identifying unit 250d of the information processing device 200 identifies the type of point mutation (Step S204). The sequence of operations performed for identifying the type of point mutation is same as the sequence of operations performed from Step S105 to Step S109 illustrated in FIG. 15.

Based on the type of point mutation, the identifying unit 250d generates the third-type sequence data 240e by correcting the second-type sequence data 140e (Step S205). Then, the identifying unit 250d compares the first-type sequence data 140d and the third-type sequence data 240e, and identifies genetic mutation (Step S206).

Subsequently, the identifying unit 250d registers the information indicating the identified type of mutation and the identified genetic mutation in the detection result table 250h (Step S207). The information processing device 200 outputs the detection result table 240h to the display unit 130 (Step S208).

Given below is the explanation about the effects achieved in the information processing device 200 according to the second embodiment. After identifying the type of point mutation included in the second-type sequence data 140e, the information processing device 200 generates the third-type sequence data 240e by correcting the second-type sequence data 140e; and identifies nonidentical codons between the first-type sequence data 140d and the third-type sequence data 240e. As a result, even after the determination of the type of point mutation, as a result of performing comparison in the units of encoded codons in a consistent manner, the underlying genetic mutation can be detected.

For the purpose of illustration, the explanation is given about the case in which the information processing device 200 according to the second embodiment generates the third-type sequence data 240e, and compares it with the first-type sequence data 140d. However, that is not the only possible case. Alternatively, instead of generating the third-type sequence data 240e, the information processing device 200 can convert the second-type sequence data 140e into the units of bytes, and compare the conversion result with the first-type sequence data 140d in the units of bytes.

Given below is the explanation of the other operations performed in the information processing device 200 according to the second embodiment. When the input of a search query is an amino-acid sequence, the information processing device 200 performs codon-amino acid conversion based on the first-type sequence data 140d that is obtained by encoding the reference codon sequence data 140a written using base symbols; and generates fourth-type sequence data (not illustrated in the drawings). Then, the information processing device 200 compares, in the units of amino acids, the fourth-type sequence data, which is obtained as a result of codon-amino acid conversion, with the amino-acid sequence specified in the search query; and identifies mutation positions.

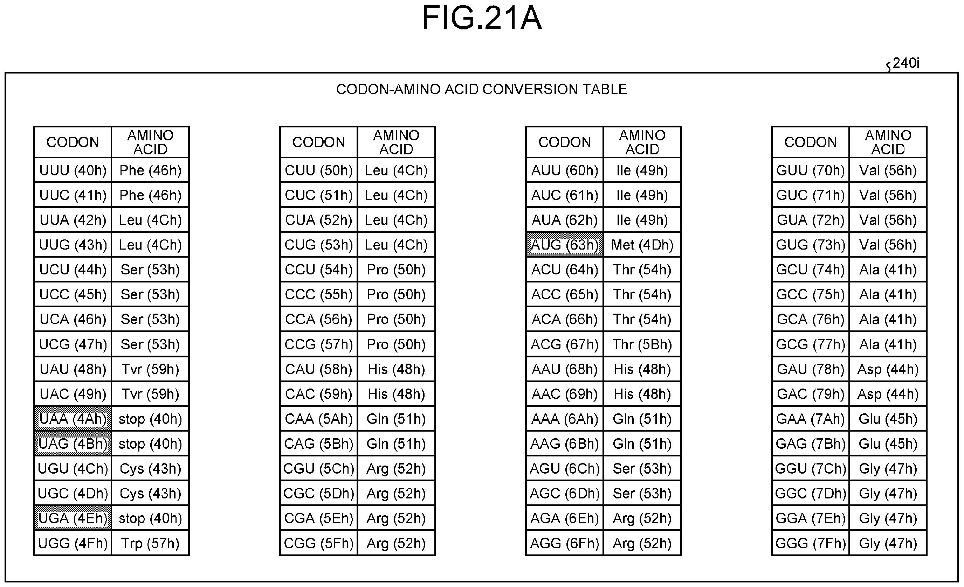

FIG. 21A is a diagram illustrating an exemplary data structure of the codon-amino acid conversion table. As illustrated in FIG. 21A, in a codon-amino acid conversion table 240i, encoded codons and encoded amino acids are held in a corresponding manner. For example, the encoded codon "UUU (40h)" is associated to the encoded amino acid "Phe (50h)". Although not illustrated in FIG. 19, the codon-amino acid conversion table 240i is stored in the memory unit 240 of the information processing device 200.

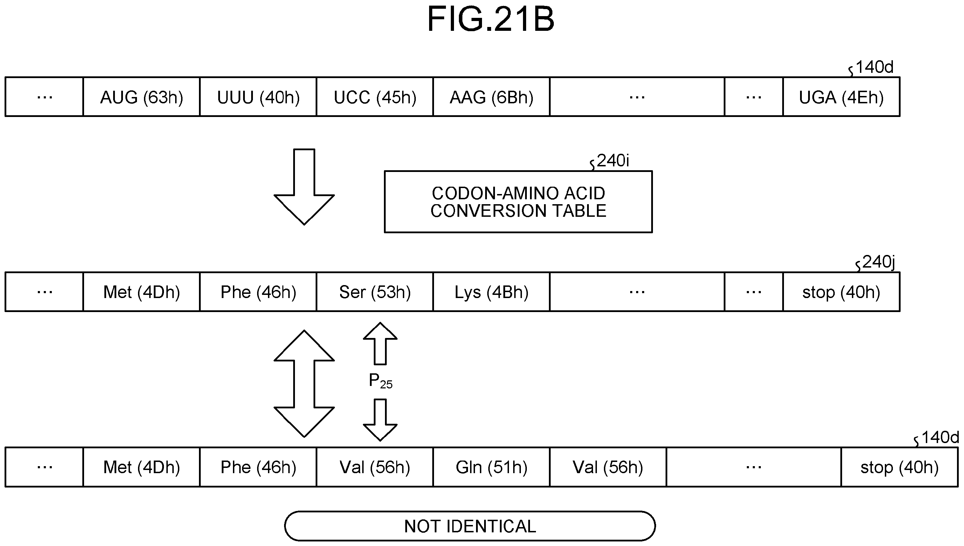

FIG. 21B is a diagram for explaining the other operations performed in the information processing device according to the second embodiment. As illustrated in FIG. 21B, the information processing device 200 compares the first-type sequence data 140d and the codon-amino acid conversion table 240i; converts the encoded codons into encoded amino acids; and generates fourth-type sequence data 240j. For example, the codon "AUG (63h)" is converted into the amino acid "Met (4Dh)". Although not illustrated in FIG. 19, the fourth-type sequence data 240j is stored in the memory unit 240 of the information processing device 200.

Then, the information processing device 200 compares the fourth-type sequence data 240j and the second-type sequence data 140e, and identifies mutation positions at which the amino acids are not identical. In the example illustrated in FIG. 21B, it is determined that the amino acids are not identical from a sequence position $P_{25}$ onward.

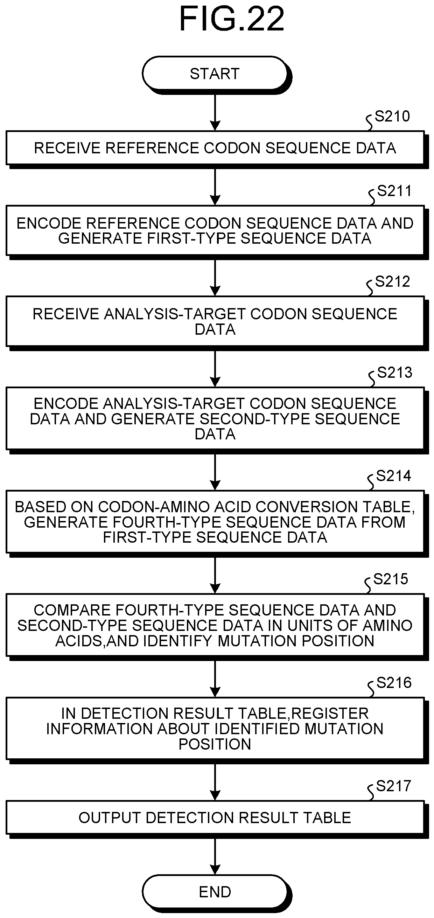

Given below is the explanation of an exemplary sequence of operations performed in the information processing device 200 according to the second embodiment when the input of a search query is an amino-acid sequence. FIG. 22 is a flowchart (2) for explaining a sequence of operations performed in the information processing device according to the second embodiment. As illustrated in FIG. 22, the receiving unit 150a of the information processing device 200 receives the reference codon sequence data (Step S210). Then, the encoding unit 150b of the information processing device 200 encodes the reference codon sequence data 140a and generates the first-type sequence data 140d (Step S211).

The receiving unit 150a receives the amino-acid sequence data to be analyzed (Step S212). Then, the encoding unit 150b encodes the amino-acid sequence data to be analyzed, and generates the second-type sequence data 140e (Step S213). At Step S213, the encoding unit 150b converts the amino acid conversion data, which is to be analyzed, into the second-type sequence data 140e based on the code conversion table 140c. Although the specific explanation is not given, it is assumed that the code conversion table 140c is used to hold the amino acids and the encoded amino acids in a corresponding manner.

Then, based on the codon-amino acid conversion table 240i, the comparing unit 150c of the information processing device 200 generates the fourth-type sequence data 240j from the first-type sequence data 140d (Step S214). Subsequently, the comparing unit 150c compares the fourth-type sequence data 240j and the second-type sequence data 140e in the units of amino acids, and identifies mutation positions (Step S215).

The information processing device 200 registers the information about the mutation positions, which are identified by the comparing unit 150c, in the detection result table 240h (Step S216). Then, the information processing device 200 outputs the detection result table 240h to the display unit 130 (Step S217).

In this way, when the input of a search query is an amino-acid sequence, the information processing device 200 performs codon-amino acid conversion based on the first-type sequence data 140d, which is obtained by encoding the reference codon sequence data 140a written using base symbols, and compares the conversion result with the search query. Thus, even when the input of a search query is an amino-acid sequence, it becomes possible to identify the amino acids in which mutation has occurred.

[Third embodiment]

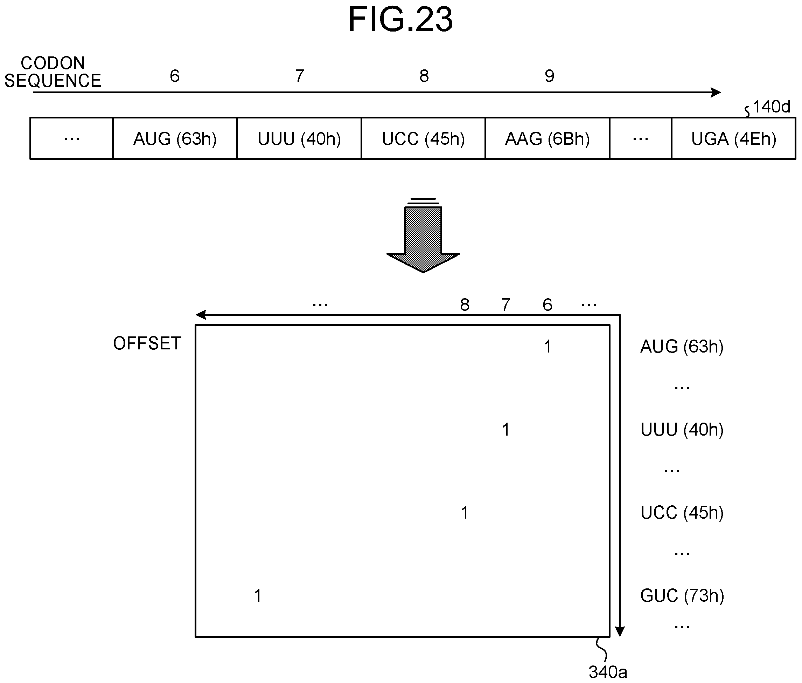

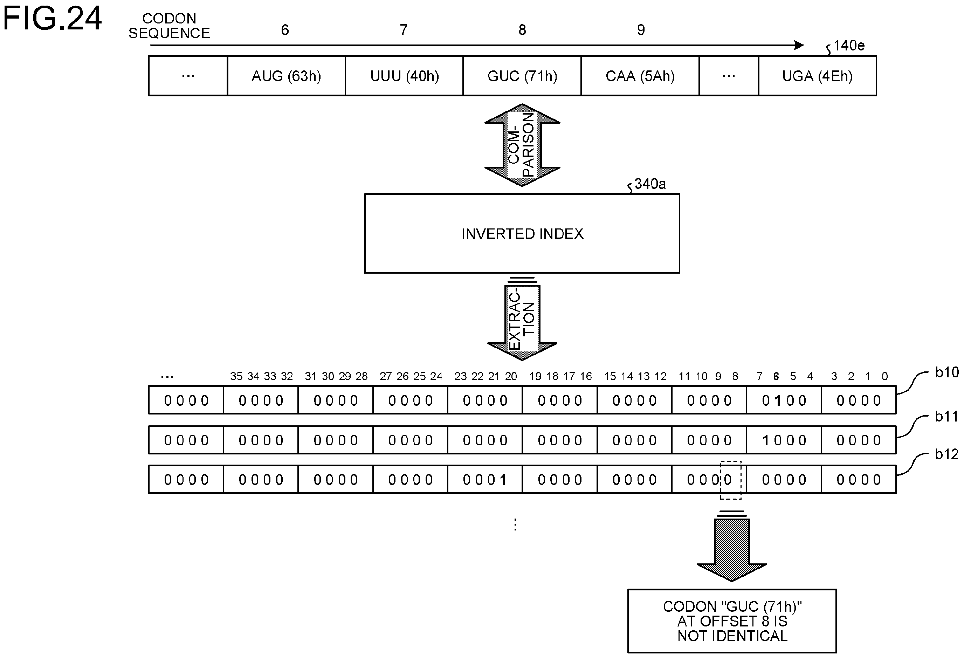

FIGS. 23 and 24 are diagrams for explaining the operations performed in an information processing device according to a third embodiment. Although not illustrated in FIGS. 23 and 24, in an identical manner to the information processing device 100 according to the first embodiment, upon receiving the reference codon sequence data 140a, the information processing device according to the third embodiment encodes the reference codon sequence data 140a based on the code conversion table 140c and generates the first-type sequence data 140d; as well as generates an inverted index 340a at the same time. Moreover, upon receiving the analysis-target codon sequence data 140b to be analyzed, the information processing device performs encoding based on the code conversion table 140c and generates the second-type sequence data 140e.

The following explanation is given regarding FIG. 23. At the same time of generating the first-type sequence data 140d, the information processing device according to the third embodiment generates the inverted index 340a. The inverted index 340a represents information indicating the relationship between the types of the encoded codes, which are included in the first-type sequence data 140d, and the sequence positions (offsets) using bitmaps.

The horizontal axis of the inverted index 340a corresponds to the offsets. The vertical axis of the inverted index 340a corresponds to the types of the encoded codons. The inverted index 340a is illustrated using bitmaps of "0" and "1"; and, in the initial state, all bitmaps are set to "0".

Herein, the offset implies the offset from the first codon included in the sequence data. In the third embodiment, the first codon is assumed to have the offset of "0". For example, regarding the first-type sequence data 140d, if the codon "AUG (63h)" is the seventh codon from the beginning, then it has the offset of "6".

The information processing device scans the first-type sequence data 140d from the beginning; identifies the relationship between the types of the encoded codons and the offsets; and sets "1" at corresponding positions in the inverted index 340a. For example, since the codon "AUG (63h)" is present at the offset "6", the information processing device sets "1" at the intersecting position of the column of the offset "6" and the row of the codon type "AUG (63h)". The information processing device performs such operations in a repeated manner and generates the inverted index 340a.

The following explanation is given regarding FIG. 24. The information processing device sequentially reads the encoded codons from the start codon in the second-type sequence data 140e and obtains, from the inverted index 340a, the bitmaps corresponding to the types of the read codons. Herein, for example, "AUG (63h)" represents the start codon.

The information processing device obtains, from the inverted index 340a, a bitmap b10 of the codon "AUG (63h)", a bitmap b11 of the codon "UUU (40h)", a bitmap b12 of the codon "GUC (71h)", and so on in a sequential manner. The bitmap b10 is the bitmap corresponding to the row of the codon type "AUG (63h)" in the inverted index 340a. The bitmap b11 is the bitmap corresponding to the row of the codon type "UUU (40h)" in the inverted index 340a. The bitmap b12 is the bitmap corresponding to the row of the codon type "GUC (71h)" in the inverted index 340a.

The information processing device focuses on the positions of "1" in the bitmap b10 to b12 and, as long as the position of "1" shifts to the left side by one offset in sequence, determines that the codons are identical in the first-type sequence data 140d and the second-type sequence data 140e. When the position of "1" stops shifting to the left side by one offset in sequence, the information processing device determines that the codons are not identical in the first-type sequence data 140d and the second-type sequence data 140e. In the example illustrated in FIG. 24, in the step from the bitmap b11 to the bitmap b12, the position of "1" has shifted from the offset "7" to the offset "20". Hence, non-identicalness is identified regarding the codon "GUC (71h)" at the offset (sequence position) "8".

As explained above, the information processing device according to the third embodiment generates the inverted index 340a based on the first-type sequence data 140d. The information processing device obtains, from the inverted index 340a, the bitmaps corresponding to the codon types in a sequential manner from the first codon included in the second-type sequence data 140e; and identifies nonidentical codons based on the positions of the flag "1" in a plurality of obtained bitmaps. As a result, it becomes possible to perform a high-speed search for the codons having point mutation.

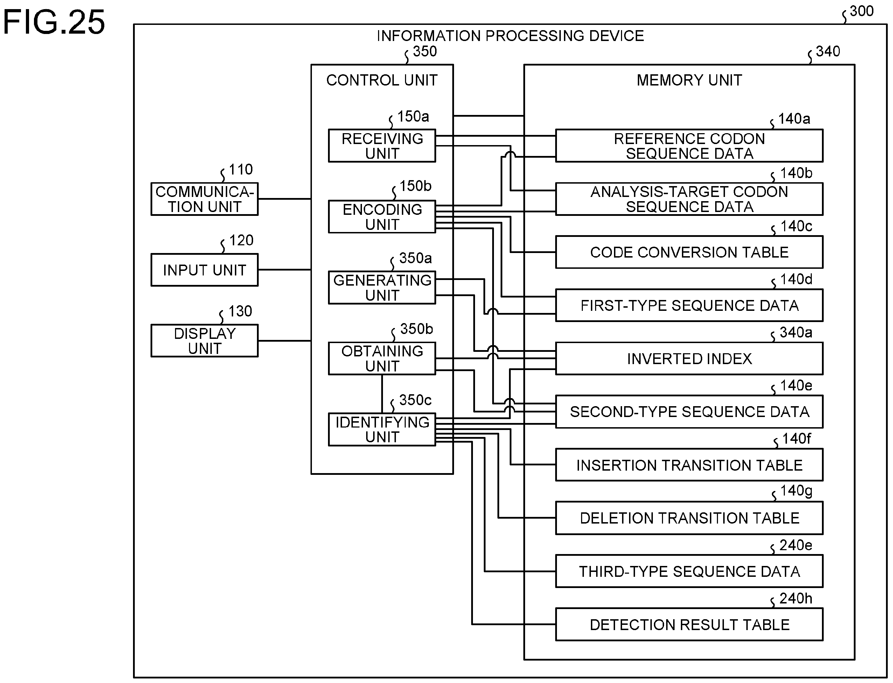

Given below is the explanation of a configuration of the information processing device according to the third embodiment. FIG. 25 is a functional block diagram illustrating a configuration of the information processing device according to the third embodiment. As illustrated in FIG. 25, an information processing device 300 includes the communication unit 110, the input unit 120, the display unit 130, a memory unit 340, and a control unit 350. Herein, regarding the communication unit 110, the input unit 120, and the display unit 130; the explanation is identical to the explanation of the communication unit 110, the input unit 120, and the display unit 130 given with reference to FIG. 5.

The memory unit 340 is used to store the reference codon sequence data 140a, the analysis-target codon sequence data 140b, the code conversion table 140c, the first-type sequence data 140d, the inverted index 340a, and the second-type sequence data 140e. Moreover, the memory unit 340 is used to store the insertion transition table 140f, the deletion transition table 140g, the third-type sequence data 240e, and the detection result table 240h. Examples of the memory unit 340 include a semiconductor memory such as a RAM, a ROM, or a flash memory; and a memory device such as an HDD. Meanwhile, although not illustrated in FIG. 25, the memory unit 340 can also be used to store the codon-amino acid conversion table 240i and the fourth-type sequence data 240j.

Regarding the reference codon sequence data 140a, the analysis-target codon sequence data 140b, the code conversion table 140c, the first-type sequence data 140d, and the second-type sequence data 140e stored in the memory unit 240; the explanation is identical to the explanation given in the first embodiment. Moreover, regarding the insertion transition table 140f and the deletion transition table 140g stored in the memory unit 340, the explanation is identical to the explanation given in the first embodiment. Furthermore, regarding the third-type sequence data 240e and the detection result table 240h stored in the memory unit 340, the explanation is identical to the explanation given in the second embodiment.

The inverted index 340a represents information indicating the relationship between the types of the encoded codes, which are included in the first-type sequence data 140d, and the sequence positions (offsets) using bitmaps. As explained with reference to FIG. 23, the horizontal axis of the inverted index 340a corresponds to the offsets. The vertical axis of the inverted index 340a corresponds to the types of the encoded codons.

The control unit 350 includes the receiving unit 150a, the encoding unit 150b, a generating unit 350a, an obtaining unit 350b, and an identifying unit 350c. The control unit 350 is implemented using a CPU or an MPU. Alternatively, the control unit 350 can be implemented using a hardwired logic such as an ASIC or an FPGA.

The receiving unit 150a is a processing unit that receives the reference codon sequence data 140a and the analysis-target codon sequence data 140b from the input unit 120 or an external device. Then, the receiving unit 150a registers the reference codon sequence data 140a and the analysis-target codon sequence data 140b in the memory unit 340. Besides that, the operations of the receiving unit 150a are identical to the explanation according to the first embodiment.

The encoding unit 150b is a processing unit that encodes the reference codon sequence data 140a and the analysis-target codon sequence data 140b based on the code conversion table 140c. Besides that, the operations of the encoding unit 150b are identical to the explanation according to the first embodiment.

The generating unit 350a is a processing unit that generates the inverted index 340a based on the first-type sequence data 140d. The generating unit 350a scans the first-type sequence data 140d from the beginning; identifies the relationship between the types of the encoded codons and the offsets (sequence positions); and sets "1" at the corresponding locations in the inverted index 340a. For example, since the codon "AUG (63h)" is present at the offset "6", the generating unit 350a sets "1" at the intersecting position of the column of the offset "6" and the row of the codon type "AUG (63h)". The generating unit 350a performs such operations in a repeated manner and generates the inverted index 340a.

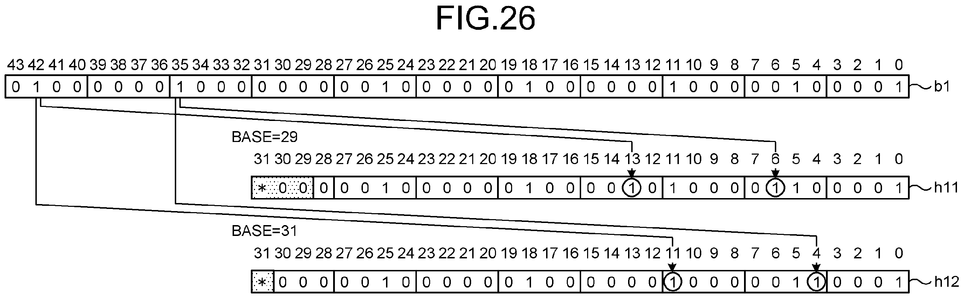

Upon generating the inverted index 340a, in order to reduce the information volume, the generating unit 350a can perform hashing of the inverted index 340a. FIG. 26 is a diagram for explaining an example of the operations for hashing an inverted index.

In the example illustrated in FIG. 26, a 32-bit register is taken into consideration and, based on the prime numbers (bases) "29" and "31", the bitmaps of each row in the inverted index 340a are hashed. Herein, as an example, the explanation is given about a case in which hashed bitmaps h11 and h12 are generated from the bitmap b1.

The bitmap b1 represents a bitmap obtained by extracting a particular row of an inverted index (for example, the inverted index 340a illustrated in FIG. 23). A hashed bitmap h11 is a bitmap hashed using the base "29". A hashed bitmap h12 is a bitmap hashed using the base "31".

The generating unit 350a associates, to the positions in the hashed bitmap, the values obtained as the remainders when the positions of the bits of the bitmap b1 are divided by a single base. When "1" is set at the position of a bit in the bitmap b1, the generating unit 350a sets "1" at the corresponding position in the hashed bitmap.

Given below is the explanation of an example of the operations performed to generate the hashed bitmap h11 having the base "29" from the bitmap b1. Firstly, the generating unit 350a copies the information about the positions "0 to 28" of the bitmap b1 in the hashed bitmap h11. Subsequently, if the bit position "35" in the bitmap b1 is divided by the base "29", the remainder is equal to "6". Hence, the position "35" in the bitmap b1 is associated to the position "6" in the hashed bitmap h11. Since "1" is set at the position "35" in the bitmap b1, the generating unit 350a sets "1" at the position "6" in the hashed bitmap h11.

If the bit position "42" in the bitmap b1 is divided by the base "29", the remainder is equal to "13". Hence, the position "42" in the bitmap b1 is associated to the position "13" in the hashed bitmap h11. Since "1" is set at the position "42" in the bitmap b1, the generating unit 350a sets "1" at the position "13" in the hashed bitmap h11.

Regarding the positions from the position "29" onward in the bitmap b1, the generating unit 350a repeatedly performs the operations explained above and generates the hashed bitmap h11.

Given below is the explanation of an example of the operations performed to generate the hashed bitmap h12 having the base "31" from the bitmap b1. Firstly, the generating unit 350a copies the information about the positions "0 to 30" of the bitmap b1 in the hashed bitmap h12. Subsequently, if the bit position "35" in the bitmap b1 is divided by the base "31", the remainder is equal to "4". Hence, the position "35" in the bitmap b1 is associated to the position "4" in the hashed bitmap h12. Since "1" is set at the position "35" in the bitmap b1, the generating unit 350a sets "1" at the position "4" in the hashed bitmap h12.

If the bit position "42" in the bitmap b1 is divided by the base "31", the remainder is equal to "11". Hence, the position "42" in the bitmap b1 is associated to the position "11" in the hashed bitmap h12. Since "1" is set at the position "42" in the bitmap b1, the generating unit 350a sets "1" at the position "11" in the hashed bitmap h12.

Regarding the positions from the position "31" onward in the bitmap b1, the generating unit 350a repeatedly performs the operations explained above and generates the hashed bitmap h12.

Regarding each row in the inverted index 340a, the generating unit 350a performs compression according to the loop back technique explained above, and obtains a hashed inverted index. Meanwhile, the hashed bitmaps corresponding to the bases "29" and "31" are attached with the information about the corresponding row (the types of the encoded codons) of the respective source bitmaps.

The obtaining unit 350b is a processing unit that sequentially obtains, from the inverted index 340a, the bitmaps corresponding to the encoded codons included in the second-type sequence data 140e. Then, the obtaining unit 350b outputs the information about the obtained bitmaps to the identifying unit 350c. Herein, it is assumed that the bitmap information output to the identifying unit 350c is sorted in the order in which it was read.

The obtaining unit 350b reads the encoded codons in sequence from the start codon in the second-type sequence data 140e and obtains, from the inverted index 340a, the bitmap corresponding to the type of the read codon. For example, it is assumed that "AUG (63h)" represents the start codon and that the second-type sequence data 140e is as illustrated in FIG. 24. The obtaining unit 350b reads the bitmap b10 of "AUG (63h)", the bitmap b11 of "UUU (40h)", the bitmap b12 of "GUC (71h)", the bitmap (not illustrated) of "CAA (5Ah)", and the bitmaps of the subsequent codons.

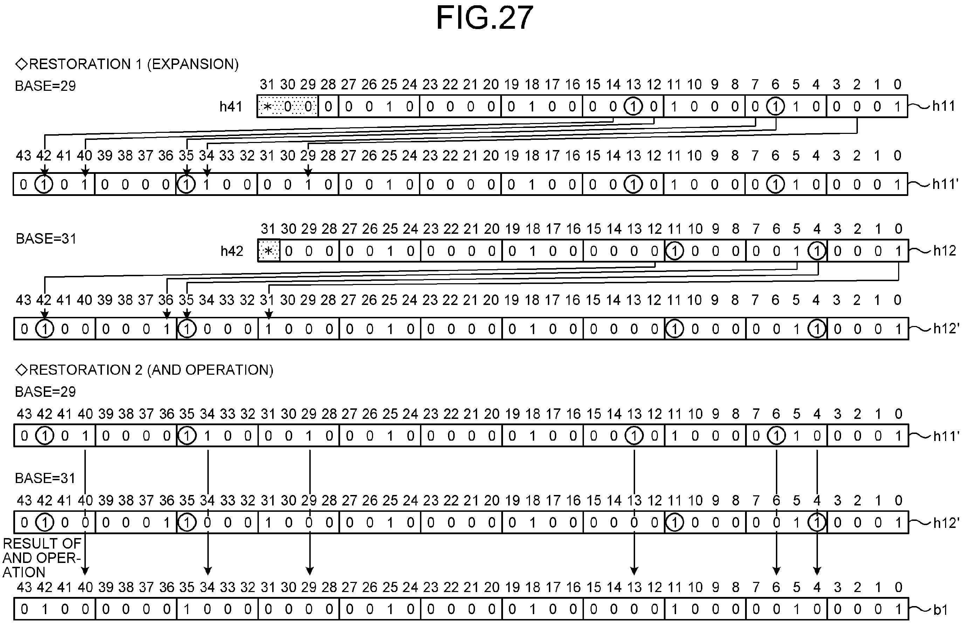

Meanwhile, when the inverted index 340a is hashed, the obtaining unit 350b performs the following operations and restores the hashed inverted index 340a. FIG. 27 is a diagram illustrating an example of the operations for restoring an inverted index. Herein, as an example, the explanation is given about a case in which the obtaining unit 350b restores the bitmap b1 based on the hashed bitmaps h11 and h12.

The obtaining unit 350b generates an intermediate bitmap h11' from the hashed bitmap h11 corresponding to the base "29". The obtaining unit 350b copies the values of the positions "0" to "28" in the hashed bitmap h11 to the positions "0" to "28" in the intermediate bitmap h11'.

Regarding the values from the position "29" onward in the intermediate bitmap h11', the obtaining unit 350b repeatedly performs, after every position "29", the operation of copying the values of the positions "0" to "28" in the hashed bitmap h11. In the example illustrated in FIG. 27, the values of the positions "0" to "14" in the hashed bitmap h11 are copied to the positions "29" to "43" in the intermediate bitmap h11'.

The obtaining unit 350b generates an intermediate map h12' from the hashed bitmap h12 corresponding to the base "31". The obtaining unit 350b copies the values of the positions "0" to "30" in the hashed bitmap h12 to the positions "0" to "30" in the intermediate bitmap h12'.

Regarding the values from the position "31" onward in the intermediate bitmap h12', the obtaining unit 350b repeatedly performs, after every position "31", the operation of copying the values of the positions "0" to "30" in the hashed bitmap h12. In the example illustrated in FIG. 27, the values of the positions "0" to "12" in the hashed bitmap h12 are copied to the positions "31" to "43" in the intermediate bitmap h12'.

After generating the intermediate bitmaps h11' and h12', the obtaining unit 350b performs the AND operation of the intermediate bitmaps h11' and h12' so as to restore the pre-hashing bitmap b1. Regarding the other hashed bitmaps too, the obtaining unit 350b can perform identical operations and restore the bitmaps corresponding to the codons (i.e., restore the inverted index 340a).

Returning to the explanation with reference to FIG. 25, the identifying unit 350c performs operations to identify the mutation position at which the first-type sequence data 140d and the second-type sequence data 140e become nonidentical; performs operations to identify the type of point mutation; and performs operations to identify genetic mutation.

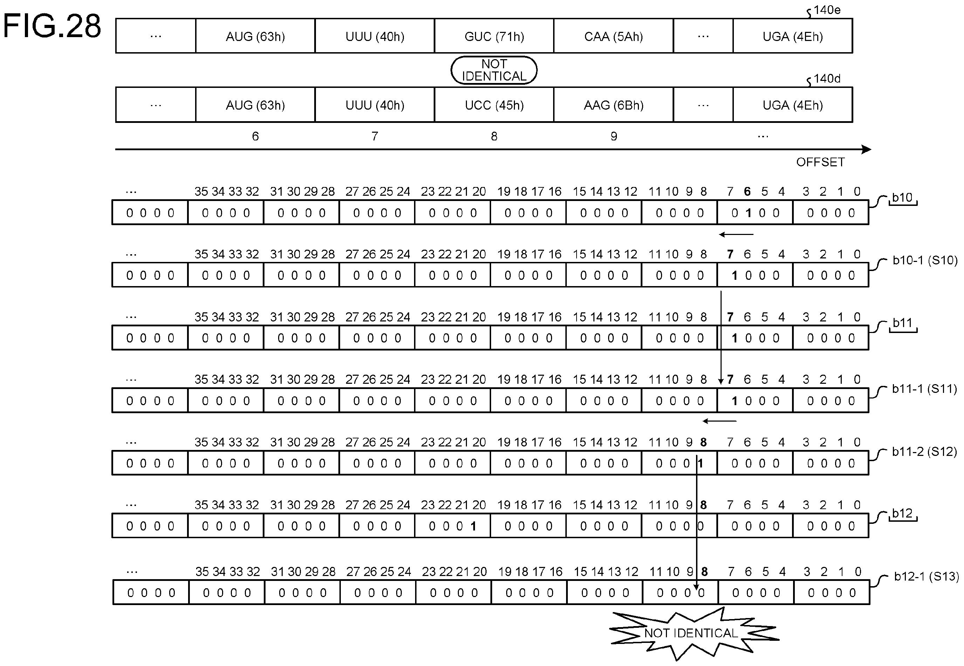

Given below is the explanation of the operations performed by the identifying unit 350c for identifying the mutation position at which the first-type sequence data 140d and the second-type sequence data 140e become nonidentical. FIG. 28 is a diagram for explaining the operations performed by the identifying unit according to the third embodiment. The bitmaps b10, b11, and b12 illustrated in FIG. 28 are the bitmaps received from the obtaining unit 350b.

The identifying unit 350c performs left-side shifting of the bitmap b10 and generates a bitmap b10-1 (Step S10). Then, the identifying unit 350c performs the AND operation of the bitmap 10-1 and the bitmap b11, and calculates a bitmap b11-1 (Step S11). In the bitmap b11-1, the bit "1" is set at the offset "7". Thus, it implies that the first-type sequence data 140d and the second-type sequence data 140e are identical from the offset "6" to the offset "7".

Moreover, the identifying unit 350c performs left-side shifting of the bitmap b11-1 and calculates a bitmap 11-2 (Step S12). Then, the identifying unit 350c performs the AND operation of the bitmap b11-2 and the bitmap b12, and calculates a bitmap b12-1 (Step S13). In the bitmap b11-2, the bit "1" is set at the offset "8". However, in the bitmap b12-1, the offset "8" has the bit "0" set therein. Hence, the identifying unit 350c determines that the first-type sequence data 140d and the second-type sequence data 140e are not identical at the offset (sequence position) "8".

Given below is the explanation of the operations performed by the identifying unit 350c for identifying the type of point mutation. Based on a nonidentical mutation position (offset) and based on the insertion transition table 140f and the deletion transition table 140g, the identifying unit 350c identifies the type of point mutation that has occurred at the mutation position. Once the type of point mutation is identified, the identifying unit 350c generates the third-type sequence data 240e by correcting the second-type sequence data 140e.

Herein, the operations performed by the identifying unit 350c for identifying the type of point mutation are identical to the operations performed by the identifying unit 150d according to the first embodiment. Moreover, the operations performed by the identifying unit 350c for generating the third-type sequence data 240e by correcting the second-type sequence data 140e based on the type of point mutation are identical to the operations performed by the identifying unit 250d according to the second embodiment.

Given below is the explanation of the operations performed by the identifying unit 350c for identifying genetic mutation. The identifying unit 350c sequentially obtains, from the inverted index 340a, the bitmaps corresponding to the types of the encoded codons included in the third-type sequence data 240e. In the case of reading a bitmap, in an identical manner to the obtaining unit 350b, the identifying unit 350c reads the encoded codons in sequence from the start codon, and obtains the bitmaps corresponding to the types of the read codons from the inverted index 340a.

Once the bitmaps are obtained, in an identical manner to the explanation given with reference to FIG. 24, the identifying unit 350c repeatedly performs the operations of performing the AND operation of a left-shifted bitmap, which is obtained by performing left-side shifting of a bitmap, and the subsequent bitmap, and calculating a new bitmap. Then, at the offset in the new bitmap from which the bit "1" is no more included, the identifying unit 350c determines that the first-type sequence data 140d and the third-type sequence data 240e become nonidentical. Thus, the identifying unit 350c determines that the codon in the third-type sequence data 240e corresponding to the offset determined to be nonidentical is the codon representing genetic mutation.

The identifying unit 350c performs the operations explained above and registers, in the detection result table 240h, the information about the type of point mutation and the mutation position (offset), as well as registers the information about the codon identified as genetic mutation and its sequence position (offset).

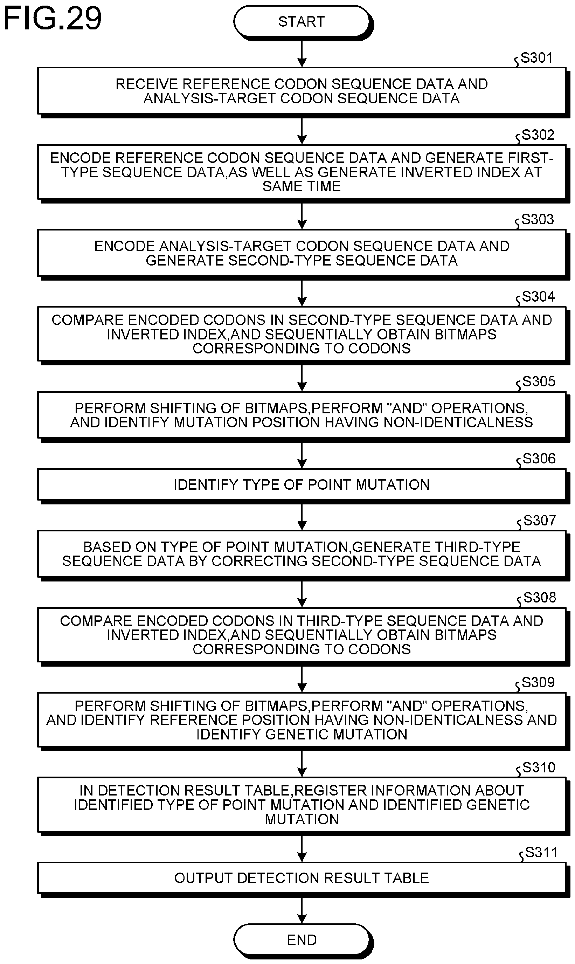

Given below is the explanation of an exemplary sequence of operations performed in the information processing device 300 according to the third embodiment. FIG. 29 is a flowchart for explaining a sequence of operations performed in the information processing device according to the third embodiment. As illustrated in FIG. 29, the receiving unit 150a of the information processing device 300 receives the reference codon sequence data 140a and the analysis-target codon sequence data 140b (Step S301) .

The encoding unit 150b of the information processing device 300 encodes the reference codon sequence data 140a and generates the first-type sequence data 140d; as well as generates the inverted index 340a at the same time (Step S302).

The encoding unit 150b of the information processing device 300 encodes the reference codon sequence data 140b and generates the second-type sequence data 140d (Step S303). The obtaining unit 350b of the information processing device 300 compares the encoded codons in the second-type sequence data 140e and the inverted index 340a, and sequentially obtains the bitmaps corresponding to the codons (Step S304).

The identifying unit 350c of the information processing device 300 performs shifting of the bitmaps and performs the AND operations, and identifies the mutation position (offset) having non-identicalness (Step S305). Moreover, the identifying unit 350c identifies the type of point mutation (Step S306).

Then, the identifying unit 350c generates the third-type sequence data 240e by correcting the second-type sequence data 140e based on the type of point mutation (Step S307). The identifying unit 350c compares the encoded codons in the third-type sequence data and the inverted index 340a, and sequentially obtains the bitmaps corresponding to the codons (Step S308).

Subsequently, the identifying unit 350c performs shifting of the bitmaps and performs the AND operations, and identifies the mutation position (offset) having non-identicalness and identifies genetic mutation (Step S309). Then, the identifying unit 350c registers the information about the identified type of point mutation and the identified genetic mutation in the detection result table 240h (Step S310). Subsequently, the information processing device 300 outputs the detection result table 240h to the display unit 130 for display purposes (Step S311).

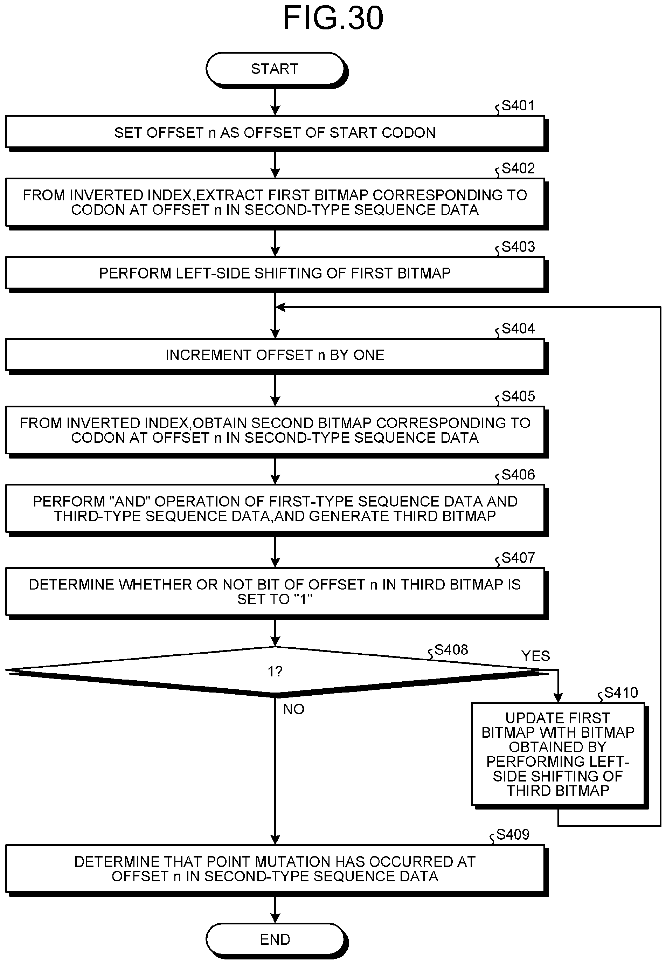

Given below is the explanation of an exemplary sequence of operations performed by the identifying unit 350c for identifying, based on bitmaps, the offset corresponding to point mutation. FIG. 30 is a flowchart for explaining the operations performed by the identifying unit according to the third embodiment for identifying the offset corresponding to point mutation. As illustrated in FIG. 30, the identifying unit 350c of the information processing device 300 identifies the offset n as the offset for the start codon (Step S401). Then, the obtaining unit 350b of the information processing device 100 obtains, from the inverted index 340a, a first bitmap corresponding to the codon at the offset n in the second-type sequence data 140e (Step S402).

The identifying unit 350c performs left-side shifting of the first bitmap (Step S403). Then, the identifying unit 350c increments the offset n by one (Step S404). Subsequently, the obtaining unit 350b obtains, from the inverted index 340a, a second bitmap corresponding to the codon at the offset n included in the second-type sequence data (Step S405).

Then, the identifying unit 350c performs the AND operation of the first bitmap and the second bitmap, and generates a third bitmap (Step S406). Moreover, the identifying unit 350c determines whether or not the bit of the offset n in the third bitmap is set to "1" (Step S407).

If the bit of the offset n in the third bitmap is not set to "1" (No at Step S408), then the identifying unit 350c determines that point mutation has occurred at the offset n included in the second-type sequence data (Step S409) .

On the other hand, if the bit of the offset n in the third bitmap is set to "1" (Yes at Step S408), then the identifying unit 350c updates the first bitmap with a bitmap obtained by performing left-side shifting of the third bitmap (Step S410). Then, the system control returns to Step S404.

Given below is the explanation about the effects achieved in the information processing device 300 according to the third embodiment. The information processing device 300 according to the third embodiment sequentially obtains, from the inverted index 340a, the bitmaps corresponding to the types of codons starting from the start codon included in the second-type sequence data 140e, and identifies nonidentical codons based on the shifting of a plurality of obtained bitmaps and the AND operation thereof. As a result, it becomes possible to perform a high-speed search for the codons having point mutation or genetic mutation.

Meanwhile, for the purpose of illustration, the explanation is given about the case in which the information processing device 300 according to the third embodiment generates the third-type sequence data 240e, and compares it with the first-type sequence data 140d. However, that is not the only possible case. Alternatively, instead of generating the third-type sequence data 240e, the information processing device 200 can convert the second-type sequence data 140e into the units of bytes, and compare the conversion result with the first-type sequence data 140d in the units of bytes.

Given below is the explanation of the other operations performed in the information processing device 300 according to the third embodiment. When the input of a search query is an amino-acid sequence, the information processing device 300 encodes the reference codon sequence data 140a written using base symbols; and generates an inverted index in a corresponding manner to the codons. Moreover, the information processing device 300 converts the codon sequence into an amino-acid sequence; generates an inverted index associated to the amino acids; and identifies the mutation position using that inverted index.

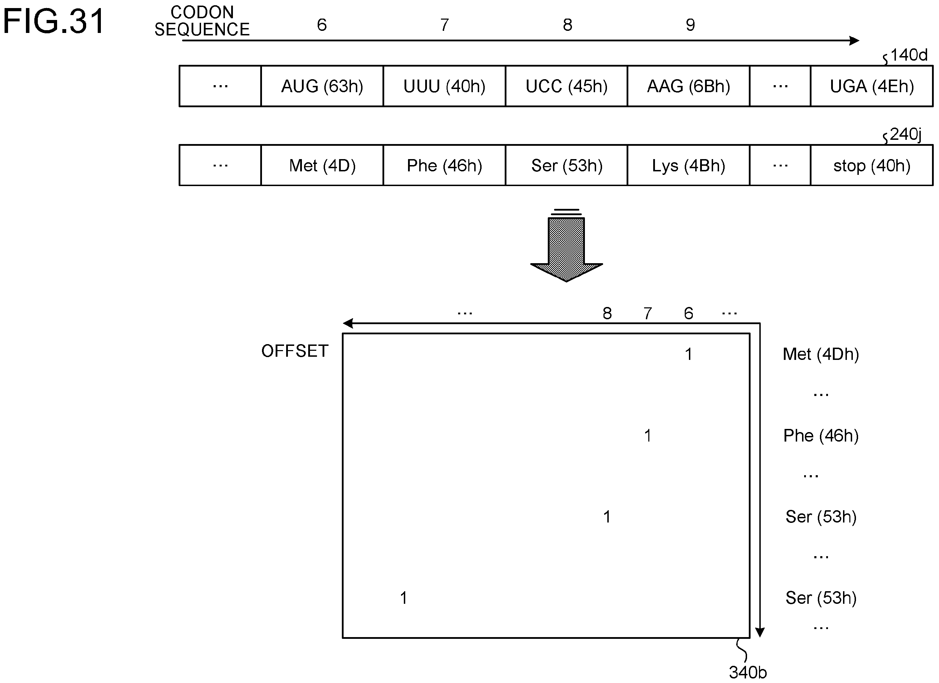

FIG. 31 is a diagram for explaining the other operations performed in the information processing device according to the third embodiment. As illustrated in FIG. 31, the information processing device generates the fourth-type sequence data 240j based on the first-type sequence data 140d and based on the codon-amino acid conversion table 240i illustrated in FIG. 21A; as well as generates an inverted index 340b at the same time. The inverted index 340b represents information indicating the relationship between the types of the encoded codes, which are included in the fourth-type sequence data 240j, and the sequence positions (offsets) using bitmaps.

The information processing device 300 performs the operation of identifying the mutation position using the inverted index 340b corresponding to the amino-acid sequence. For example, the information processing device 300 obtains, from the inverted index 340b, the bitmaps corresponding to the types of amino acids starting from the first amino acid included in the amino-acid sequence data; and, based on the positions of the flags of a plurality of obtained bitmaps, identifies the sequence positions, from among the amino acids included in the amino-acid sequence data, that are not identical with respect to the fourth-type sequence data 240j.

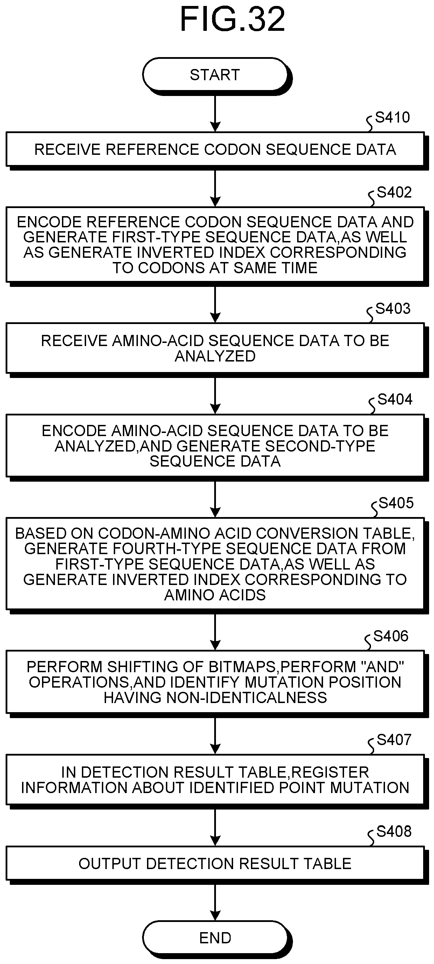

Given below is the explanation of an exemplary sequence of operations performed in the information processing device 300 according to third embodiment when the input of a search query is an amino-acid sequence. FIG. 32 is a flowchart (2) for explaining a sequence of operations performed in the information processing device according to the third embodiment.

As illustrated in FIG. 32, the receiving unit 150a of the information processing device 300 receives the reference codon sequence data (Step S401). Then, the encoding unit 150b of the information processing device 300 encodes the reference codon sequence data and generates the first-type sequence data 140d; and the generating unit 350a generates the inverted index 340a (Step S402).

The receiving unit 150a receives the amino-acid sequence data to be analyzed (Step S403). Then, the encoding unit 150b encodes the amino-acid sequence data to be analyzed, and generates the second-type sequence data 140e (Step S404).

Then, based on the codon-amino acid conversion table 240i, the generating unit 350a generates the fourth-type sequence data 240j from the first-type sequence data 140d, and at the same time generates the inverted index 340b corresponding to the amino acids (Step S405).

The identifying unit 350c of the information processing device 400 performs shifting of the bitmaps and performs the AND operations, and identifies the nonidentical mutation position (offsets) (Step S406). Then, the identifying unit 350c registers the information about the identified mutation in the detection result table 240h (Step S407). The information processing device 300 outputs the detection result table 240h to the display unit 130 for display purposes (Step S408).

As explained above, when the input of a search query is an amino-acid sequence, the information processing device 300 generates the inverted index 340b corresponding to the amino acids, and compares the inverted index 340b with the second-type sequence data 140e. Thus, even when the input of a search query is an amino-acid sequence, the amino acids in which mutation has occurred can be identified using the inverted index.

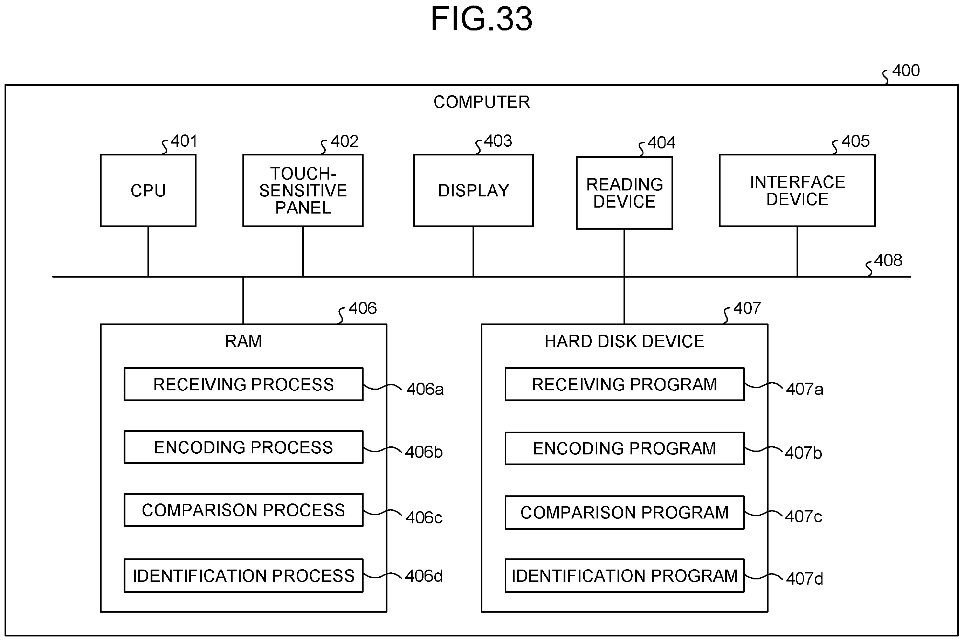

Given below is the explanation of an exemplary hardware configuration of a computer that implements the functions identical to the functions of the information processing device 100 according to the first embodiment and the information processing device 200 according to the second embodiment. FIG. 33 is a diagram illustrating an exemplary hardware configuration of a computer that implements the functions identical to the functions of the information processing devices according to the first and second embodiments.

As illustrated in FIG. 33, a computer 400 includes a CPU 401 that performs a variety of arithmetic processing; an input device 402 that receives input of data from the user; and a display 403. Moreover, the computer 400 includes a reading device 404 that reads programs from a memory medium; and an interface device 405 that communicates data with external devices via a wired network or a wireless network. Furthermore, the computer 400 includes a RAM 406 that is used to temporarily store a variety of information; and includes a hard disk device 407. The devices 401 to 407 are connected to each other by a bus 408.

The hard disk device 407 includes a receiving program 407a, an encoding program 407b, a comparison program 407c, and an identification program 407d. The CPU 401 reads the receiving program 407a, the encoding program 407b, the comparison program 407c, and the identification program 407d and loads them in the RAM 406.

The receiving program 407a functions as a receiving process 406a. The encoding program 407b functions as an encoding process 406b. The comparison program 407c functions as a comparison process 406c. The identification program 407d functions as an identification process 406d.

The operations of the receiving process 406a correspond to the operations of the receiving unit 150a. The operations of the encoding process 406b correspond to the operations of the encoding unit 150b. The operations of the comparison process 406c correspond to the operations of the comparing unit 150c. The operations of the identification process 406d correspond to the operations of the identifying units 150d and 250d.

The programs 407a to 407d need not always be stored in the hard disk device 407 from the beginning. Alternatively, for example, the programs 407a to 407d can be stored in a "portable physical medium" such as a flexible disk (FD), a CD-ROM, a DVD, a magneto-optical disk, or an IC card that is insertable in the computer 400. Then, the computer 400 can read and execute the programs 407a to 407d.

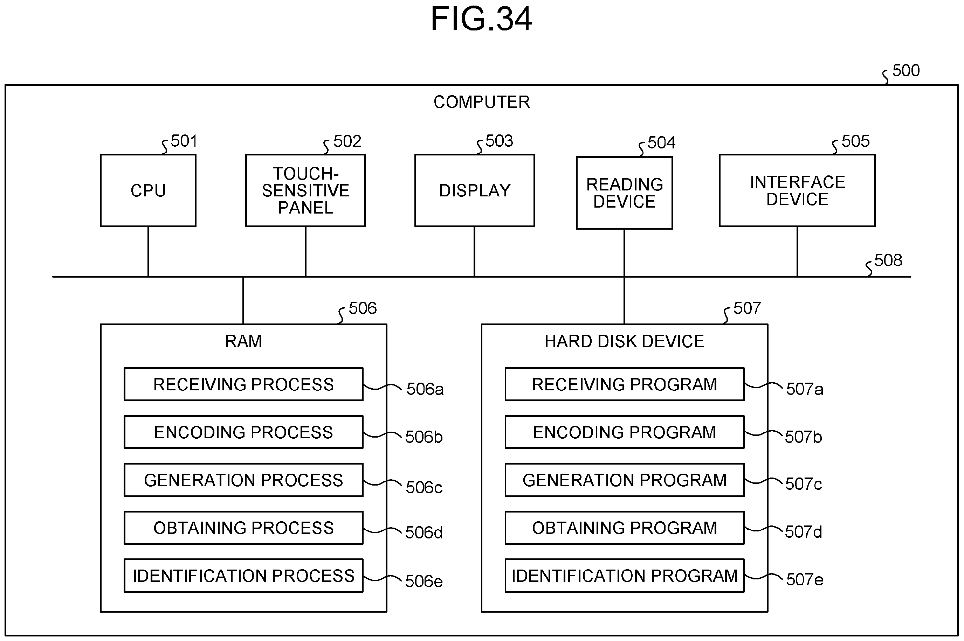

Given below is the explanation of an exemplary hardware configuration of a computer that implements the functions identical to the functions of the information processing device 300 according to the third embodiment. FIG. 34 is a diagram illustrating an exemplary hardware configuration of a computer that implements the functions identical to the functions of the information processing device according to the third embodiment.

As illustrated in FIG. 34, a computer 500 includes a CPU 501 that performs a variety of arithmetic processing; an input device 502 that receives input of data from the user; and a display 503. Moreover, the computer 500 includes a reading device 504 that reads programs from a memory medium; and an interface device 505 that communicates data with external devices via a wired network or a wireless network. Furthermore, the computer 500 includes a RAM 506 that is used to temporarily store a variety of information; and includes a hard disk device 507. The devices 501 to 507 are connected to each other by a bus 508.

The hard disk device 507 includes a receiving program 507a, an encoding program 507b, a generation program 507c, an obtaining program 507d, and an identification program 507e. The CPU 501 reads the receiving program 507a, the encoding program 507b, the generation program 507c, the obtaining program 507d, and the identification program 507e; and load them in the RAM 406.

The receiving program 507a functions as a receiving process 506a. The encoding program 507b functions as an encoding process 506b. The generation program 507c functions as a generation process 506c. The obtaining program 507d functions as an obtaining process 506d. The identification program 507e functions as an identification process 506e.

The operations of the receiving process 406a correspond to the operations of the receiving unit 150a. The operations of the encoding process 406b correspond to the operations of the encoding unit 150b. The operations of the generation process 506c correspond to the operations of the generating unit 350a. The operations of the obtaining process 506d correspond to the operations of the obtaining unit 350b. The operations of the identification process 506e correspond to the operations of the identifying unit 350c.

The programs 507a to 507d need not always be stored in the hard disk device 507 from the beginning. Alternatively, for example, the programs 507a to 507e can be stored in a "portable physical medium" such as a flexible disk (FD), a CD-ROM, a DVD, a magneto-optical disk, or an IC card that is insertable in the computer 500. Then, the computer 500 can read and execute the programs 507a to 507e.

[Explanation of Reference]

| | |
|---|---|
| 100, 200, 300 | information processing device |
| 110 | communication unit |
| 120 | input unit |
| 130 | display unit |
| 140, 240, 340 | memory unit |
| 140a | reference codon sequence data |
| 140b | analysis-target codon sequence data |
| 140c | code conversion table |
| 140d | first-type sequence data |
| 140e | second-type sequence data |
| 140f | insertion transition table |
| 140g | deletion transition table |
| 140h, 240h | detection result table |
| 150, 250, 350 | control unit |
| 150a | receiving unit |
| 150b | encoding unit |
| 150c | comparing unit |
| 150d, 250d, 350c | identifying unit |
| 240e | third-type sequence data |
| 240i | codon-amino acid conversion table |
| 240j | fourth-type sequence data |
| 350a | generating unit |
| 350b | obtaining unit |

**Claims**

1. An identification method implemented in a computer, comprising:

   obtaining reference codon sequence data and analysis-target codon sequence data;
   comparing codons included in the obtained reference codon sequence data and codons included in the obtained analysis-target codon sequence data, at each sequence position of codon;
   identifying that, based on result of the comparing, includes identifying, from among codons included in the analysis-target codon sequence data, codon positioned at each of a plurality of sequence positions subsequent to sequence position at which codons are nonidentical; and
   identifying that includes

      referring to a memory unit configured to store type of mutation, which has occurred at a particular codon included in particular codon sequence data, in a corresponding manner to codon positioned at each of a plurality of sequence positions subsequent to the particular codon, on account of occurrence of the mutation in the particular codon, and
      identifying type of mutation associated to codon positioned at each of the plurality of identified sequence positions.

2. The identification method according to claim 1, wherein
   in the memory unit, mutant codon is stored in a corresponding manner to codon positioned at each of a plurality of sequence positions subsequent to sequence position of the particular codon, on account of occurrence of the mutation in the particular codon, and
   the identification method further includes identifying that includes

      comparing the memory unit, type of identified mutation, and codon positioned at each of the plurality of identified sequence positions, and
      identifying the mutant codon.

3. The identification method according to claim 2, further including identifying that includes
   correcting the analysis-target codon sequence data based on the mutant codon,
   comparing corrected codon sequence data and the reference codon sequence data, and
   identifying nonidentical codons.

4. The identification method according to claim 2, wherein
   regarding sequence position at which the mutant codon is not identical to codon in the reference codon sequence data, when codon positioned at subsequent sequence position of concerned sequence position is identical to the mutant codon,
   identifying the type of mutation includes determining that the type of mutation is base insertion.

5. The identification method according to claim 4, wherein
   regarding sequence position at which the mutant codon is not identical to codon in the reference codon sequence data, when codon positioned after subsequent sequence position of concerned sequence position is identical to the mutant codon,
   identifying the type of mutation includes determining that the type of mutation is base deletion.

6. The identification method according to claim 5, wherein, when the type of mutation is neither the base insertion nor the base deletion, identifying the type of mutation includes determining that the type of mutation is base substitution.

7. An identification method implemented in a computer, comprising:

   obtaining reference codon sequence data and analysis-target codon sequence data;
   generating

      first-type sequence data by encoding the reference codon sequence data in units of codons, and
      second-type sequence data by encoding the analysis-target codon sequence data in units of codons; and

   identifying that includes

comparing the first-type sequence data and the second-type sequence data in units of codes each of which is equivalent to a codon, and

identifying position at which codes are not identical.

8. The identification method according to claim 7, wherein
when amino-acid sequence data is obtained as analysis-target sequence data,
the identification method further includes converting codes in units of codons in the first-type sequence data into codes in units of amino acids, and
the identifying includes identifying position at which codes are not identical, based on the amino-acid sequence data in encoded form and based on the first-type sequence data after conversion.

9. An identification method implemented in a computer, comprising:

generating that includes

obtaining reference codon sequence data, and
generating an inverted index in which types of codons included in the reference codon sequence data and sequence positions of the codons are associated using flags of bitmaps;

obtaining that, when analysis-target codon sequence data is obtained, includes sequentially obtaining, from the inverted index, bitmaps corresponding to types of codons starting from start codon included in the analysis-target codon sequence data; and

identifying that, based on positions of flags of a plurality of obtained bitmaps, includes identifying, from among codons included in the analysis-target codon sequence data, sequence position at which codon is not identical to codon in the reference codon sequence data.

10. The identification method according to claim 9, wherein,
when amino-acid sequence data is obtained as analysis-target sequence data, the generating includes generating an amino-acid inverted index in which types of codons included in the reference codon sequence data are converted into types of amino acids,
the obtaining includes sequentially obtaining, from the amino-acid inverted index, bitmaps corresponding to types of amino acids starting from first amino acid included in the amino-acid sequence data, and
based on positions of flags of a plurality of obtained bitmaps, the identifying includes identifying, from among amino acids included in the amino-acid sequence data, sequence position having non-identicalness with the reference codon sequence data.

11. An identification program that causes a computer to execute:

obtaining reference codon sequence data and analysis-target codon sequence data;
comparing codons included in the obtained reference codon sequence data and codons included in the obtained analysis-target codon sequence data, at each sequence position of codon;
identifying that, based on result of the comparing, includes identifying, from among codons included in the analysis-target codon sequence data, codon positioned at each of a plurality of sequence positions subsequent to sequence position at which codons are nonidentical; and
identifying that includes

referring to a memory unit configured to store type of mutation, which has occurred at a particular codon included in particular codon sequence data, in a corresponding manner to codon positioned at each of a plurality of sequence positions subsequent to the particular codon, on account of occurrence of the mutation in the particular codon, and
identifying type of mutation associated to codon positioned at each of the plurality of identified sequence positions.

12. The identification program according to claim 11, wherein
in the memory unit, mutant codon is stored in a corresponding manner to codon positioned at each of a plurality of sequence positions subsequent to sequence position of the particular codon, on account of occurrence of the mutation in the particular codon, and
the identification program further causes the computer to execute identifying that includes

comparing the memory unit, type of identified mutation, and codon positioned at each of the plurality of identified sequence positions, and

identifying the mutant codon.

13. The identification program according to claim 12, further causes the computer to execute identifying that includes correcting the analysis-target codon sequence data based on the mutant codon,

comparing corrected codon sequence data and the reference codon sequence data, and

identifying nonidentical codons.

14. The identification program according to claim 12, wherein

regarding sequence position at which the mutant codon is not identical to codon in the reference codon sequence data, when codon positioned at subsequent sequence position of concerned sequence position is identical to the mutant codon,

identifying the type of mutation includes determining that the type of mutation is base insertion.

15. The identification program according to claim 14, wherein

regarding sequence position at which the mutant codon is not identical to codon in the reference codon sequence data, when codon positioned after subsequent sequence position of concerned sequence position is identical to the mutant codon,

identifying the type of mutation includes determining that the type of mutation is base deletion.

16. The identification program according to claim 15, wherein, when the type of mutation is neither the base insertion nor the base deletion, identifying the type of mutation includes determining that the type of mutation is base substitution.

17. An identification program that causes a computer to execute:

obtaining reference codon sequence data and analysis-target codon sequence data;

generating

first-type sequence data by encoding the reference codon sequence data in units of codons, and

second-type sequence data by encoding the analysis-target codon sequence data in units of codons; and

identifying that includes

comparing the first-type sequence data and the second-type sequence data in units of codes each of which is equivalent to a codon, and

identifying position at which codes are not identical.

18. The identification program according to claim 17, wherein

when amino-acid sequence data is obtained as analysis-target sequence data,

the identification program further causes the computer to execute converting codes in units of codons in the first-type sequence data into codes in units of amino acids, and

the identifying includes identifying position at which codes are not identical, based on the amino-acid sequence data in encoded form and based on the first-type sequence data after conversion.

19. An identification program that causes a computer to execute:

generating that includes

obtaining reference codon sequence data, and

generating an inverted index in which types of codons included in the reference codon sequence data and sequence positions of the codons are associated using flags of bitmaps;

obtaining that, when analysis-target codon sequence data is obtained, includes sequentially obtaining, from the inverted index, bitmaps corresponding to types of codons starting from start codon included in the analysis-target codon sequence data; and

identifying that, based on positions of flags of a plurality of obtained bitmaps, includes identifying, from among codons included in the analysis-target codon sequence data, sequence position at which codon is not identical to codon in the reference codon sequence data.

20. The identification program according to claim 19, wherein,
    when amino-acid sequence data is obtained as analysis-target sequence data, the generating includes generating an amino-acid inverted index in which types of codons included in the reference codon sequence data are converted into types of amino acids,
    the obtaining includes sequentially obtaining, from the amino-acid inverted index, bitmaps corresponding to types of amino acids starting from first amino acid included in the amino-acid sequence data, and
    based on positions of flags of a plurality of obtained bitmaps, the identifying includes identifying, from among amino acids included in the amino-acid sequence data, sequence position having non-identicalness with the reference codon sequence data.

21. An information processing device comprising:

    a comparing unit configured to

        obtain reference codon sequence data and analysis-target codon sequence data, and
        compare codons included in the obtained reference codon sequence data and codons included in the obtained analysis-target codon sequence data, at each sequence position of codon; and

    an identifying unit configured to

        based on result of comparison, identify, from among codons included in the analysis-target codon sequence data, codon positioned at each of a plurality of sequence positions subsequent to sequence position at which codons are nonidentical, and
        refer to a memory unit that stores type of mutation, which has occurred at a particular codon included in particular codon sequence data, in a corresponding manner to codon positioned at each of a plurality of sequence positions subsequent to the particular codon, on account of occurrence of the mutation in the particular codon, and identify type of mutation associated to codon positioned at each of the plurality of identified sequence positions.

22. The information processing device according to claim 21, wherein
    in the memory unit, mutant codon is stored in a corresponding manner to codon positioned at each of a plurality of sequence positions subsequent to sequence position of the particular codon, on account of occurrence of the mutation in the particular codon, and
    the identifying unit is configured to

        compare the memory unit, type of identified mutation, and codon positioned at each of the plurality of identified sequence positions, and
        identify the mutant codon.

23. The information processing device according to claim 22, wherein the identifying unit is configured to
    correct the analysis-target codon sequence data based on the mutant codon,
    compare corrected codon sequence data and the reference codon sequence data, and
    identify nonidentical codons.

24. The information processing device according to claim 22, wherein
    regarding sequence position at which the mutant codon is not identical to codon in the reference codon sequence data, when codon positioned at subsequent sequence position of concerned sequence position is identical to the mutant codon,
    the identifying unit is configured to determine that the type of mutation is base insertion.

25. The information processing device according to claim 24, wherein
    regarding sequence position at which the mutant codon is not identical to codon in the reference codon sequence data, when codon positioned after subsequent sequence position of concerned sequence position is identical to the mutant codon,
    the identifying unit is configured to determine that the type of mutation is base deletion.

**26.** The information processing device according to claim 25, wherein, when the type of mutation is neither the base insertion nor the base deletion, the identifying unit is configured to determine that the type of mutation is base substitution.

**27.** An information processing device comprising:

an encoding unit configured to

obtain reference codon sequence data and analysis-target codon sequence data, and generate

first-type sequence data by encoding the reference codon sequence data in units of codons, and second-type sequence data by encoding the analysis-target codon sequence data in units of codons; and

an identifying unit configured to

compare the first-type sequence data and the second-type sequence data in units of codes each of which is equivalent to a codon, and identify position at which codes are not identical.

**28.** The information processing device according to claim 27, wherein
when amino-acid sequence data is obtained as analysis-target sequence data,
the encoding unit further is configured to convert codes in units of codons in the first-type sequence data into codes in units of amino acids, and
the identifying unit is configured to identify position at which codes are not identical, based on the amino-acid sequence data in encoded form and based on the first-type sequence data after conversion.

**29.** An information processing device comprising:

a generating unit configured to

obtain reference codon sequence data, and generate an inverted index in which types of codons included in the reference codon sequence data and sequence positions of the codons are associated using flags of bitmaps; and

an identifying unit configured to

when analysis-target codon sequence data is obtained, sequentially obtain, from the inverted index, bitmaps corresponding to types of codons starting from start codon included in the analysis-target codon sequence data, and
based on positions of flags of a plurality of obtained bitmaps, identify, from among codons included in the analysis-target codon sequence data, sequence position at which codon is not identical to codon in the reference codon sequence data.

**30.** The information processing device according to claim 29, wherein

when amino-acid sequence data is obtained as analysis-target sequence data, the generating unit is configured to generate an amino-acid inverted index in which types of codons included in the reference codon sequence data are converted into types of amino acids,
the identifying unit is configured to

sequentially obtain, from the amino-acid inverted index, bitmaps corresponding to types of amino acids starting from first amino acid included in the amino-acid sequence data, and
based on positions of flags of a plurality of obtained bitmaps, identify, from among amino acids included in the amino-acid sequence data, sequence position having non-identicalness with the reference codon sequence data.

# FIG.1

| ... | AUG | UUU | UCC | AAG | UGC | ... | UGA | 20A |

| Asn | Phe | Ser | Lys | Cys | TERMI-NATION |

$P_{20}$

COMPARISON

| ... | AUG | UUU | GUC | CAA | GUG | ... | UGA | 20B |

| Asn | Phe | Val | Gln | Val | TERMI-NATION |

NOT IDENTICAL

FIG.2

P$_{20}$

20B

| | UUU | MUTATION CODON GUC | MUTATION 1 CODON CAA | MUTATION 2 CODON GUG | ... |

140f

| MUTATION n CODON CAA | MUTATION n+1 CODON GUG | MUTANT n CODON (INSERTION) AAG |

IDENTICAL CODONS

POINT MUTATION= BASE INSERTION

20A

P$_{20}$

| ... | AUG | UUU | UCC MUTANT CODON | AAG MUTANT 1 CODON | UGC | ... |

# FIG.3

EP 3 848 935 A1

30A

| ... | AUG | UUU | UCC | AAG | UGC | ... | UGA |
|-----|-----|-----|-----|-----|-----|-----|-----|

Asn    Phe    Ser    Lys                    TERMI-NATION

$P_{30}$

COMPARISON

30B

| ... | AUG | UUU | UCA | AGU | GCU | ... | UGA |
|-----|-----|-----|-----|-----|-----|-----|-----|

Asn    Phe    Ser    Ser    Ala                TERMI-NATION

NOT IDENTICAL

# FIG.4

P$_{30}$

| MUTATION CODON | MUTATION 1 CODON | MUTATION 2 CODON | | 30B |
|:---:|:---:|:---:|:---:|
| UCA | AGU | GCU | ... |

| MUTATION n CODON | MUTATION n+1 CODON | MUTANT n+1 CODON (DELETION) | 140g |
|:---:|:---:|:---:|
| AGU | GCU | UGC |

IDENTICAL CODONS

MUTATION= BASE DELETION

| | | | | | | 30A |
|:---:|:---:|:---:|:---:|:---:|:---:|:---:|
| ... | AUG | UUU | UCC | AAG | UGC | ... |

MUTANT CODON

MUTANT 1 CODON

MUTANT 2 CODON

P$_{30}$

# FIG.5

INFORMATION PROCESSING DEVICE — 100

CONTROL UNIT — 150

- RECEIVING UNIT — 150a
- ENCODING UNIT — 150b
- COMPARING UNIT — 150c
- IDENTIFYING UNIT — 150d

COMMUNICATION UNIT — 110

INPUT UNIT — 120

DISPLAY UNIT — 130

MEMORY UNIT — 140

- REFERENCE CODON SEQUENCE DATA — 140a
- ANALYSIS-TARGET CODON SEQUENCE DATA — 140b
- CODE CONVERSION TABLE — 140c
- FIRST-TYPE SEQUENCE DATA — 140d
- SECOND-TYPE SEQUENCE DATA — 140e
- INSERTION TRANSITION TABLE — 140f
- DELETION TRANSITION TABLE — 140g
- DETECTION RESULT TABLE — 140h

EP 3 848 935 A1

# FIG.6

| ... | AUG | UUU | UCC | AAG | UGC | ... | UGA |
|-----|-----|-----|-----|-----|-----|-----|-----|

140a

# FIG.7

| ··· | AUG | UUU | GUC | CAA | GUG | ··· | UGA |
|-----|-----|-----|-----|-----|-----|-----|-----|

140b

# FIG.8

140c

|  | 4 (0100) | 5 (0101) | 6 (0110) | 7 (0111) |
|---|---|---|---|---|
| 0 (0000) | UUU | CUU | AUU | GUU |
| 1 (0001) | UUC | CUC | AUC | GUC |
| 2 (0010) | UUA | CUA | AUA | GUA |
| 3 (0011) | UUG | CUG | AUG | GUG |
| 4 (0100) | UCU | CCU | ACU | GCU |
| 5 (0101) | UCC | CCC | ACC | GCC |
| 6 (0110) | UCA | CCA | ACA | GCA |
| 7 (0111) | UCG | CCG | ACG | GCG |
| 8 (1000) | UAU | CAU | AAU | GAU |
| 9 (1001) | UAC | CAC | AAC | GAC |
| A (1010) | UAA | CAA | AAA | GAA |
| B (1011) | UAG | CAG | AAG | GAG |
| C (1100) | UGU | CGU | AGU | GGU |
| D (1101) | UGC | CGC | AGC | GGC |
| E (1110) | UGA | CGA | AGA | GGA |
| F (1111) | UGG | CGG | AGG | GGG |

# FIG.9

140d

| ... | AUG (63h) | UUU (40h) | UCC (45h) | AAG (6Bh) | UGC (4Dh) | ... | UGA (4Eh) |

# FIG.10

| ··· | AUG (63h) | UUU (40h) | GUC (71h) | CAA (5Ah) | GUG (73h) | ··· | UGA (4Eh) |

140e

# FIG.11

140f

INSERTION TRANSITION TABLE

50U

TRANSITION TABLE

50C

TRANSITION TABLE

50A

TRANSITION TABLE

50G

TRANSITION TABLE

# FIG.12A

50U

| MUTATION n CODON | | | | MUTATION n+1 CODON | | | | MUTANT n CODON (INSERTION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| UUU (40h) | CUU (50h) | AUU (60h) | GUU (70h) | UUU (40h) | | | | UUU (40h) | UUU (40h) | UUU (40h) | UUU (40h) |
| UUC (41h) | CUC (51h) | AUC (61h) | GUC (71h) | UUC (41h) | | | | UCU (44h) | UCU (44h) | UCU (44h) | UCU (44h) |
| UUA (42h) | CUA (52h) | AUA (62h) | GUA (72h) | UUA (42h) | | | | UAU (48h) | UAU (48h) | UAU (48h) | UAU (48h) |
| UUG (43h) | CUG (53h) | AUG (63h) | GUG (73h) | UUG (43h) | | | | UGU (4Ch) | UGU (4Ch) | UGU (4Ch) | UGU (4Ch) |
| UCU (44h) | CCU (54h) | ACU (64h) | GCU (74h) | UCU (44h) | | | | CUU (50h) | CUU (50h) | CUU (50h) | CUU (50h) |
| UCC (45h) | CCC (55h) | ACC (65h) | GCC (75h) | UCC (45h) | | | | CCU (54h) | CCU (54h) | CCU (54h) | CCU (54h) |
| UCA (46h) | CCA (56h) | ACA (66h) | GCA (76h) | UCA (46h) | | | | CAU (58h) | CAU (58h) | CAU (58h) | CAU (58h) |
| UCG (47h) | CCG (57h) | ACG (67h) | GCG (77h) | UCG (47h) | | | | CGU (5Ch) | CGU (5Ch) | CGU (5Ch) | CGU (5Ch) |
| UAU (48h) | CAU (58h) | AAU (68h) | GAU (78h) | UAU (48h) | | | | AUU (60h) | AUU (60h) | AUU (60h) | AUU (60h) |
| UAC (49h) | CAC (59h) | AAC (69h) | GAC (79h) | UAC (49h) | | | | ACU (64h) | ACU (64h) | ACU (64h) | ACU (64h) |
| UAA (4Ah) | CAA (5Ah) | AAA (6Ah) | GAA (7Ah) | UAA (4Ah) | | | | AAU (68h) | AAU (68h) | AAU (68h) | AAU (68h) |
| UAG (4Bh) | CAG (5Bh) | AAG (6Bh) | GAG (7Bh) | UAG (4Bh) | | | | AGU (6Ch) | AGU (6Ch) | AGU (6Ch) | AGU (6Ch) |
| UGU (4Ch) | CGU (5Ch) | AGU (6Ch) | GGU (7Ch) | UGU (4Ch) | | | | GUU (70h) | GUU (70h) | GUU (70h) | GUU (70h) |
| UGC (4Dh) | CGC (5Dh) | AGC (6Dh) | GGC (7Dh) | UGC (4Dh) | | | | GCU (74h) | GCU (74h) | GCU (74h) | GCU (74h) |
| UGA (4Eh) | CGA (5Eh) | AGA (6Eh) | GGA (7Eh) | UGA (4Eh) | | | | GAU (78h) | GAU (78h) | GAU (78h) | GAU (78h) |
| UGG (4Fh) | CGG (5Fh) | AGG (6Fh) | GGG (7Fh) | UGG (4Fh) | | | | GGU (7Ch) | GGU (7Ch) | GGU (7Ch) | GGU (7Ch) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

EP 3 848 935 A1

# FIG.12B

50C

| MUTATION n CODON | | | | MUTATION n+1 CODON | | | | MUTANT n CODON (INSERTION) | | | |
|---|---|---|---|---|---|---|---|---|---|---|---|
| 4 (0100) | 5 (0101) | 6 (0110) | 7 (0111) | 4(0100) | 5 (0101) | 6 (0110) | 7 (0111) | | | | |
| UUU (40h) | CUU (50h) | AUU (60h) | GUU (70h) | | CUU (50h) | | | UUC (41h) | UUC (41h) | UUC (41h) | UUC (41h) |
| UUC (41h) | CUC (51h) | AUC (61h) | GUC (71h) | | CUC (51h) | | | UCC (45h) | UCC (45h) | UCC (45h) | UCC (45h) |
| UUA (42h) | CUA (52h) | AUA (62h) | GUA (72h) | | CUA (52h) | | | UAC (49h) | UAC (49h) | UAC (49h) | UAC (49h) |
| UUG (43h) | CUG (53h) | AUG (63h) | GUG (73h) | | CUG (53h) | | | UGC (4Dh) | UGC (4Dh) | UGC (4Dh) | UGC (4Dh) |
| UCU (44h) | CCU (54h) | ACU (64h) | GCU (74h) | | CCU (54h) | | | CUC (51h) | CUC (51h) | CUC (51h) | CUC (51h) |
| UCC (45h) | CCC (55h) | ACC (65h) | GCC (75h) | | CCC (55h) | | | CCC (55h) | CCC (55h) | CCC (55h) | CCC (55h) |
| UCA (46h) | CCA (56h) | ACA (66h) | GCA (76h) | | CCA (56h) | | | CAC (59h) | CAC (59h) | CAC (59h) | CAC (59h) |
| UCG (47h) | CCG (57h) | ACG (67h) | GCG (77h) | | CCG (57h) | | | CGC (5Dh) | CGC (5Dh) | CGC (5Dh) | CGC (5Dh) |
| UAU (48h) | CAU (58h) | AAU (68h) | GAU (78h) | | CAU (58h) | | | AUC (61h) | AUC (61h) | AUC (61h) | AUC (61h) |
| UAC (49h) | CAC (59h) | AAC (69h) | GAC (79h) | | CAC (59h) | | | ACC (65h) | ACC (65h) | ACC (65h) | ACC (65h) |
| UAA (4Ah) | CAA (5Ah) | AAA (6Ah) | GAA (7Ah) | | CAA (5Ah) | | | AAC (69h) | AAC (69h) | AAC (69h) | AAC (69h) |
| UAG (4Bh) | CAG (5Bh) | AAG (6Bh) | GAG (7Bh) | | CAG (5Bh) | | | AGC (6Dh) | AGC (6Dh) | AGC (6Dh) | AGC (6Dh) |
| UGU (4Ch) | CGU (5Ch) | AGU (6Ch) | GGU (7Ch) | | CGU (5Ch) | | | GUC (71h) | GUC (71h) | GUC (71h) | GUC (71h) |
| UGC (4Dh) | CGC (5Dh) | AGC (6Dh) | GGC (7Dh) | | CGC (5Dh) | | | GCC (75h) | GCC (75h) | GCC (75h) | GCC (75h) |
| UGA (4Eh) | CGA (5Eh) | AGA (6Eh) | GGA (7Eh) | | CGA (5Eh) | | | GAC (79h) | GAC (79h) | GAC (79h) | GAC (79h) |
| UGG (4Fh) | CGG (5Fh) | AGG (6Fh) | GGG (7Fh) | | CGG (5Fh) | | | GGC (7Dh) | GGC (7Dh) | GGC (7Dh) | GGC (7Dh) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

EP 3 848 935 A1

# FIG.12C

50A

| MUTATION n CODON | | | | MUTATION n+1 CODON | | | | MUTANT n CODON (INSERTION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| UUU (40h) | CUU (50h) | AUU (60h) | GUU (70h) | | AUU (60h) | | | UUA (42h) | UUA (42h) | UUA (42h) | UUA (42h) |
| UUC (41h) | CUC (51h) | AUC (61h) | GUC (71h) | | AUC (61h) | | | UCA (46h) | UCA (46h) | UCA (46h) | UCA (46h) |
| UUA (42h) | CUA (52h) | AUA (62h) | GUA (72h) | | AUA (62h) | | | UAA (4Ah) | UAA (4Ah) | UAA (4Ah) | UAA (4Ah) |
| UUG (43h) | CUG (53h) | AUG (63h) | GUG (73h) | | AUG (63h) | | | UGA (4Eh) | UGA (4Eh) | UGA (4Eh) | UGA (4Eh) |
| UCU (44h) | CCU (54h) | ACU (64h) | GCU (74h) | | ACU (64h) | | | CUA (52h) | CUA (52h) | CUA (52h) | CUA (52h) |
| UCC (45h) | CCC (55h) | ACC (65h) | GCC (75h) | | ACC (65h) | | | CCA (56h) | CCA (56h) | CCA (56h) | CCA (56h) |
| UCA (46h) | CCA (56h) | ACA (66h) | GCA (76h) | | ACA (66h) | | | CAA (5Ah) | CAA (5Ah) | CAA (5Ah) | CAA (5Ah) |
| UCG (47h) | CCG (57h) | ACG (67h) | GCG (77h) | | ACG (67h) | | | CGA (5Eh) | CGA (5Eh) | CGA (5Eh) | CGA (5Eh) |
| UAU (48h) | CAU (58h) | AAU (68h) | GAU (78h) | | AAU (68h) | | | AUA (62h) | AUA (62h) | AUA (62h) | AUA (62h) |
| UAC (49h) | CAC (59h) | AAC (69h) | GAC (79h) | | AAC (69h) | | | ACA (66h) | ACA (66h) | ACA (66h) | ACA (66h) |
| UAA (4Ah) | CAA (5Ah) | AAA (6Ah) | GAA (7Ah | | AAA (6Ah) | | | AAA (6Ah) | AAA (6Ah) | AAA (6Ah) | AAA (6Ah) |
| UAG (4Bh) | CAG (5Bh) | AAG (6Bh) | GAG (7Bh) | | AAG (6Bh) | | | AGA (6Eh) | AGA (6Eh) | AGA (6Eh) | AGA (6Eh) |
| UGU (4Ch) | CGU (5Ch) | AGU (6Ch) | GGU (7Ch) | | AGU (6Ch) | | | GUA (72h) | GUA (72h) | GUA (72h) | GUA (72h) |
| UGC (4Dh) | CGC (5Dh) | AGC (6Dh) | GGC (7Dh) | | AGC (6Dh) | | | GCA (76h) | GCA (76h) | GCA (76h) | GCA (76h) |
| UGA (4Eh) | CGA (5Eh) | AGA (6Eh) | GGA (7Eh) | | AGA (6Eh) | | | GAA (7Ah) | GAA (7Ah) | GAA (7Ah) | GAA (7Ah) |
| UGG (4Fh) | CGG (5Fh) | AGG (6Fh) | GGG (7Fh) | | AGG (6Fh) | | | GGA (7Eh) | GGA (7Eh) | GGA (7Eh) | GGA (7Eh) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

EP 3 848 935 A1

## FIG.12D

50A

| MUTATION n CODON | | | | MUTATION n+1 CODON | | | | MUTANT n CODON (INSERTION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| UUU (40h) | CUU (50h) | AUU (60h) | GUU (70h) | | | | GUU (70h) | UUG (43h) | UUG (43h) | UUG (43h) | UUG (43h) |
| UUC (41h) | CUC (51h) | AUC (61h) | GUC (71h) | | | | GUC (71h) | UCG (47h) | UCG (47h) | UCG (47h) | UCG (47h) |
| UUA (42h) | CUA (52h) | AUA (62h) | GUA (72h) | | | | GUA (72h) | UAG (4Bh) | UAG (4Bh) | UAG (4Bh) | UAG (4Bh) |
| UUG (43h) | CUG (53h) | AUG (63h) | GUG (73h) | | | | GUG (73h) | UGG (4Fh) | UGG (4Fh) | UGG (4Fh) | UGG (4Fh) |
| UCU (44h) | CCU (54h) | ACU (64h) | GCU (74h) | | | | GCU (74h) | CUG (53h) | CUG (53h) | CUG (53h) | CUG (53h) |
| UCC (45h) | CCC (55h) | ACC (65h) | GCC (75h) | | | | GCC (75h) | CCG (57h) | CCG (57h) | CCG (57h) | CCG (57h) |
| UCA (46h) | CCA (56h) | ACA (66h) | GCA (76h) | | | | GCA (76h) | CAG (5Bh) | CAG (5Bh) | CAG (5Bh) | CAG (5Bh) |
| UCG (47h) | CCG (57h) | ACG (67h) | GCG (77h) | | | | GCG (77h) | CGG (5Fh) | CGG (5Fh) | CGG (5Fh) | CGG (5Fh) |
| UAU (48h) | CAU (58h) | AAU (68h) | GAU (78h) | | | | GAU (78h) | AUG (63h) | AUG (63h) | AUG (63h) | AUG (63h) |
| UAC (49h) | CAC (59h) | AAC (69h) | GAC (79h) | | | | GAC (79h) | ACG (67h) | ACG (67h) | ACG (67h) | ACG (67h) |
| UAA (4Ah) | CAA (5Ah) | AAA (6Ah) | GAA (7Ah | | | | GAA (7Ah | AAG (6Bh) | AAG (6Bh) | AAG (6Bh) | AAG (6Bh) |
| UAG (4Bh) | CAG (5Bh) | AAG (6Bh) | GAG (7Bh) | | | | GAG (7Bh) | AGG (6Fh) | AGG (6Fh) | AGG (6Fh) | AGG (6Fh) |
| UGU (4Ch) | CGU (5Ch) | AGU (6Ch) | GGU (7Ch) | | | | GGU (7Ch) | GUG (73h) | GUG (73h) | GUG (73h) | GUG (73h) |
| UGC (4Dh) | CGC (5Dh) | AGC (6Dh) | GGC (7Dh) | | | | GGC (7Dh) | GCG (77h) | GCG (77h) | GCG (77h) | GCG (77h) |
| UGA (4Eh) | CGA (5Eh) | AGA (6Eh) | GGA (7Eh) | | | | GGA (7Eh) | GAG (7Bh) | GAG (7Bh) | GAG (7Bh) | GAG (7Bh) |
| UGG (4Fh) | CGG (5Fh) | AGG (6Fh) | GGG (7Fh) | | | | GGG (7Fh) | GGG (7F) | GGG (7F) | GGG (7F) | GGG (7F) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

EP 3 848 935 A1

# FIG.13

140g

DELETION TRANSITION
TABLE

55U

TRANSITION TABLE

55C

TRANSITION TABLE

55A

TRANSITION TABLE

55G

TRANSITION TABLE

# FIG.14A

ς55U

| MUTATION n CODON | | | | MUTATION n+1 CODON | | | | MUTANT n+1 CODON (DELETION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| UUU (40h) | CUU (50h) | AUU (60h) | GUU (70h) | UUU (40h) | CUU (50h) | AUU (60h) | GUU (70h) | UUU (40h) | UCU (44h) | UAU (48h) | UGU (4Ch) |
| | | | | UUC (41h) | CUC (51h) | AUC (61h) | GUC (71h) | UUU (40h) | UCU (44h) | UAU (48h) | UGU (4Ch) |
| | | | | UUA (42h) | CUA (52h) | AUA (62h) | GUA (72h) | UUU (40h) | UCU (44h) | UAU (48h) | UGU (4Ch) |
| | | | | UUG (43h) | CUG (53h) | AUG (63h) | GUG (73h) | UUU (40h) | UCU (44h) | UAU (48h) | UGU (4Ch) |
| UCU (44h) | CCU (54h) | ACU (64h) | GCU (74h) | UCU (44h) | CCU (54h) | ACU (64h) | GCU (74h) | UUC (41h) | UCC (45h) | UAC (49h) | UGC (4Dh) |
| | | | | UCC (45h) | CCC (55h) | ACC (65h) | GCC (75h) | UUC (41h) | UCC (45h) | UAC (49h) | UGC (4Dh) |
| | | | | UCA (46h) | CCA (56h) | ACA (66h) | GCA (76h) | UUC (41h) | UCC (45h) | UAC (49h) | UGC (4Dh) |
| | | | | UCG (47h) | CCG (57h) | ACG (67h) | GCG (77h) | UUC (41h) | UCC (45h) | UAC (49h) | UGC (4Dh) |
| UAU (48h) | CAU (58h) | AAU (68h) | GAU (78h) | UAU (48h) | CAU (58h) | AAU (68h) | GAU (78h) | UUA (42h) | UCA (46h) | UAA (4Ah) | UGA (4Eh) |
| | | | | UAC (49h) | CAC (59h) | AAC (69h) | GAC (79h) | UUA (42h) | UCA (46h) | UAA (4Ah) | UGA (4Eh) |
| | | | | UAA (4Ah) | CAA (5Ah) | AAA (6Ah) | GAA (7Ah) | UUA (42h) | UCA (46h) | UAA (4Ah) | UGA (4Eh) |
| | | | | UAG (4Bh) | CAG (5Bh) | AAG (6Bh) | GAG (7Bh) | UUA (42h) | UCA (46h) | UAA (4Ah) | UGA (4Eh) |
| UGU (4Ch) | CGU (5Ch) | AGU (6Ch) | GGU (7Ch) | UGU (4Ch) | CGU (5Ch) | AGU (6Ch) | GGU (7Ch) | UUG (43h) | UCG (47h) | UAG (4Bh) | UGG (4Fh) |
| | | | | UGC (4Dh) | CGC (5Dh) | AGC (6Dh) | GGC (7Dh) | UUG (43h) | UCG (47h) | UAG (4Bh) | UGG (4Fh) |
| | | | | UGA (4Eh) | CGA (5Eh) | AGA (6Eh) | GGA (7Eh) | UUG (43h) | UCG (47h) | UAG (4Bh) | UGG (4Fh) |
| | | | | UGG (4Fh) | CGG (5Fh) | AGG (6Fh) | GGG (7Fh) | UUG (43h) | UCG (47h) | UAG (4Bh) | UGG (4Fh) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

EP 3 848 935 A1

# FIG.14B

EP 3 848 935 A1

55C

| MUTATION n CODON | | | | MUTATION n+1 CODON | | | | MUTANT n+1 CODON (DELETION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| | | | | UUU (40h) | CUU (50h) | AUU (60h) | GUU (70h) | CUU (50h) | CCU (54h) | CAU (58h) | CGU (5Ch) |
| UUC (41h) | CUC (51h) | AUC (61h) | GUC (71h) | UUC (41h) | CUC (51h) | AUC (61h) | GUC (71h) | CUU (50h) | CCU (54h) | CAU (58h) | CGU (5Ch) |
| | | | | UUA (42h) | CUA (52h) | AUA (62h) | GUA (72h) | CUU (50h) | CCU (54h) | CAU (58h) | CGU (5Ch) |
| | | | | UUG (43h) | CUG (53h) | AUG (63h) | GUG (73h) | CUU (50h) | CCU (54h) | CAU (58h) | CGU (5Ch) |
| | | | | UCU (44h) | CCU (54h) | ACU (64h) | GCU (74h) | CUC (51h) | CCC (55h) | CAC (59h) | CGC (5Dh) |
| UCC (45h) | CCC (55h) | ACC (65h) | GCC (75h) | UCC (45h) | CCC (55h) | ACC (65h) | GCC (75h) | CUC (51h) | CCC (55h) | CAC (59h) | CGC (5Dh) |
| | | | | UCA (46h) | CCA (56h) | ACA (66h) | GCA (76h) | CUC (51h) | CCC (55h) | CAC (59h) | CGC (5Dh) |
| | | | | UCG (47h) | CCG (57h) | ACG (67h) | GCG (77h) | CUC (51h) | CCC (55h) | CAC (59h) | CGC (5Dh) |
| | | | | UAU (48h) | CAU (58h) | AAU (68h) | GAU (78h) | CUA (52h) | CCA (56h) | CAA (5Ah) | CGA (5Eh) |
| UAC (49h) | CAC (59h) | AAC (69h) | GAC (79h) | UAC (49h) | CAC (59h) | AAC (69h) | GAC (79h) | CUA (52h) | CCA (56h) | CAA (5Ah) | CGA (5Eh) |
| | | | | UAA (4Ah) | CAA (5Ah) | AAA (6Ah) | GAA (7Ah) | CUA (52h) | CCA (56h) | CAA (5Ah) | CGA (5Eh) |
| | | | | UAG (4Bh) | CAG (5Bh) | AAG (6Bh) | GAG (7Bh) | CUA (52h) | CCA (56h) | CAA (5Ah) | CGA (5Eh) |
| | | | | UGU (4Ch) | CGU (5Ch) | AGU (6Ch) | GGU (7Ch) | CUG (53h) | CCG (57h) | CAG (5Bh) | CGG (5Fh) |
| UGC (4Dh) | CGC (5Dh) | AGC (6Dh) | GGC (7Dh) | UGC (4Dh) | CGC (5Dh) | AGC (6Dh) | GGC (7Dh) | CUG (53h) | CCG (57h) | CAG (5Bh) | CGG (5Fh) |
| | | | | UGA (4Eh) | CGA (5Eh) | AGA (6Eh) | GGA (7Eh) | CUG (53h) | CCG (57h) | CAG (5Bh) | CGG (5Fh) |
| | | | | UGG (4Fh) | CGG (5Fh) | AGG (6Fh) | GGG (7Fh) | CUG (53h) | CCG (57h) | CAG (5Bh) | CGG (5Fh) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

# FIG.14C

55A

| MUTATION n CODON | | | | MUTATION n+1 CODON | | | | MUTANT n+1 CODON (DELETION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| | | | | UUU (40h) | CUU (50h) | AUU (60h) | GUU (70h) | AUU (60h) | ACU (64h) | AAU (68h) | AGU (6Ch) |
| | | | | UUC (41h) | CUC (51h) | AUC (61h) | GUC (71h) | AUU (60h) | ACU (64h) | AAU (68h) | AGU (6Ch) |
| UUA (42h) | CUA (52h) | AUA (62h) | GUA (72h) | UUA (42h) | CUA (52h) | AUA (62h) | GUA (72h) | AUU (60h) | ACU (64h) | AAU (68h) | AGU (6Ch) |
| | | | | UUG (43h) | CUG (53h) | AUG (63h) | GUG (73h) | AUU (60h) | ACU (64h) | AAU (68h) | AGU (6Ch) |
| | | | | UCU (44h) | CCU (54h) | ACU (64h) | GCU (74h) | AUC (61h) | ACC (65h) | AAC (69h) | AGC (6Dh) |
| | | | | UCC (45h) | CCC (55h) | ACC (65h) | GCC (75h) | AUC (61h) | ACC (65h) | AAC (69h) | AGC (6Dh) |
| UCA (46h) | CCA (56h) | ACA (66h) | GCA (76h) | UCA (46h) | CCA (56h) | ACA (66h) | GCA (76h) | AUC (61h) | ACC (65h) | AAC (69h) | AGC (6Dh) |
| | | | | UCG (47h) | CCG (57h) | ACG (67h) | GCG (77h) | AUC (61h) | ACC (65h) | AAC (69h) | AGC (6Dh) |
| | | | | UAU (48h) | CAU (58h) | AAU (68h) | GAU (78h) | AUA (62h) | ACA (66h) | AAA (6Ah) | AGA (6Eh) |
| | | | | UAC (49h) | CAC (59h) | AAC (69h) | GAC (79h) | AUA (62h) | ACA (66h) | AAA (6Ah) | AGA (6Eh) |
| UAA (4Ah) | CAA (5Ah) | AAA (6Ah) | GAA (7Ah) | UAA (4Ah) | CAA (5Ah) | AAA (6Ah) | GAA (7Ah) | AUA (62h) | ACA (66h) | AAA (6Ah) | AGA (6Eh) |
| | | | | UAG (4Bh) | CAG (5Bh) | AAG (6Bh) | GAG (7Bh) | AUA (62h) | ACA (66h) | AAA (6Ah) | AGA (6Eh) |
| | | | | UGU (4Ch) | CGU (5Ch) | AGU (6Ch) | GGU (7Ch) | AUG (63h) | ACG (67h) | AAG (6Bh) | AGG (6Fh) |
| | | | | UGC (4Dh) | CGC (5Dh) | AGC (6Dh) | GGC (7Dh) | AUG (63h) | ACG (67h) | AAG (6Bh) | AGG (6Fh) |
| UGA (4Eh) | CGA (5Eh) | AGA (6Eh) | GGA (7Eh) | UGA (4Eh) | CGA (5Eh) | AGA (6Eh) | GGA (7Eh) | AUG (63h) | ACG (67h) | AAG (6Bh) | AGG (6Fh) |
| | | | | UGG (4Fh) | CGG (5Fh) | AGG (6Fh) | GGG (7Fh) | AUG (63h) | ACG (67h) | AAG (6Bh) | AGG (6Fh) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

# FIG.14D

EP 3 848 935 A1

55G

| MUTATION n CODON | | | | MUTATION n+1 CODON | | | | MUTANT n+1 CODON (DELETION) | | | |
|---|---|---|---|---|---|---|---|---|---|---|---|
| 4 (0100) | 5 (0101) | 6 (0110) | 7 (0111) | 4 (0100) | 5 (0101) | 6 (0110) | 7 (0111) | | | | |
| | | | | UUU (40h) | CUU (50h) | AUU (60h) | GUU (70h) | GUU (70h) | GCU (74h) | GAU (78h) | GGU (7Ch) |
| | | | | UUC (41h) | CUC (51h) | AUC (61h) | GUC (71h) | GUU (70h) | GCU (74h) | GAU (78h) | GGU (7Ch) |
| UUG (43h) | CUG (53h) | AUG (63h) | GUG (73h) | UUA (42h) | CUA (52h) | AUA (62h) | GUA (72h) | GUU (70h) | GCU (74h) | GAU (78h) | GGU (7Ch) |
| | | | | UUG (43h) | CUG (53h) | AUG (63h) | GUG (73h) | GUU (70h) | GCU (74h) | GAU (78h) | GGU (7Ch) |
| | | | | UCU (44h) | CCU (54h) | ACU (64h) | GCU (74h) | GUC (71h) | GCC (75h) | GAC (79) | GGC (7Dh) |
| | | | | UCC (45h) | CCC (55h) | ACC (65h) | GCC (75h) | GUC (71h) | GCC (75h) | GAC (79) | GGC (7Dh) |
| UCG (47h) | CCG (57h) | ACG (67h) | GCG (77h) | UCA (46h) | CCA (56h) | ACA (66h) | GCA (76h) | GUC (71h) | GCC (75h) | GAC (79) | GGC (7Dh) |
| | | | | UCG (47h) | CCG (57h) | ACG (67h) | GCG (77h) | GUC (71h) | GCC (75h) | GAC (79) | GGC (7Dh) |
| | | | | UAU (48h) | CAU (58h) | AAU (68h) | GAU (78h) | GUA (72h) | GCA (76h) | GAA (7Ah) | GGA (7Eh) |
| | | | | UAC (49h) | CAC (59h) | AAC (69h) | GAC (79h) | GUA (72h) | GCA (76h) | GAA (7Ah) | GGA (7Eh) |
| UAG (4Bh) | CAG (5Bh) | AAG (6Bh) | GAG (7Bh) | UAA (4Ah) | CAA (5Ah) | AAA (6Ah) | GAA (7Ah) | GUA (72h) | GCA (76h) | GAA (7Ah) | GGA (7Eh) |
| | | | | UAG (4Bh) | CAG (5Bh) | AAG (6Bh) | GAG (7Bh) | GUA (72h) | GCA (76h) | GAA (7Ah) | GGA (7Eh) |
| | | | | UGU (4Ch) | CGU (5Ch) | AGU (6Ch) | GGU (7Ch) | GUG (73h) | GCG (77h) | GAG (7Bh) | GGG (7F) |
| | | | | UGC (4Dh) | CGC (5Dh) | AGC (6Dh) | GGC (7Dh) | GUG (73h) | GCG (77h) | GAG (7Bh) | GGG (7F) |
| UGG (4Fh) | CGG (5Fh) | AGG (6Fh) | GGG (7Fh) | UGA (4Eh) | CGA (5Eh) | AGA (6Eh) | GGA (7Eh) | GUG (73h) | GCG (77h) | GAG (7Bh) | GGG (7F) |
| | | | | UGG (4Fh) | CGG (5Fh) | AGG (6Fh) | GGG (7Fh) | GUG (73h) | GCG (77h) | GAG (7Bh) | GGG (7F) |
| 1ST COLUMN | 2ND COLUMN | 3RD COLUMN | 4TH COLUMN | 1ST COLUMN | 2ND COLUMN | 3RD COLUMN | 4TH COLUMN | 1ST COLUMN | 2ND COLUMN | 3RD COLUMN | 4TH COLUMN |

## FIG.15

```
          ( START )
              │
              ▼  S101
┌─────────────────────────────┐
│ RECEIVE REFERENCE CODON      │
│ SEQUENCE DATA AND ANALYSIS-  │
│ TARGET CODON SEQUENCE DATA   │
└─────────────────────────────┘
              │
              ▼  S102
┌─────────────────────────────┐
│ ENCODE REFERENCE CODON       │
│ SEQUENCE DATA AND ANALYSIS-  │
│ TARGET CODON SEQUENCE DATA,  │
│ AND GENERATE FIRST-TYPE      │
│ SEQUENCE DATA AND SECOND-TYPE│
│ SEQUENCE DATA,RESPECTIVELY   │
└─────────────────────────────┘
              │
              ▼  S103
┌─────────────────────────────┐
│ COMPARE FIRST-TYPE SEQUENCE  │
│ DATA AND SECOND-TYPE SEQUENCE│
│ DATA IN UNITS OF CODONS      │
│ (SINGLE BYTES),AND IDENTIFY  │
│ MUTATION POSITION HAVING     │
│ NONIDENTICAL CODONS          │
└─────────────────────────────┘
              │
              ▼  S104
┌─────────────────────────────┐
│ BASED ON MUTATION POSITION,  │
│ IDENTIFY FIRST-TYPE MUTANT   │
│ CODON,MUTANT n CODON,AND     │
│ MUTANT n+1 CODON IN FIRST-   │
│ TYPE SEQUENCE DATA,AND       │
│ IDENTIFY SECOND-TYPE MUTATION│
│ CODON, MUTATION n CODON,AND  │
│ MUTATION n+1 CODON           │
└─────────────────────────────┘
              │
              ▼  S105
```

IN INSERTION TRANSITION TABLE, IS PRE-BASE-INSERTION MUTANT n CODON,WHICH IS IDENTIFIED FROM MUTATION n CODON AND MUTATION n+1 CODON, IDENTICAL TO SUBSEQUENT CODON OF FIRST-TYPE MUTANT CODON? — NO

YES
S106
IDENTIFY "BASE INSERTION" AS TYPE OF POINT MUTATION

S107
IN DELETION TRANSITION TABLE, IS PRE-BASE-INSERTION MUTANT n CODON,WHICH IS IDENTIFIED FROM MUTATION n CODON AND MUTATION n+1 CODON, IDENTICAL TO CODON POSITIONED AFTER SUBSEQUENT CODON OF FIRST-TYPE MUTANT CODON? — NO

YES
S108
IDENTIFY "BASE DELETION" AS TYPE OF POINT MUTATION

S109
IDENTIFY "BASE SUBSTITUTION" AS TYPE OF POINT MUTATION

S110
IN DETECTION RESULT TABLE,REGISTER INFORMATION ABOUT IDENTIFIED TYPE OF POINT MUTATION

S111
OUTPUT DETECTION RESULT TABLE

( END )

FIG.16

EP 3 848 935 A1

140e
··· | AUG (63h) | GUC (71h) | CAA (5Ah) | GUG (73h) | CAU (48h) | ··· | GUC (71h) | GAA (7Ah) | ··· | UGA (4Eh)

$P_{40}$   $P_{41}$

240e
··· | AUG (63h) | GUC (71h) | AAG (6Bh) | UGC (4Dh) | ··· | UCG (47h) | AAA (6Ah) | ··· | UGA (4Eh)

COM-PARISON   $P_{42}$   $P_{43}$

140d
··· | AUG (63h) | UCC (45h) | AAG (6Bh) | UGC (4Dh) | ··· | UCC (45h) | AAG (6Bh) | ··· | UGA (4Eh)

## FIG.17

| ··· | AUG (63h) | UCA (40h) | AGU (63h) | GCU (74h) | ··· | CGA (5Eh) | AAU (68h) | ··· | UGA (N) 4Eh |

140e

P50        P51

| ··· | AUG (63h) | UCA (40h) | AGU (63h) | UGC (4Dh) | ··· | UCG (47h) | AAA (6Ah) | ··· | UGA (4Eh) |

240e

COM-PARISON        P52        P53

| ··· | AUG (63h) | UCC (45h) | AAG (5Ah) | UGC (4Dh) | ··· | UCC (45h) | AAG (6Bh) | ··· | UGA (4Eh) |

140d

EP 3 848 935 A1

# FIG.18

140e

| ··· | AUG (63h) | GCC (75h) | AAG (6Bh) | ··· | UCG (47h) | AAA (74h) | ··· | UGA (4Eh) |

P60    P61

240e

| ··· | AUG (63h) | GCC (75h) | AAG (6Bh) | ··· | UCG (47h) | AAA (6Ah) | ··· | UGA (4Eh) |

COM-PARISON

P62    P63

140d

| ··· | AUG (63h) | UCC (45h) | AAG (6Bh) | ··· | UCC (45h) | AAG (6Bh) | ··· | UGA (4Eh) |

# FIG.19

INFORMATION PROCESSING DEVICE ⌇200

CONTROL UNIT ⌇250

MEMORY UNIT ⌇240

RECEIVING UNIT ⌇150a

ENCODING UNIT ⌇150b

COMPARING UNIT ⌇150c

IDENTIFYING UNIT ⌇250d

COMMUNICA-TION UNIT ⌇110

INPUT UNIT ⌇120

DISPLAY UNIT ⌇130

REFERENCE CODON SEQUENCE DATA ⌇140a

ANALYSIS-TARGET CODON SEQUENCE DATA ⌇140b

CODE CONVERSION TABLE ⌇140c

FIRST-TYPE SEQUENCE DATA ⌇140d

SECOND-TYPE SEQUENCE DATA ⌇140e

INSERTION TRANSITION TABLE ⌇140f

DELETION TRANSITION TABLE ⌇140g

THIRD-TYPE SEQUENCE DATA ⌇240e

DETECTION RESULT TABLE ⌇240h

# FIG.20

START

S201

RECEIVE REFERENCE CODON SEQUENCE DATA AND
ANALYSIS-TARGET CODON SEQUENCE DATA

S202

ENCODE REFERENCE CODON SEQUENCE DATA AND ANALYSIS-TARGET
CODON SEQUENCE DATA,AND GENERATE FIRST-TYPE SEQUENCE DATA
AND SECOND-TYPE SEQUENCE DATA,RESPECTIVELY

S203

COMPARE FIRST-TYPE SEQUENCE DATA AND SECOND-TYPE SEQUENCE
DATA IN UNITS OF CODONS(SINGLE BYTES),AND IDENTIFY MUTATION
POSITION HAVING NONIDENTICAL CODONS

S204

IDENTIFY TYPE OF POINT MUTATION

S205

BASED ON TYPE OF POINT MUTATION,GENERATE THIRD-TYPE
SEQUENCE DATA BY CORRECTING SECOND-TYPE SEQUENCE DATA

S206

COMPARE FIRST-TYPE SEQUENCE DATA AND THIRD-TYPE SEQUENCE
DATA,AND IDENTIFY GENETIC MUTATION

S207

IN DETECTION RESULT TABLE,REGISTER INFORMATION ABOUT
IDENTIFIED TYPE OF MUTATION AND IDENTIFIED GENETIC MUTATION

S208

OUTPUT DETECTION RESULT TABLE

END

# FIG.21A

EP 3 848 935 A1

⌐240i

## CODON-AMINO ACID CONVERSION TABLE

| CODON | AMINO ACID | | CODON | AMINO ACID | | CODON | AMINO ACID | | CODON | AMINO ACID |
|-------|------------|--|-------|------------|--|-------|------------|--|-------|------------|
| UUU (40h) | Phe (46h) | | CUU (50h) | Leu (4Ch) | | AUU (60h) | Ile (49h) | | GUU (70h) | Val (56h) |
| UUC (41h) | Phe (46h) | | CUC (51h) | Leu (4Ch) | | AUC (61h) | Ile (49h) | | GUC (71h) | Val (56h) |
| UUA (42h) | Leu (4Ch) | | CUA (52h) | Leu (4Ch) | | AUA (62h) | Ile (49h) | | GUA (72h) | Val (56h) |
| UUG (43h) | Leu (4Ch) | | CUG (53h) | Leu (4Ch) | | AUG (63h) | Met (4Dh) | | GUG (73h) | Val (56h) |
| UCU (44h) | Ser (53h) | | CCU (54h) | Pro (50h) | | ACU (64h) | Thr (54h) | | GCU (74h) | Ala (41h) |
| UCC (45h) | Ser (53h) | | CCC (55h) | Pro (50h) | | ACC (65h) | Thr (54h) | | GCC (75h) | Ala (41h) |
| UCA (46h) | Ser (53h) | | CCA (56h) | Pro (50h) | | ACA (66h) | Thr (54h) | | GCA (76h) | Ala (41h) |
| UCG (47h) | Ser (53h) | | CCG (57h) | Pro (50h) | | ACG (67h) | Thr (5Bh) | | GCG (77h) | Ala (41h) |
| UAU (48h) | Tvr (59h) | | CAU (58h) | His (48h) | | AAU (68h) | His (48h) | | GAU (78h) | Asp (44h) |
| UAC (49h) | Tvr (59h) | | CAC (59h) | His (48h) | | AAC (69h) | His (48h) | | GAC (79h) | Asp (44h) |
| UAA (4Ah) | stop (40h) | | CAA (5Ah) | Gln (51h) | | AAA (6Ah) | Gln (51h) | | GAA (7Ah) | Glu (45h) |
| UAG (4Bh) | stop (40h) | | CAG (5Bh) | Gln (51h) | | AAG (6Bh) | Gln (51h) | | GAG (7Bh) | Glu (45h) |
| UGU (4Ch) | Cys (43h) | | CGU (5Ch) | Arg (52h) | | AGU (6Ch) | Ser (53h) | | GGU (7Ch) | Gly (47h) |
| UGC (4Dh) | Cys (43h) | | CGC (5Dh) | Arg (52h) | | AGC (6Dh) | Ser (53h) | | GGC (7Dh) | Gly (47h) |
| UGA (4Eh) | stop (40h) | | CGA (5Eh) | Arg (52h) | | AGA (6Eh) | Arg (52h) | | GGA (7Eh) | Gly (47h) |
| UGG (4Fh) | Trp (57h) | | CGG (5Fh) | Arg (52h) | | AGG (6Fh) | Arg (52h) | | GGG (7Fh) | Gly (47h) |

# FIG.21B

| ··· | AUG (63h) | UUU (40h) | UCC (45h) | AAG (6Bh) | ··· | ··· | UGA (4Eh) |

140d

CODON-AMINO ACID CONVERSION TABLE

240i

| ··· | Met (4Dh) | Phe (46h) | Ser (53h) | Lys (4Bh) | ··· | ··· | stop (40h) |

240j

$P_{25}$

| ··· | Met (4Dh) | Phe (46h) | Val (56h) | Gln (51h) | Val (56h) | ··· | stop (40h) |

140d

NOT IDENTICAL

EP 3 848 935 A1

# FIG.22

START

S210

RECEIVE REFERENCE CODON SEQUENCE DATA

S211

ENCODE REFERENCE CODON SEQUENCE DATA AND
GENERATE FIRST-TYPE SEQUENCE DATA

S212

RECEIVE ANALYSIS-TARGET CODON SEQUENCE
DATA

S213

ENCODE ANALYSIS-TARGET CODON SEQUENCE
DATA AND GENERATE SECOND-TYPE SEQUENCE
DATA

S214

BASED ON CODON-AMINO ACID CONVERSION TABLE,
GENERATE FOURTH-TYPE SEQUENCE DATA FROM
FIRST-TYPE SEQUENCE DATA

S215

COMPARE FOURTH-TYPE SEQUENCE DATA AND
SECOND-TYPE SEQUENCE DATA IN UNITS OF AMINO
ACIDS,AND IDENTIFY MUTATION POSITION

S216

IN DETECTION RESULT TABLE,REGISTER
INFORMATION ABOUT IDENTIFIED MUTATION
POSITION

S217

OUTPUT DETECTION RESULT TABLE

END

# FIG.23

CODON
SEQUENCE     6       7       8       9

140d

| ... | AUG (63h) | UUU (40h) | UCC (45h) | AAG (6Bh) | ... | UGA (4Eh) |

⬇

              ...      8   7   6   ...

OFFSET

| | | | 1 | | AUG (63h) |
|---|---|---|---|---|---|
| | | | | | ... |
| | | 1 | | | UUU (40h) |
| | | | | | ... |
| | 1 | | | | UCC (45h) |
| | | | | | ... |
| 1 | | | | | GUC (73h) |
| | | | | | ... |

340a

FIG.24

CODON
SEQUENCE        6          7          8          9

140e

| ··· | AUG (63h) | UUU (40h) | GUC (71h) | CAA (5Ah) | ··· | UGA (4Eh) |

COM-
PARISON

340a

INVERTED INDEX

EXTRAC-
TION

··· 35 34 33 32  31 30 29 28  27 26 25 24  23 22 21 20  19 18 17 16  15 14 13 12  11 10 9 8  7 **6** 5 4  3 2 1 0

b10

| 0 0 0 0 | 0 0 0 0 | 0 0 0 0 | 0 0 0 0 | 0 0 0 0 | 0 0 0 0 | 0 0 0 0 | 0 0 0 0 | 0 **1** 0 0 | 0 0 0 0 |

b11

| 0 0 0 0 | 0 0 0 0 | 0 0 0 0 | 0 0 0 0 | 0 0 0 0 | 0 0 0 0 | 0 0 0 0 | 0 0 0 0 | **1** 0 0 0 | 0 0 0 0 |

b12

| 0 0 0 0 | 0 0 0 0 | 0 0 0 0 | 0 0 0 0 | 0 0 0 **1** | 0 0 0 0 | 0 0 0 0 | 0 0 0 0 | 0 0 0 0 | 0 0 0 0 |

:

CODON "GUC (71h)"
AT OFFSET 8 IS
NOT IDENTICAL

EP 3 848 935 A1

# FIG.25

**INFORMATION PROCESSING DEVICE** ⟋300

## CONTROL UNIT ⟋350

- RECEIVING UNIT ⟋150a
- ENCODING UNIT ⟋150b
- GENERATING UNIT ⟋350a
- OBTAINING UNIT ⟋350b
- IDENTIFYING UNIT ⟋350c

## MEMORY UNIT ⟋340

- REFERENCE CODON SEQUENCE DATA ⟋140a
- ANALYSIS-TARGET CODON SEQUENCE DATA ⟋140b
- CODE CONVERSION TABLE ⟋140c
- FIRST-TYPE SEQUENCE DATA ⟋140d
- INVERTED INDEX ⟋340a
- SECOND-TYPE SEQUENCE DATA ⟋140e
- INSERTION TRANSITION TABLE ⟋140f
- DELETION TRANSITION TABLE ⟋140g
- THIRD-TYPE SEQUENCE DATA ⟋240e
- DETECTION RESULT TABLE ⟋240h

- COMMUNICA-TION UNIT ⟋110
- INPUT UNIT ⟋120
- DISPLAY UNIT ⟋130

# FIG.26

# FIG.27

◇RESTORATION 1 (EXPANSION)

BASE=29

◇RESTORATION 2 (AND OPERATION)

EP 3 848 935 A1

# FIG.28

| ... | AUG (63h) | UUU (40h) | GUC (71h) | CAA (5Ah) | ... | UGA (4Eh) | 140e |

NOT IDENTICAL

| ... | AUG (63h) | UUU (40h) | UCC (45h) | AAG (6Bh) | ... | UGA (4Eh) | 140d |

6     7     8     9     ...

→ OFFSET

... 35 34 33 32   31 30 29 28   27 26 25 24   23 22 21 20   19 18 17 16   15 14 13 12   11 10 9 8   7 **6** 5 4   3 2 1 0   b10

| 0 0 0 0 | 0 0 0 0 | 0 0 0 0 | 0 0 0 0 | 0 0 0 0 | 0 0 0 0 | 0 0 0 0 | 0 0 0 0 | 0 **1** 0 0 | 0 0 0 0 |

←

... 35 34 33 32   31 30 29 28   27 26 25 24   23 22 21 20   19 18 17 16   15 14 13 12   11 10 9 8   **7** 6 5 4   3 2 1 0   b10-1 (S10)

| 0 0 0 0 | 0 0 0 0 | 0 0 0 0 | 0 0 0 0 | 0 0 0 0 | 0 0 0 0 | 0 0 0 0 | 0 0 0 0 | **1** 0 0 0 | 0 0 0 0 |

... 35 34 33 32   31 30 29 28   27 26 25 24   23 22 21 20   19 18 17 16   15 14 13 12   11 10 9 8   **7** 6 5 4   3 2 1 0   b11

| 0 0 0 0 | 0 0 0 0 | 0 0 0 0 | 0 0 0 0 | 0 0 0 0 | 0 0 0 0 | 0 0 0 0 | 0 0 0 0 | **1** 0 0 0 | 0 0 0 0 |

... 35 34 33 32   31 30 29 28   27 26 25 24   23 22 21 20   19 18 17 16   15 14 13 12   11 10 9 8   **7** 6 5 4   3 2 1 0   b11-1 (S11)

| 0 0 0 0 | 0 0 0 0 | 0 0 0 0 | 0 0 0 0 | 0 0 0 0 | 0 0 0 0 | 0 0 0 0 | 0 0 0 0 | **1** 0 0 0 | 0 0 0 0 |

←

... 35 34 33 32   31 30 29 28   27 26 25 24   23 22 21 20   19 18 17 16   15 14 13 12   11 10 9 **8**   7 6 5 4   3 2 1 0   b11-2 (S12)

| 0 0 0 0 | 0 0 0 0 | 0 0 0 0 | 0 0 0 0 | 0 0 0 0 | 0 0 0 0 | 0 0 0 0 | 0 0 0 **1** | 0 0 0 0 | 0 0 0 0 |

... 35 34 33 32   31 30 29 28   27 26 25 24   23 22 21 20   19 18 17 16   15 14 13 12   11 10 9 **8**   7 6 5 4   3 2 1 0   b12

| 0 0 0 0 | 0 0 0 0 | 0 0 0 0 | 0 0 0 0 | 0 0 0 **1** | 0 0 0 0 | 0 0 0 0 | 0 0 0 0 | 0 0 0 0 | 0 0 0 0 |

... 35 34 33 32   31 30 29 28   27 26 25 24   23 22 21 20   19 18 17 16   15 14 13 12   11 10 9 **8**   7 6 5 4   3 2 1 0   b12-1 (S13)

| 0 0 0 0 | 0 0 0 0 | 0 0 0 0 | 0 0 0 0 | 0 0 0 0 | 0 0 0 0 | 0 0 0 0 | 0 0 0 **0** | 0 0 0 0 | 0 0 0 0 |

NOT IDENTICAL

# FIG.29

START

S301

RECEIVE REFERENCE CODON SEQUENCE DATA AND
ANALYSIS-TARGET CODON SEQUENCE DATA

S302

ENCODE REFERENCE CODON SEQUENCE DATA AND GENERATE FIRST-
TYPE SEQUENCE DATA,AS WELL AS GENERATE INVERTED INDEX AT
SAME TIME

S303

ENCODE ANALYSIS-TARGET CODON SEQUENCE DATA AND
GENERATE SECOND-TYPE SEQUENCE DATA

S304

COMPARE ENCODED CODONS IN SECOND-TYPE SEQUENCE DATA AND
INVERTED INDEX,AND SEQUENTIALLY OBTAIN BITMAPS
CORRESPONDING TO CODONS

S305

PERFORM SHIFTING OF BITMAPS,PERFORM "AND" OPERATIONS,
AND IDENTIFY MUTATION POSITION HAVING NON-IDENTICALNESS

S306

IDENTIFY TYPE OF POINT MUTATION

S307

BASED ON TYPE OF POINT MUTATION,GENERATE THIRD-TYPE
SEQUENCE DATA BY CORRECTING SECOND-TYPE SEQUENCE DATA

S308

COMPARE ENCODED CODONS IN THIRD-TYPE SEQUENCE DATA AND
INVERTED INDEX,AND SEQUENTIALLY OBTAIN BITMAPS
CORRESPONDING TO CODONS

S309

PERFORM SHIFTING OF BITMAPS,PERFORM "AND" OPERATIONS,
AND IDENTIFY REFERENCE POSITION HAVING NON-IDENTICALNESS AND
IDENTIFY GENETIC MUTATION

S310

IN DETECTION RESULT TABLE,REGISTER INFORMATION ABOUT
IDENTIFIED TYPE OF POINT MUTATION AND IDENTIFIED GENETIC
MUTATION

S311

OUTPUT DETECTION RESULT TABLE

END

# FIG.30

START

S401
SET OFFSET n AS OFFSET OF START CODON

S402
FROM INVERTED INDEX,EXTRACT FIRST BITMAP CORRESPONDING TO CODON AT OFFSET n IN SECOND-TYPE SEQUENCE DATA

S403
PERFORM LEFT-SIDE SHIFTING OF FIRST BITMAP

S404
INCREMENT OFFSET n BY ONE

S405
FROM INVERTED INDEX,OBTAIN SECOND BITMAP CORRESPONDING TO CODON AT OFFSET n IN SECOND-TYPE SEQUENCE DATA

S406
PERFORM "AND" OPERATION OF FIRST-TYPE SEQUENCE DATA AND THIRD-TYPE SEQUENCE DATA,AND GENERATE THIRD BITMAP

S407
DETERMINE WHETHER OR NOT BIT OF OFFSET n IN THIRD BITMAP IS SET TO "1"

S408
1?

YES

NO

S410
UPDATE FIRST BITMAP WITH BITMAP OBTAINED BY PERFORMING LEFT-SIDE SHIFTING OF THIRD BITMAP

S409
DETERMINE THAT POINT MUTATION HAS OCCURRED AT OFFSET n IN SECOND-TYPE SEQUENCE DATA

END

# FIG.31

CODON SEQUENCE

| | ... | AUG (63h) | UUU (40h) | UCC (45h) | AAG (6Bh) | ... | UGA (4Eh) |

140d

| | ... | Met (4D) | Phe (46h) | Ser (53h) | Lys (4Bh) | ... | stop (40h) |

240j

OFFSET

Met (4Dh)
...
Phe (46h)
...
Ser (53h)
...
Ser (53h)
...

340b

# FIG.32

START

S410

RECEIVE REFERENCE CODON SEQUENCE DATA

S402

ENCODE REFERENCE CODON SEQUENCE DATA AND GENERATE FIRST-TYPE SEQUENCE DATA,AS WELL AS GENERATE INVERTED INDEX CORRESPONDING TO CODONS AT SAME TIME

S403

RECEIVE AMINO-ACID SEQUENCE DATA TO BE ANALYZED

S404

ENCODE AMINO-ACID SEQUENCE DATA TO BE ANALYZED,AND GENERATE SECOND-TYPE SEQUENCE DATA

S405

BASED ON CODON-AMINO ACID CONVERSION TABLE, GENERATE FOURTH-TYPE SEQUENCE DATA FROM FIRST-TYPE SEQUENCE DATA,AS WELL AS GENERATE INVERTED INDEX CORRESPONDING TO AMINO ACIDS

S406

PERFORM SHIFTING OF BITMAPS,PERFORM "AND" OPERATIONS,AND IDENTIFY MUTATION POSITION HAVING NON-IDENTICALNESS

S407

IN DETECTION RESULT TABLE,REGISTER INFORMATION ABOUT IDENTIFIED POINT MUTATION

S408

OUTPUT DETECTION RESULT TABLE

END

# FIG.33

COMPUTER — 400

| CPU — 401 | TOUCH-SENSITIVE PANEL — 402 | DISPLAY — 403 | READING DEVICE — 404 | INTERFACE DEVICE — 405 |

— 408

RAM — 406

| RECEIVING PROCESS | 406a |
| ENCODING PROCESS | 406b |
| COMPARISON PROCESS | 406c |
| IDENTIFICATION PROCESS | 406d |

HARD DISK DEVICE — 407

| RECEIVING PROGRAM | 407a |
| ENCODING PROGRAM | 407b |
| COMPARISON PROGRAM | 407c |
| IDENTIFICATION PROGRAM | 407d |

EP 3 848 935 A1

# FIG.34

COMPUTER — 500

CPU — 501

TOUCH-SENSITIVE PANEL — 502

DISPLAY — 503

READING DEVICE — 504

INTERFACE DEVICE — 505

508

RAM — 506
- RECEIVING PROCESS — 506a
- ENCODING PROCESS — 506b
- GENERATION PROCESS — 506c
- OBTAINING PROCESS — 506d
- IDENTIFICATION PROCESS — 506e

HARD DISK DEVICE — 507
- RECEIVING PROGRAM — 507a
- ENCODING PROGRAM — 507b
- GENERATION PROGRAM — 507c
- OBTAINING PROGRAM — 507d
- IDENTIFICATION PROGRAM — 507e

EP 3 848 935 A1

# FIG.35

| AMINO ACID | | | BASE (THREE BASE SEQUENCES:CODON) | | | |
|---|---|---|---|---|---|---|
| NAME | AB-BRE-VIA-TION | SYM-BOL | | | | |
| ALANINE | Ala | A | GCU | GCC | GCA | GCG |
| CYSTEINE | Cys | C | UGU | UGC | | |
| ASPARAGINE ACID | Asp | D | GAU | GAC | | |
| GLUTAMINE ACID | Glu | E | | | GAA | GAG |
| PHENYLALANINE | Phe | F | UUU | UUC | | |
| GLYCINE | Gly | G | GGU | GGC | GGA | GGG |
| HISTIDINE | His | H | CAU | CAC | | |
| ISOLEUCINE | Ile | I | AUU | AUC | AUA | |
| LYSINE | Lys | K | | | AAA | AAG |
| LEUCINE | Leu | L | CUU | CUC | UUA<br>CUA | UUG<br>CUG |
| METHIONINE (START) | Met | M | | | | AUG |
| ASPARAGINE | Asn | N | AAU | AAC | | |
| PROLINE | Pro | P | CCU | CCC | CCA | CCG |
| GLUTAMINE | Gln | Q | | | CAA | CAG |
| ARGININE | Arg | R | CGU<br>AGA | CGC | CGA | CGG<br>AGG |
| SERINE | Ser | S | UCU<br>AGU | UCC<br>AGC | UCA | UCG |
| THREONINE | Thr | T | ACU | ACC | ACA | ACG |
| VALINE | Val | V | GUU | GUC | GUA | GUG |
| TRYPTOPHAN | Trp | W | | | | UGG |
| TYROSINE | Tyr | Y | UAU | UAC | | |
| (TERMINATION) | | | | | UAA | UAG |
| (TERMINATION) | | | | | UGA | |
| UNDECIDED AMINO ACID | Xxx | X | | | | |
| EITHER GLUTAMINE ACID OR GLUTAMINE) | Glx | Z | | | | |

# FIG.36

| | Ala<br>A | Arg<br>R | Asn<br>N | Asp<br>D | Cys<br>C | Cln<br>Q | Clu<br>E | Cly<br>G | His<br>H | Ile<br>I | Leu<br>L | Lys<br>K | Met<br>M | Phe<br>F | Pro<br>P | Ser<br>S | Thr<br>T | Trp<br>W | Tyr<br>Y | Val<br>V |
|---|---|---|---|---|---|---|---|---|---|---|---|---|---|---|---|---|---|---|---|---|
| A | 5 | -2 | -1 | -2 | -1 | -1 | -1 | 0 | -2 | -1 | -2 | -1 | -1 | -3 | -1 | 1 | 0 | -3 | -2 | 0 |
| R | -2 | 7 | -1 | -2 | -4 | 1 | 0 | -3 | 0 | -4 | -3 | 3 | -2 | -3 | -3 | -1 | -1 | -3 | -1 | -3 |
| N | -1 | -1 | 7 | 2 | -2 | 0 | 0 | 0 | 1 | -3 | -4 | 0 | -2 | -4 | -2 | 1 | 0 | -4 | -2 | -3 |
| D | -2 | -2 | 2 | 8 | -4 | 0 | 2 | -1 | -1 | -4 | -4 | -1 | -4 | -5 | -1 | 0 | -1 | -5 | -3 | -4 |
| C | -1 | -4 | -2 | -4 | 13 | -3 | -3 | -3 | -3 | -2 | -2 | -3 | -2 | -2 | -4 | -1 | -1 | -5 | -3 | -1 |
| Q | -1 | 1 | 0 | 0 | -3 | 7 | 2 | -2 | 1 | -3 | -2 | 2 | 0 | -4 | -1 | 0 | -1 | -1 | -1 | -3 |
| E | -1 | 0 | 0 | 2 | -3 | -2 | 6 | -3 | 0 | -4 | -3 | 1 | -2 | -3 | -1 | -1 | -1 | -3 | -2 | -3 |
| G | 0 | -3 | 0 | -1 | -3 | -2 | -3 | 8 | -2 | -4 | -4 | -2 | -3 | -4 | -2 | 0 | -2 | -3 | -3 | -4 |
| H | -2 | 0 | 1 | -1 | -3 | 1 | 0 | -2 | 10 | -4 | -3 | 0 | -1 | -1 | -2 | -1 | -2 | -3 | 2 | -4 |
| I | -1 | -4 | -3 | -4 | -2 | -3 | -4 | -4 | -4 | 5 | 2 | -3 | 2 | 0 | -3 | -3 | -1 | -3 | -1 | 4 |
| L | -2 | -3 | -4 | -4 | -2 | -2 | -3 | -4 | -3 | 2 | 5 | -3 | 3 | 1 | -4 | -3 | -1 | -2 | -1 | 1 |
| K | -1 | 3 | 0 | -1 | -3 | 2 | 1 | -2 | 0 | -3 | -3 | 6 | -2 | -4 | -1 | 0 | -1 | -3 | -2 | -3 |
| M | -1 | -2 | -2 | -4 | -2 | 0 | -2 | -3 | -1 | 2 | 3 | -2 | 7 | 0 | -3 | -2 | -1 | -1 | 0 | 1 |
| F | -3 | -3 | -4 | -5 | -2 | -4 | -3 | -4 | -1 | 0 | 1 | -4 | 0 | 8 | -4 | -3 | -2 | 1 | 4 | -1 |
| P | -1 | -3 | -2 | -1 | -4 | -1 | -1 | -2 | -2 | -3 | -4 | -1 | -3 | -4 | 10 | -1 | -1 | -4 | -3 | -3 |
| S | 1 | -1 | 1 | 0 | -1 | 0 | -1 | 0 | -1 | -3 | -3 | 0 | -2 | -3 | -1 | 5 | 2 | -4 | -2 | -2 |
| T | 0 | -1 | 0 | -1 | -1 | -1 | -1 | -2 | -2 | -1 | -1 | -1 | -1 | -2 | -1 | 2 | 5 | -3 | -2 | 0 |
| W | -3 | -3 | -4 | -5 | -5 | -1 | -3 | -3 | -3 | -3 | -2 | -3 | -1 | 1 | -4 | -4 | -3 | 15 | 2 | -3 |
| Y | -2 | -1 | -2 | -3 | -3 | -1 | -2 | -3 | 2 | -1 | -1 | -2 | 0 | 4 | -3 | -2 | -2 | 2 | 8 | -1 |
| V | 0 | -3 | -3 | -4 | -1 | -3 | -3 | -4 | -4 | 4 | 1 | -3 | 1 | -1 | -3 | -2 | 0 | -3 | -1 | 5 |

EP 3 848 935 A1

# FIG.37

|   | H | E | A | G | A | W | G | H | E | E |
|---|---|---|---|---|---|---|---|---|---|---|
|   | 0 | 0 | 0 | 0 | 0 | 0 | 0 | 0 | 0 | 0 | 0 |
| P | 0 | 0 | 0 | 0 | 0 | 0 | 0 | 0 | 0 | 0 | 0 |
| A | 0 | 0 | 0 | 5 | 0 | 5 | 0 | 0 | 0 | 0 | 0 |
| W | 0 | 0 | 0 | 0 | 2 | 5 | 20 | 12 | 4 | 0 | 0 |
| H | 0 | 10 | 2 | 0 | 0 | 0 | 12 | 18 | 22 | 14 | 6 |
| E | 0 | 2 | 16 | 8 | 0 | 0 | 4 | 10 | 18 | 28 | 20 |
| A | 0 | 0 | 8 | 21 | 13 | 5 | 0 | 4 | 10 | 20 | 27 |
| E | 0 | 0 | 6 | 13 | 18 | 12 | 4 | 0 | 4 | 16 | 26 |

x = AWGHE

y = AW-HE

**EP 3 848 935 A1**

<table>
<tr><td colspan="3" align="center">INTERNATIONAL SEARCH REPORT</td><td>International application No.<br>PCT/JP2018/033329</td></tr>
</table>

A. CLASSIFICATION OF SUBJECT MATTER
Int. Cl. G06F19/18(2011.01)i

According to International Patent Classification (IPC) or to both national classification and IPC

B. FIELDS SEARCHED

Minimum documentation searched (classification system followed by classification symbols)
Int. Cl. G06F19/18

Documentation searched other than minimum documentation to the extent that such documents are included in the fields searched
Published examined utility model applications of Japan 1922-1996
Published unexamined utility model applications of Japan 1971-2018
Registered utility model specifications of Japan 1996-2018
Published registered utility model applications of Japan 1994-2018

Electronic data base consulted during the international search (name of data base and, where practicable, search terms used)
PubMed

C. DOCUMENTS CONSIDERED TO BE RELEVANT

| Category* | Citation of document, with indication, where appropriate, of the relevant passages | Relevant to claim No. |
|---|---|---|
| Y | WO 2017/201050 A1 (MINATI, Ludovico) 23 November 2017, paragraph [0100] (Family: none) | 7-10, 17-20, 27-30 |
| A | | 1-6, 11-16, 21-26 |
| Y | JP 2003-256433 A (JAPAN SCIENCE AND TECHNOLOGY CORP.) 12 September 2003, paragraph [0025] (Family: none) | 7, 8, 17, 18, 27, 28 |
| Y | 山下達雄外1名, Suffix Array を用いたフルテキスト類似用例検索, 情報処理学会研究報告, 12 September 1997, vol.97, no.85, pp.83-90 (YAMASITA, Tatsuo, MATSUMOTO, Yuji. Full Text Approximate String Search using Suffix Arrays. IPSJ SIG Technical Report.) | 9, 10, 19, 20, 29, 30 |

☒ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>27.11.2018 | Date of mailing of the international search report<br>04.12.2018 |
|---|---|
| Name and mailing address of the ISA/<br>   Japan Patent Office<br>   3-4-3, Kasumigaseki, Chiyoda-ku,<br>   Tokyo 100-8915, Japan | Authorized officer<br><br><br>Telephone No. |

Form PCT/ISA/210 (second sheet) (January 2015)

**INTERNATIONAL SEARCH REPORT**

| International application No. |
| --- |
| PCT/JP2018/033329 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2002-132781 A (HITACHI, LTD.) 10 May 2002, entire text, all drawings & US 2002/0049545 A1 | 1-30 |
| A | US 2013/0332081 A1 (OMICIA INC.) 12 December 2013, entire text, all drawings & WO 2012/034030 A1 & EP 2614161 A | 1-30 |
| A | US 2015/0363546 A1 (GENEPEEKS INC.) 17 December 2015, entire text, all drawings & US 2016/0314245 A1 & WO 2015/195655 A1 & EP 3158486 A1 | 1-30 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009013910 A
- JP 2002132781 A
- JP 2004355522 A
- WO 2008108297 A
- JP 2015536156 A